# EUROPEAN PATENT APPLICATION

(11) **EP 4 431 504 A1**
(43) Date of publication of application: **18.09.2024**
(21) Application number: 22892062.5
(22) Date of filing: 10.11.2022
(51) Int. Cl.: C07D 401/14, C07D 401/04, C07D 413/14, C07D 403/12, C07D 405/12, C07D 409/12, C07D 471/04, A61K 31/517, A61K 31/519, A61P 35/00

(54) **AROMATIC HETEROCYCLIC COMPOUND AND APPLICATION THEREOF**

(30) Priority: 11.11.2021 CN 202111332018
(71) Applicant: Shanghai Institute of Material Medica, Chinese Academy of Sciences, Shanghai 201203 (CN)
(72) Inventor: XIONG, Bing, Shanghai 201203 (CN); LI, Jia, Shanghai 201203 (CN); LIU, Tongchao, Shanghai 201203 (CN); ZHOU, Yubo, Shanghai 201203 (CN); LI, Cong, Shanghai 201203 (CN); LI, Na, Shanghai 201203 (CN); KAN, Weijuan, Shanghai 201203 (CN); SU, Mingbo, Shanghai 201203 (CN); SHENG, Li, Shanghai 201203 (CN); HU, Xiaobei, Shanghai 201203 (CN)
(74) Representative: Lavoix
(86) International application number: PCT/CN2022/131193
(87) International publication number: WO 2023/083269

(57) **Abstract**

Disclosed are an aromatic heterocyclic compound and an application thereof. The present invention provides an aromatic heterocyclic compound as represented by formula I, and the compound has good LSD1 enzyme inhibitory activity, and can be used as an LSD1 inhibitor for treatment of tumors etc.

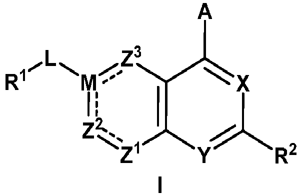

## Description

This application claims priority of the Chinese patent application 2021113320188, filed on November 11, 2021. This application refers to the full text of the above-mentioned Chinese patent application.

### Technical field

The present invention relates to an aromatic heterocyclic compound and application thereof.

### Background

Histone methylation is an important type of histone covalent modification in epigenetic regulation, which was previously considered as a relatively stable epigenetic modification until the discovery of the first specific lysine demethylase 1 (LSD1, KDM1A) by Shi Yang et al. in 2004, which led to the gradual recognition that histone methylation and demethylation is a dynamic equilibrium process [Cell, 2004, 119: 941-53.] Structurally, LSD1 can be divided into three parts: the Tower domain, the C-terminal Amino Oxidase (AOL) domain, and the N-terminal SWIRM domain. The Tower domain is composed of a set of antiparallel α-helices and plays a very important role in the catalytic function of LSD1. The N-terminal domain contains an α-helix, and can form protein-protein complexes with certain proteins, such as CoREST, NuRD and other proteins, which is beneficial to the maintenance of protein stability, thus making the proteins not easy to be deconstructed and deactivated while exercising higher functions. The C-terminal AOL domain is the structural center of the entire LSD1 when exercising the catalytic function, which is the hub of FAD recognition, and meanwhile the site for the specific binding to FAD. To a large extent, it is possible that the reduced activity of LSD1 enzyme is caused by the deformation and inactivation of this domain [PLoS Computational Biology, 2013, 9: e1003158.]. LSD1 belongs to the family of amine oxidases, which are dependent on the FAD-catalyzed oxidation reactions of H3K4 and H3K9 to produce a formaldehyde and a demethylated lysine residue for the removal of the methyl group.

LSD1 regulates many cell signaling pathways, and its dysfunction is closely associated with the development of many diseases, such as cancer, neurodegenerative diseases, cardiovascular diseases, inflammation, and viral infections. Aberrant high expression of LSD1 occurs in human hematological and solid tumor cells, such as acute myeloid leukemia [Cancer Res. 2016, 76 (7), 1975-1988.], small cell lung cancer [Cancer Cell 2015, 28 (1), 57-69.], breast cancer, adult neuroblastoma, colorectal cancer, prostate cancer [Am. J. Clin. Exp. Urol. 2014, 2 (4), 273-285.], liver cancer, gastric cancer, oral cancer, etc. [Future Med. Chem. 2017, 9: 1227-1242.]. The inhibition of LSD1 activity can lead to the inhibition of cancer cell differentiation, proliferation, migration, invasion, and tumor growth [Epigenomics 2016, 8 (8), 1103-1116.]. In addition, LSD1 inhibition enhances the tumor immunotherapy efficacy of PD-1 antibodies, which provides theoretical support for the combination of small-molecule LSD1 inhibitors and tumor immunotherapy [Cell, 2018, 174: 549-563.e19.]. Thus, LSD1 is a very promising therapeutic target.

There are currently no drugs approved for marketing as LSD1 inhibitors, and eight compounds are in the early clinical stages, six of which have a trans-phenylcyclopropylamine backbone. LSD1 inhibitor molecules with novel structural backbones are needed in this field.

### Summary

The technical problem to be solved by the present invention is that the single structure of the existing LSD1 inhibitors and other defects, and the present invention provides an aromatic heterocyclic compound and application thereof. This class of compound has better inhibitory activity against LSD1 and can be used as LSD1 inhibitors for the treatment of tumors and the like.

The present invention solves the above technical problems by the following technical solutions.

The present invention provides an aromatic heterocyclic compound of Formula I, a pharmaceutically acceptable salt thereof, a stereoisomer, a racemate, a prodrug, a co-crystalline complex, a hydrate, or a solvate thereof; 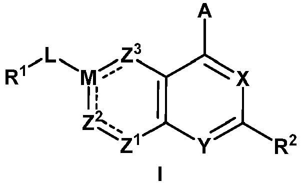
wherein, 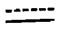 indicates a single bond or a double bond;
A is substituted 5-14-membered heteroaryl or substituted 6-14-membered aryl; wherein the substituted 5-14-membered heteroaryl and substituted 6-14-membered aryl are independently substituted by 1, 2 or 3 R^{a}; the heteroatoms in the 5-14-membered heteroaryl are selected from N, O and S and the number of heteroatoms is 1, 2, 3 or 4;
R^{a} is independently CN, F, Cl, Br, I or C₁₋₃ alkyl, and at least one is CN;
R¹ is hydrogen, halogen, substituted or unsubstituted (amino)-(C₁-C₄alkylidene)-, substituted or unsubstituted C₃₋₁₂cycloalkyl, substituted or unsubstituted 3-10memberedheterocycloalkyl, substituted or unsubstituted C₆₋C₁₄ aryl, or substituted or unsubstituted 5-14memberedheteroaryl, wherein the substituted (amino)-(C₁₋C₄alkylidene)-, substituted C₃₋₁₂cycloalkyl, substituted 3-10memberedheterocycloalkyl, substituted C₆₋C₁₄ aryl and substituted 5-14 memberedheteroaryl are independently substituted by 1, 2 or 3 R^{1a}; the heteroatoms in the 5-14-membered heteroaryl are selected from N, O and S, and the number of heteroatoms is 1, 2, 3 or 4; the heteroatoms in the saturated 3-10-membered heterocycloalkyl are selected from N, O and S, and the number of heteroatoms is 1, 2, 3 or 4;
R² is halogen, substituted or unsubstituted C₁₋₄ alkyl, substituted or unsubstituted saturated or unsaturated C₃₋₁₀cyclic hydrocarbyl, substituted or unsubstituted C₆-C₁₄ aryl, substituted or unsubstituted 3-10-membered heterocyclyl, substituted or unsubstituted 5-14-membered heteroaryl, substituted or unsubstituted 3-10-membered heterocyclyl-N(R³)-, substituted or unsubstituted 3-10-membered heterocyclyl-(C₁-C₄alkylene)-N(R³)-, substituted or unsubstituted N(R³)₂ -(C₁-C₄alkylidene)-N(R³)-, substituted or unsubstituted 3-10-membered heterocyclyl-O-, substituted or unsubstituted 3-10-membered heterocyclyl-(C₁-C₄alkylidene)-O-, substituted or unsubstituted N(R³)₂ -(C₁-C₄alkylidene)-O-, substituted or unsubstituted 3-10-membered heterocyclyl(C₁-C₄alkylidene)-; wherein the substituted C₁₋₄ alkyl, substituted saturated or unsaturated C₃₋₁₀cyclic hydrocarbyl, substituted C₆-C₁₄ aryl, substituted 3-10-membered heterocyclyl, substituted 5-14-membered heteroaryl, substituted 3-10-membered heterocyclyl-N(R³)-, substituted 3-10-membered heterocyclyl-(C₁-C₄alkylidene)-N(R³)-, substituted N(R³)₂ -(C₁₋C₄alkylene)-N(R³)-, substituted 3- 10-membered heterocyclyl-O-, substituted 3-10-membered heterocyclyl-(C₁-C₄alkylidene)-O-, and substituted N(R³)₂ -(C₁-C₄alkylidene)-O- are independently substituted by 1, 2, or 3 R^{1c}; the heteroatoms in the 5-14-membered heteroaryl are selected from N, O, and S, and the number of the heteroatoms is 1, 2, 3, or 4; and the 3-10-membered heterocyclyl is a saturated or unsaturated 3-10-membered heterocyclyl and the heteroatoms of which are selected from N, O and S, and the number of heteroatoms being 1, 2, 3 or 4;
each R^{1a} and R^{1c} are independently F, Cl, Br, I, OH, N(R³)₂, CN, COOH, CON(R³)₂ , S02N(R³)₂, C₁₋₃ alkyl, C₃₋₁₂cycloalkyl, C₁₋₃ alkyl-O-, or C₃₋₁₂cycloalkyl-O-, wherein the C₁₋₃ alkyl, C₃₋₁₂cycloalkyl, C₁₋₃ alkyl-O-, and C₃₋₁₂cycloalkyl-O- are optionally substituted by 1, 2, or 3 R^{1b};
each R^{1b} is independently F, Cl, Br, I, OH or NH₂;
each R³is independently hydrogen, substituted or unsubstituted C₁₋₄ alkyl, substituted or unsubstituted C₃₋₁₂cycloalkyl; wherein the substituted C₁₋₄ alkyl and substituted C₃₋₁₂cycloalkyl are independently substituted by 1, 2 or 3 R^{1b};
L is -NH-, -N(C₁-C₃alkyl)-, -O-, or absent (i.e., a linking bond, R¹ is directly connected to M);
X and Y are each independently N or C(R⁴);
M is C, CH or N;
Z¹, Z² and Z³ are each independently a linking bond, N, N(R⁴), C(H)(R⁴), C(R⁴), -(C=O)-, or -(C=S)-; and at most one of Z¹, Z² and Z³ is a linking bond;
R⁴is hydrogen, halogen, substituted or unsubstituted C₁₋₄ alkyl, substituted or unsubstituted C₃₋₁₂cycloalkyl; wherein the substituted C₁₋₄ alkyl and substituted C₃₋₁₂cycloalkyl are independently substituted by 1, 2 or 3 R^{1b}.

In a certain embodiment, an aromatic heterocyclic compound of Formula I, a pharmaceutically acceptable salt thereof, a stereoisomer, a racemate, a prodrug, a co-crystalline complex, a hydrate, or a solvate thereof; 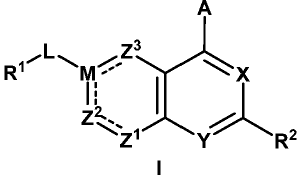 wherein, 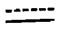 indicates a single bond or a double bond;
A is substituted 5-14-membered heteroaryl or substituted 6-14-membered aryl; wherein the substituted 5-14-membered heteroaryl and substituted 6-14-membered aryl are independently substituted by 1, 2 or 3 R^{a}; the heteroatoms in the 5-14-membered heteroaryl are selected from N, O and S and the number of heteroatoms is 1, 2, 3 or 4;
R^{a} is independently CN, F, Cl, Br, I or C₁₋₃ alkyl, and at least oneis CN;
R¹ is hydrogen, halogen, substituted or unsubstituted (amino)-(C₁-C₄alkylidene)-, substituted or unsubstituted C₃₋₁₂cycloalkyl, substituted or unsubstituted 3-10 memberedheterocycloalkyl, substituted or unsubstituted C₆-C₁₄ aryl, or substituted or unsubstituted 5-14memberedheteroaryl, wherein the substituted (amino)-(C₁-C₄alkylidene)-, substituted C₃₋₁₂cycloalkyl, substituted 3-10 memberedheterocycloalkyl, substituted C₆-C₁₄ aryl and substituted 5-14 memberedheteroaryl are independently substituted by 1, 2 or 3 R^{1a}; the heteroatoms in the 5-14-membered heteroaryl are selected from N, O and S, and the number of heteroatoms is 1, 2, 3 or 4; the heteroatoms in the saturated 3-10-membered heterocycloalkyl are selected from N, O and S, and the number of heteroatoms is 1, 2, 3 or 4;
R² is halogen, substituted or unsubstituted C₁₋₄ alkyl, substituted or unsubstituted saturated or unsaturated C₃₋₁₀cyclic hydrocarbyl, substituted or unsubstituted C₆-C₁₄ aryl, substituted or unsubstituted 3-10-membered heterocyclyl, substituted or unsubstituted 5-14-membered heteroaryl, substituted or unsubstituted 3-10-membered heterocyclyl-N(R³)-, substituted or unsubstituted 3-10-membered heterocyclyl-(C₁-C₄alkylene)-N(R³)-, substituted or unsubstituted N(R³)₂ -(C₁₋C₄alkylidene)-N(R³)-, substituted or unsubstituted 3-10-membered heterocyclyl-O-, substituted or unsubstituted 3-10-membered heterocyclyl-(C₁-C₄alkylidene)-O-, substituted or unsubstituted N(R³)₂ -(C₁-C₄alkylidene)-O-, substituted or unsubstituted 3-10-membered heterocyclyl-(C₁-C₄alkylidene)-; wherein the substituted C₁₋₄ alkyl, substituted saturated or unsaturated C₃₋₁₀cyclic hydrocarbyl, substituted C₆-C₁₄ aryl, substituted 3-10-membered heterocyclyl, substituted 5-14-membered heteroaryl, substituted 3-10-membered heterocyclyl-N(R³)-, substituted 3-10-membered heterocyclyl-(C₁₋C₄alkylidene)-N(R³)-, substituted N(R³)₂ -(C₁₋C₄alkylene)-N(R³)-, substituted 3- 10-membered heterocyclyl-O-, substituted 3-10-membered heterocyclyl-(C₁₋C₄alkylidene)-O-, and substituted N(R³)₂ -(C₁₋C₄alkylidene)-O- are independently substituted by 1, 2, or 3 R^{1c}; the heteroatoms in the 5-14-membered heteroaryl are selected from N, O, and S, and the number of the heteroatoms is 1, 2, 3, or 4; and the 3-10-membered heterocyclyl is a saturated or unsaturated 3-10-membered heterocyclylwiththe heteroatomselected from N, O and S, and the number of heteroatoms being1, 2, 3 or 4;
each R^{1a} and R^{1c}areindependently F, Cl, Br, I, OH, N(R³)₂, CN, COOH, CON(R³)₂ , SO₂N(R³)₂, C₁₋₃ alkyl, C₃₋₁₂cycloalkyl, C₁₋₃ alkyl-O-, or C₃₋₁₂cycloalkyl-O-, wherein the C₁₋₃ alkyl, C₃₋₁₂cycloalkyl, C₁₋₃ alkyl-O-, and C₃₋₁₂cycloalkyl-O- are optionally substituted by 1, 2, or 3 R^{1b};
each R^{1b} is independently F, Cl, Br, I, OH or NH₂;
each R³is independently hydrogen, substituted or unsubstituted C₁₋₄ alkyl, substituted or unsubstituted C₃₋₁₂cycloalkyl; wherein the substituted C₁₋₄ alkyl and substituted C₃₋₁₂cycloalkyl are independently substituted by 1, 2 or 3 R^{1b};
L is -NH-, -N(C₁-C₃alkyl)-, -O-, or absent (i.e., a linking bond, R¹is directly connected to M);
X and Y are each independently N or C(R⁴);
M is C, CH or N;
Z¹, Z² and Z³ are each independently a linking bond, N, N(R⁴), C(H)(R⁴), C(R⁴), or -(C=O)-; and at most one of Z¹, Z² and Z³ is a linking bond;
R⁴ is hydrogen, halogen, substituted or unsubstituted C₁₋₄ alkyl, substituted or unsubstituted C₃₋₁₂cycloalkyl; wherein the substituted C₁₋₄ alkyl and substituted C₃₋₁₂cycloalkyl are independently substituted by 1, 2 or 3 R^{1b}.

In a certain embodiment, in the aromatic heterocyclic compound of Formula I, the pharmaceutically acceptable salt thereof, thestereoisomer, theracemate, theprodrug, the co-crystalline complex, the hydrate, or thesolvate thereof, certain groups have the following definitions, and groups not mentioned are defined as described in any of the embodiments of the present invention (the contents of the present paragraph are hereinafter referred to as "in a certain embodiment"),
wherein the aromatic heterocyclic compound of formula I is as follows: 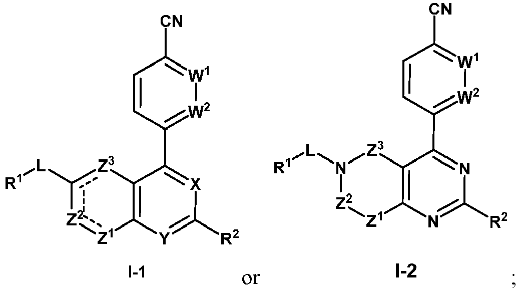 formula I-1 is e.g 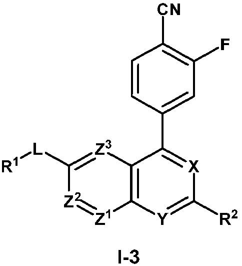 or 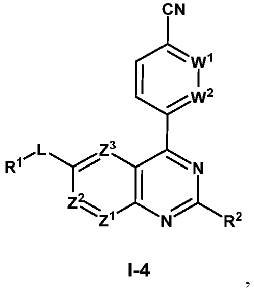 and also e.g. 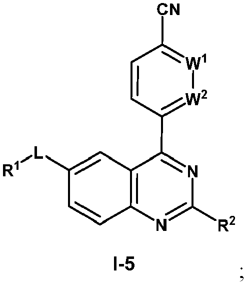 wherein ------, R¹, R², L, X, Y, Z¹, Z² and Z³ are as described in any one of the embodiments of the present invention; W¹ and W² are each independently N, C(H), or C(R^{a}); wherein R^{a} may be F, Cl, Br, I or C₁₋₃ alkyl.

In a certain embodiment, A is substituted 6-14-membered aryl, R¹is substituted or unsubstituted C₆-C₁₄ aryl, and R²is substituted or unsubstituted C₆-C₁₄ aryl, wherein the C₆-C₁₄ aryl is independently phenyl or naphthyl.

In a certain embodiment, A is substituted 5-14-membered heteroaryl, R¹is substituted or unsubstituted 5-14-membered heteroaryl, and R²is substituted or unsubstituted 5-14-membered heteroaryl, wherein the 5-14-membered heteroaryl is 5-10-membered heteroarylwiththe heteroatoms selected from N, O, and S, and the number of heteroatoms being 1 or 2; for example, 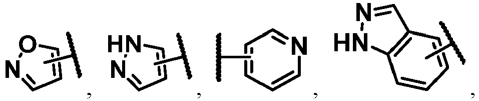 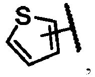 and also e.g.

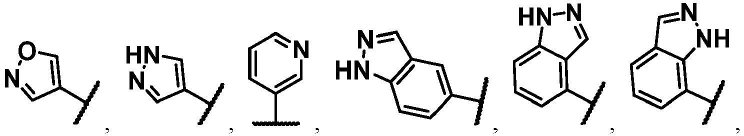

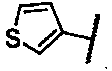

In a certain embodiment, R¹, R²and R⁴are halogen, wherein the halogenis independently F, Cl, Br, I, for example Br.

In a certain embodiment, R¹is substituted or unsubstituted (amino)-(C₁₋C₄alkylidene)-, and R²is substituted or unsubstituted 3-10-membered heterocyclyl-(C₁-C₄alkylidene)-N(R³)-, substituted or unsubstituted N(R³)₂-(C₁-C₄alkyfidene)-N(R³)-, substituted or unsubstituted 3-10-membered heterocyclyl-O-, substituted or unsubstituted 3-10-membered heterocyclyl-(C₁₋C₄alkyfidene)-O- or substituted or unsubstituted N(R³)₂ -(C₁₋C₄alkylidene)-O-, wherein the (C₁-C₄alkylidene) is-CH₂-,-CH₂CH₂ -,-CH(CH₃)-,-CH(CH₃)CH₂ - or-C(CH₃)₂-.

In a certain embodiment, R¹is substituted or unsubstituted C₃₋₁₂cycloalkyl, R^{1a} and R^{1c} are independently C₃₋₁₂cycloalkyl or C₃₋₁₂cycloalkyl-O-, R³is substituted or unsubstituted C₃₋₁₂cycloalkyl, and R⁴is substituted or unsubstituted C₃₋₁₂cycloalkyl, wherein the C₃₋₁₂cycloalkyl is independently C₃₋₇ monocyclic cycloalkyl, C₄₋₁₂ bridged or fused cycloalkyl, or C₇₋₁₂spirocycloalkyl;
the C₃₋₇ monocyclic cycloalkyliscyclopropyl, cyclobutyl, cyclopentyl or cyclohexyl.

In a certain embodiment, R¹is substituted or unsubstituted 3-10-membered heterocycloalkyl, wherein the 3-10-membered heterocycloalkyl is independently C₃₋₇ monocyclic heterocycloalkyl, C₄₋₁₂ bridged or fused heterocycloalkyl, or C₇₋₁₂spiroheterocycloalkyl, wherein the heteroatoms may be selected from N, O and S, and the number of the heteroatoms may be 1 or 2;
the C₃₋₇ monocyclic heterocycloalkyl may be pyrrolidinyl 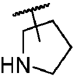 (e.g. 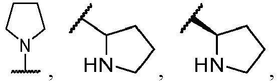 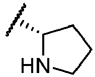 ), piperidinyl 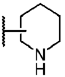 (e.g. 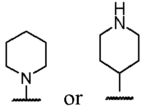 ), piperazinyl 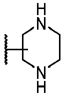 (e.g. 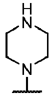 ), 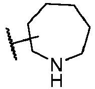 (e.g. 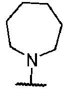 );
the C₄₋₁₂ bridged or fused heterocycloalkyl may be 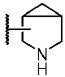 (e.g., 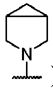 ), 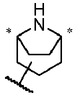 (e.g., 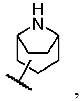 also e.g., 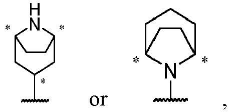 also e.g., 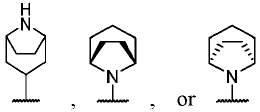 ), 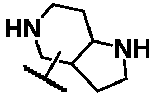 (e.g., 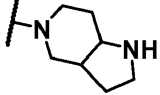 ),
the C₇₋₁₂spiroheterocycloalkyl may be 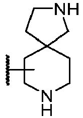 (e.g. 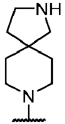 ).

In a certain embodiment, R²is substituted or unsubstituted C₁₋₄ alkyl, R³is substituted or unsubstituted C₁₋₄ alkyl, and R⁴is substituted or unsubstituted C₁₋₄ alkyl, wherein the C₁₋₄ alkylisindependently methyl, ethyl, n-propyl, isopropyl, n-butyl, sec-butyl, isobutyl, or tert-butyl.

In a certain embodiment, R²is substituted or unsubstituted saturated or unsaturated C₃₋₁₂cyclic hydrocarbyl, wherein the saturated C₃₋₁₂cyclic hydrocarbyl is C₃₋₁₂cycloalkyl, such as cyclopropyl, cyclobutyl, cyclopentyl or cyclohexyl.

In a certain embodiment, R²is substituted or unsubstituted 3-10-membered heterocyclyl, substituted or unsubstituted 3-10-membered heterocyclyl-N(R³)-, substituted or unsubstituted 3-10-membered heterocyclyl-(C₁-C₄alkylidene)-N(R³)-, substituted or unsubstituted 3-10-membered heterocyclyl-O-, or substituted or unsubstituted 3-10-membered heterocyclyl-(C₁-C₄alkylidene)-O-, wherein the 3-10-membered heterocyclyl is independently 3-10-membered heterocycloalkyl, the 3-10-membered heterocycloalkyl is independently C₃₋₇ monocyclic heterocycloalkyl, C₄₋₁₂ bridged or fused heterocycloalkyl, C₇₋₁₂spiroheterocycloalkyl, wherein the heteroatoms is selected from N, O and S, and the number of heteroatoms is 1 or 2; the C₃₋₇ monocyclic heterocycloalkyl may be pyrrolidinyl 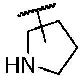 (e.g. 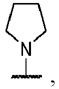 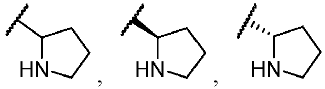 ), piperidinyl 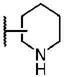 (e.g. 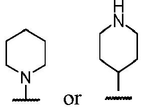 ), piperazinyl 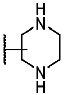 (e.g. 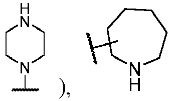 (e.g. 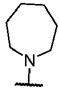 );
the C₄₋₁₂ bridged or fused heterocycloalkyl may be 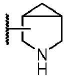 (e.g., 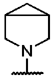 ), 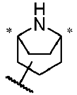 (e.g., 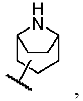 also e.g., 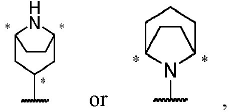 also e.g., 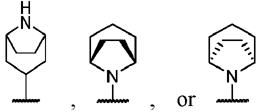 ), 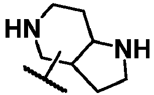 (e.g., 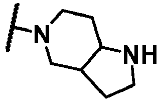 ),
the C₇₋₁₂spiroheterocycloalkyl may be 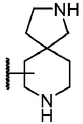 (e.g. 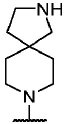 ).

In a certain embodiment, R^{a} is independently C₁₋₃ alkyl, R^{1a} and R^{1c} are independently C₁₋₃ alkyl or C₁₋₃ alkyl-O-, and L is -N(C₁-C₃ alkyl)-, wherein the C₁₋₃ alkyl is independently methyl, ethyl, n-propyl or isopropyl.

In a certain embodiment, A is 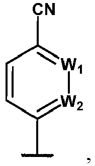 and W¹ and W² are each independently N, C(H), or C(R^{a});
wherein R^{a} may be F, Cl, Br, I or C₁₋₃ alkyl;
for example, W¹ is N, C-H or C-F; W² is C-H.

In a certain embodiment R^{a} is independently CN or F, and at least one of R^{a}is CN.

In a certain embodiment, at least one of R^{a} is CN and located at the opposite position of

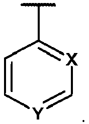

In a certain embodiment, R¹is halogen, substituted or unsubstituted C₆-C₁₄ aryl or substituted or unsubstituted 5-14-membered heteroaryl.

In a certain embodiment, R²is substituted or unsubstituted 3-10-membered N-containing heterocyclyl, substituted or unsubstituted 3-10-membered heterocyclyl-(C₁-C₄alkyfidene)-N(R³)-, orsubstituted or unsubstituted 3-10-membered heterocyclyl -(C₁₋C₄alkylidene)-O-.

In a certain embodiment, each R^{1a} and R^{1c} areindependently F, Cl, OH, N(R³)₂, CON(R³)₂, SO₂N(R³)₂, C₁₋₃alkyl, or C₁₋₃alkyl-O-, wherein the C₁₋₃ alkyl and C₁₋₃ alkyl-O- are optionally substituted by 1, 2, or 3 R^{1b}, e.g., substituted by halogen.

In a certain embodiment, each R^{1b} is independently F or NH₂.

In a certain embodiment, R³is hydrogen or substituted or unsubstituted C₁₋₄ alkyl, such as hydrogen or C₁₋₄ alkyl, or for example, hydrogen or methyl.

In a certain embodiment, L is -NH-, -O-, or absent; e.g., absent.

In a certain embodiment, X and Y are each independently N; i.e., 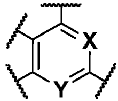 is

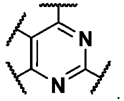

In a certain embodiment, X and Y are each independently N or CH; for example, 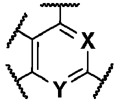 is

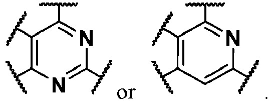

In a certain embodiment, Z¹, Z² and Z³ are each independently N, C(H)(R⁴) or C-R⁴.

In a certain embodiment, Z¹, Z² and Z³ are each independently N, N(R⁴), C(H)(R⁴), C-R⁴or -(C=O)-. In a certain embodiment, R⁴is hydrogen or halogen; for example, H or F.

In a certain embodiment, R⁴is hydrogen, halogen, or C₁₋₄ alkyl; for example, H, F, or methyl.

In a certain embodiment, R^{1a} and R^{1c} are independently F, Cl, methyl, isopropyl, methyl-O-, SO₂NH₂, CF₃-O-, CF₃-, OH-, or CONH₂.

In a certain embodiment, R¹is 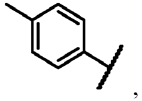 Br, 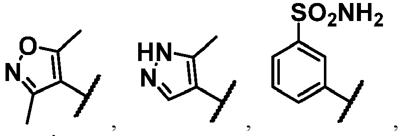 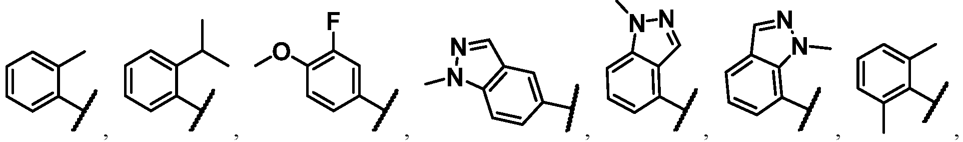 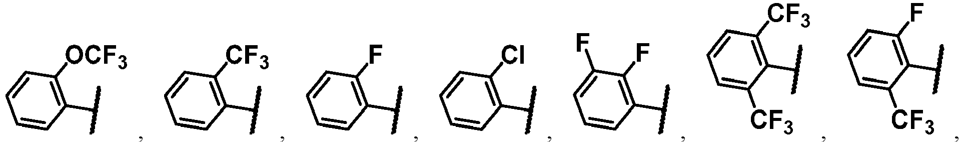 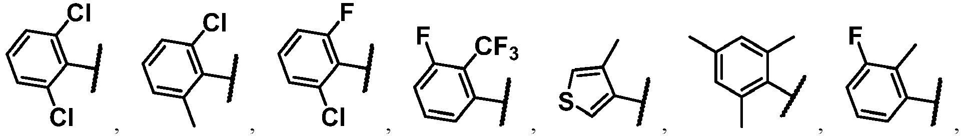 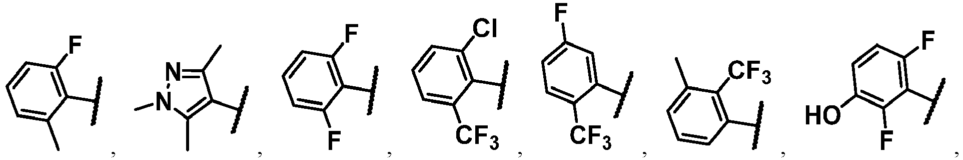 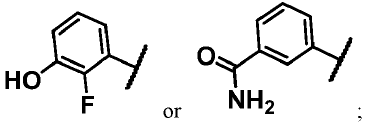 for example, 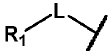 is 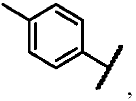 Br, 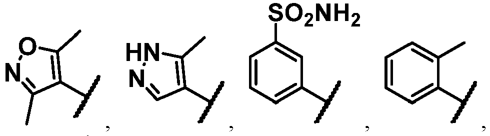 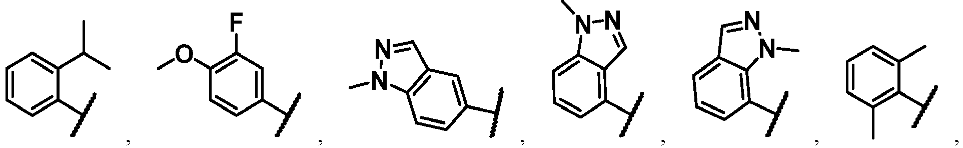 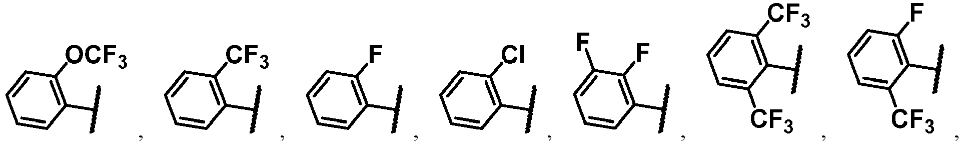 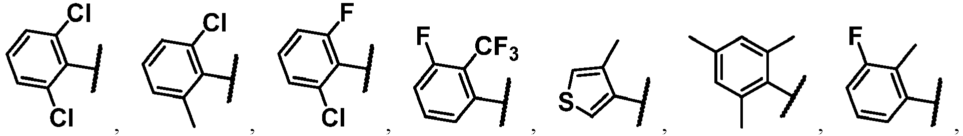 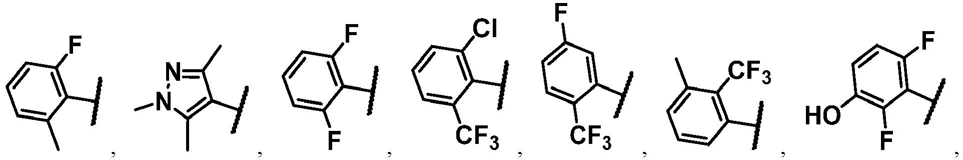 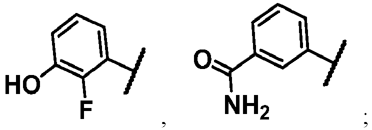 preferably 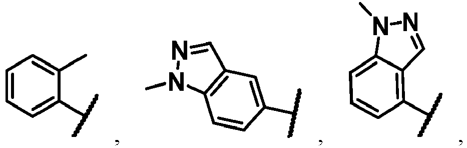 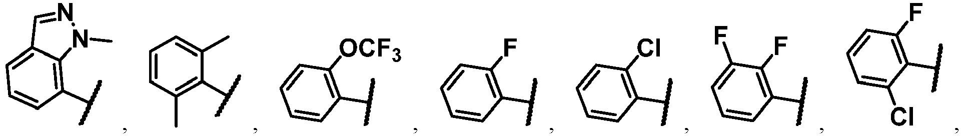 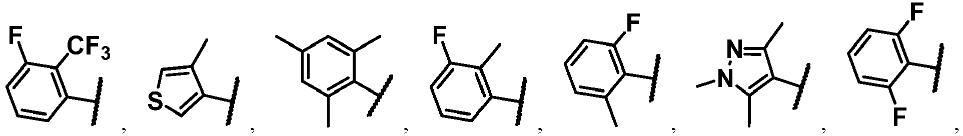 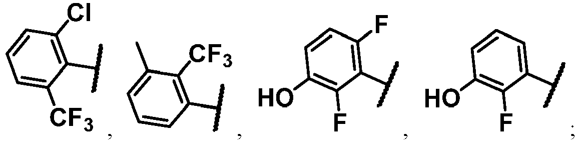 more preferably

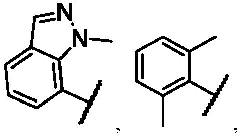

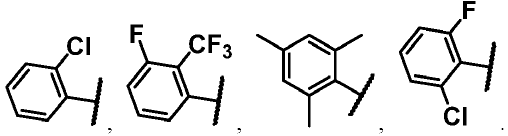

In a certain embodiment,R¹ is 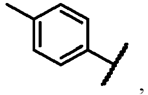 Br, 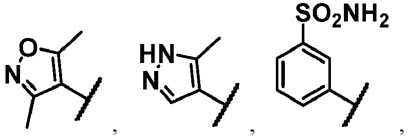 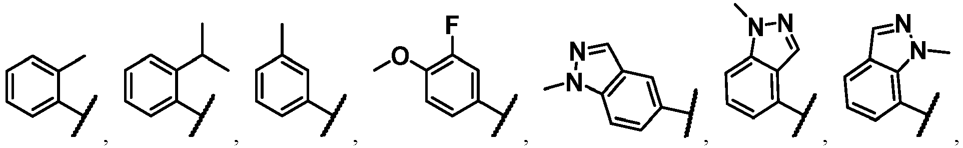 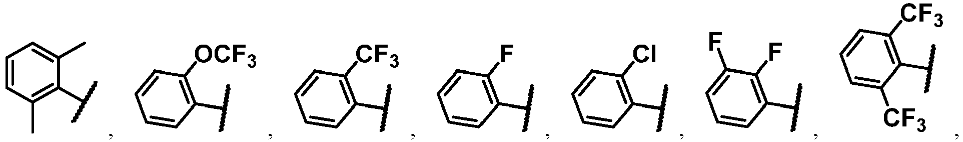 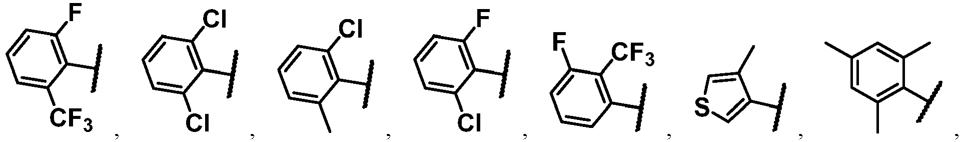 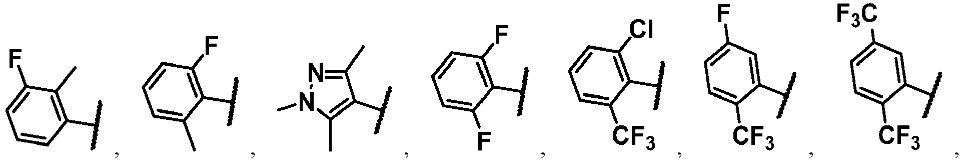 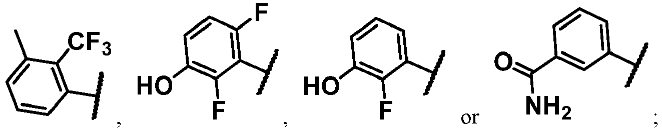 for example, 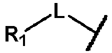 is 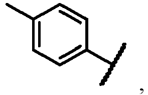 Br, 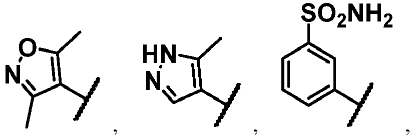 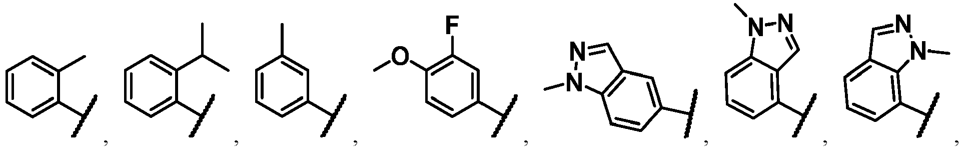 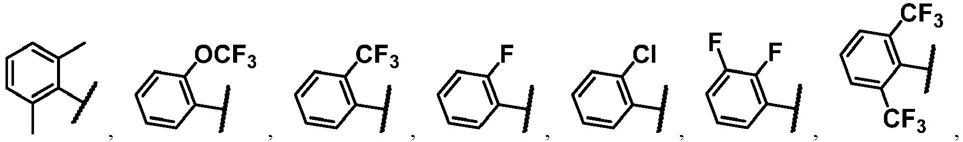 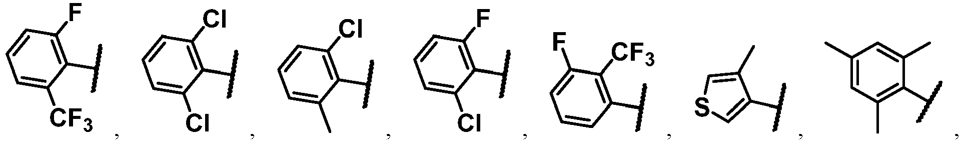 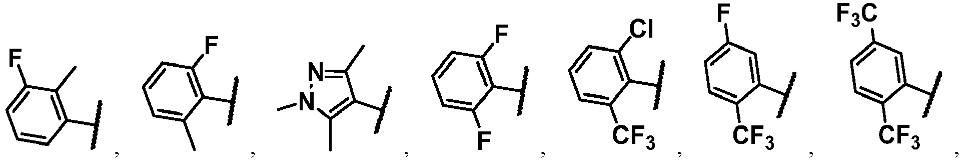 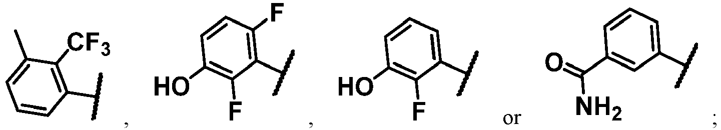 preferably 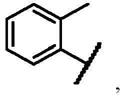 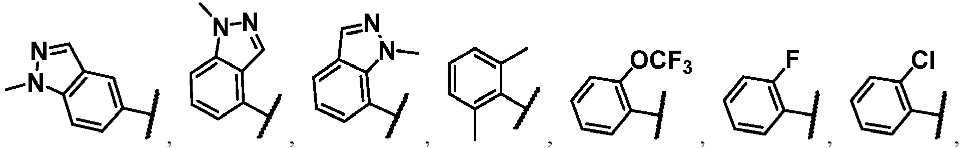 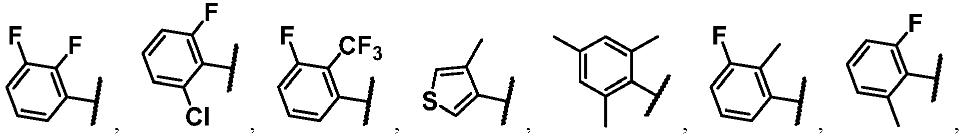 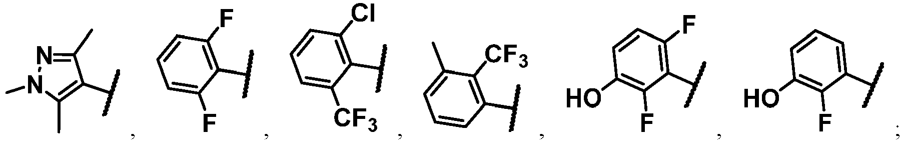 more preferably

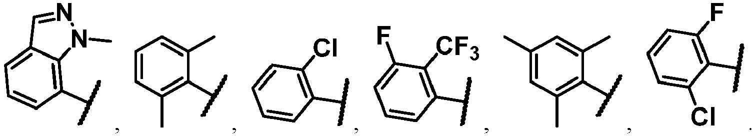

In a certain embodiment, A is 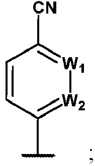 e.g., 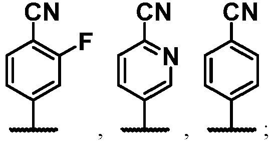 preferably

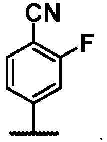

In a certain embodiment, 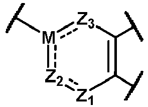 is 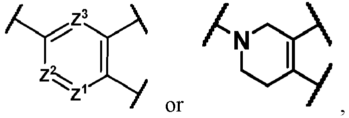 for example 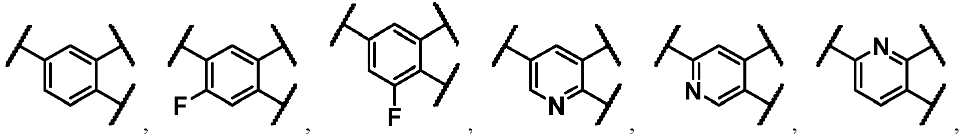 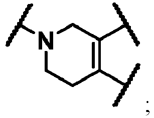 for example, 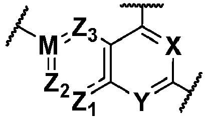 is 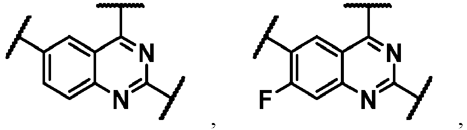 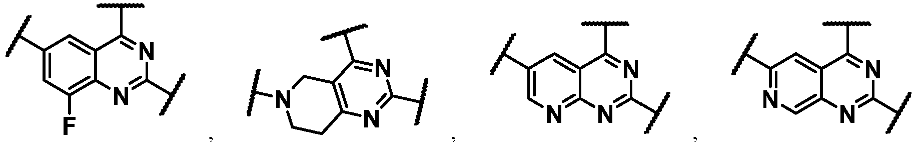 or

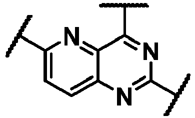

In a certain embodiment, 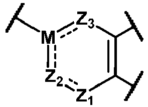 is 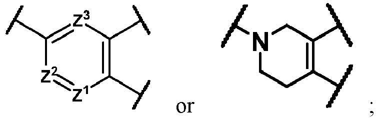 e.g., 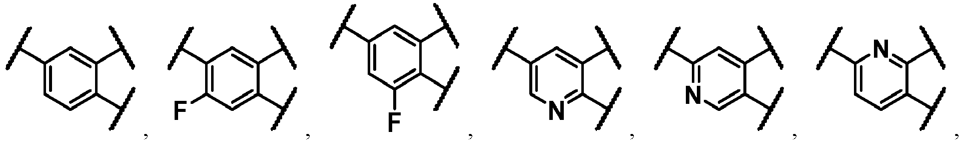 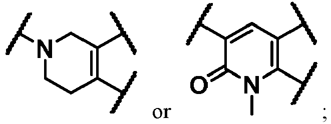 for example, 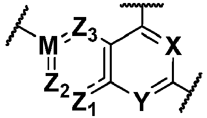 is

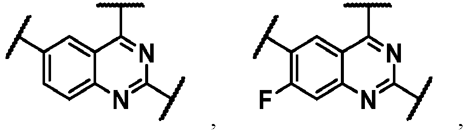

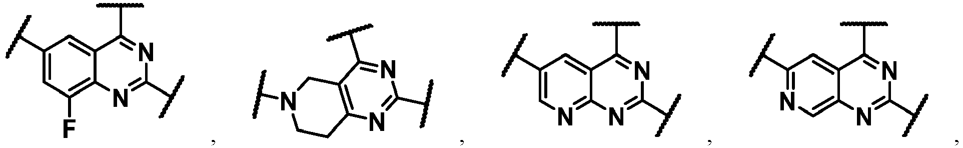

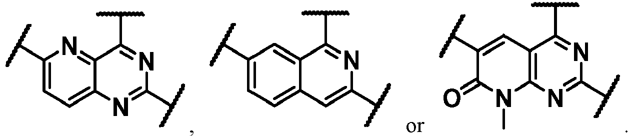

In a certain embodiment, R² is 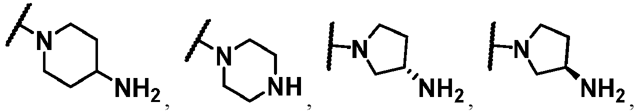 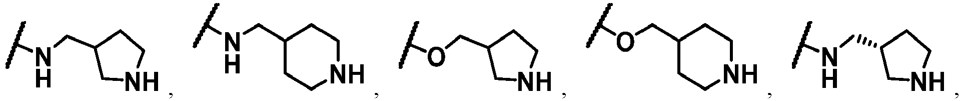 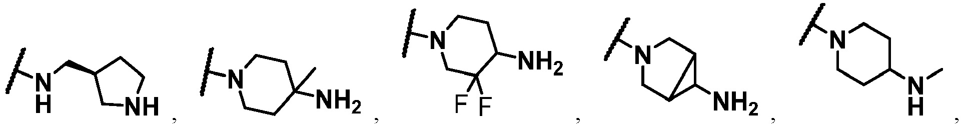 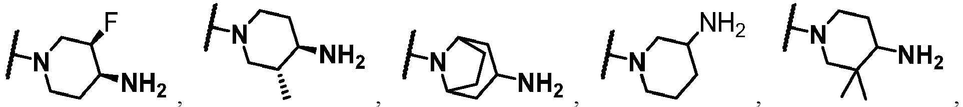 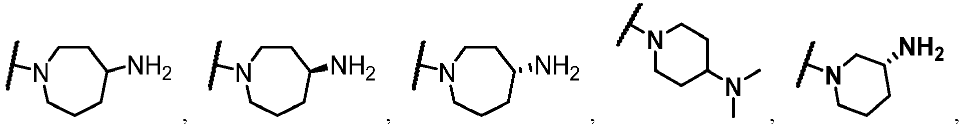 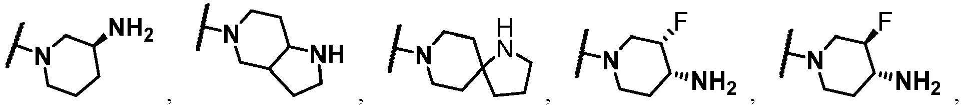 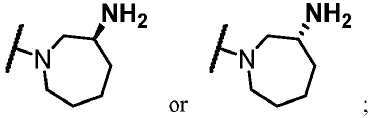 e.g., 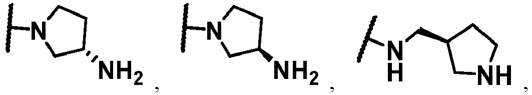 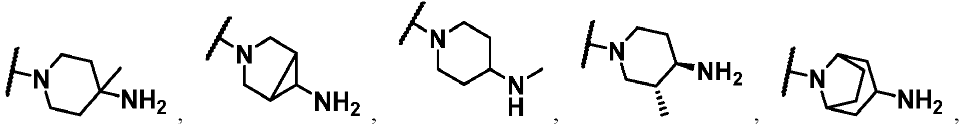 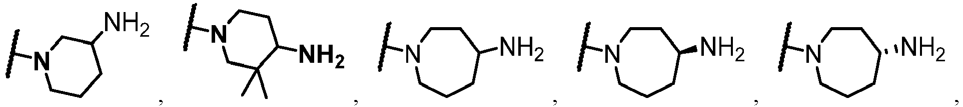 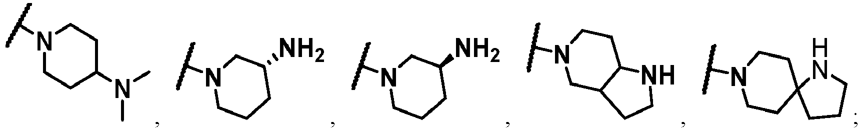 preferably

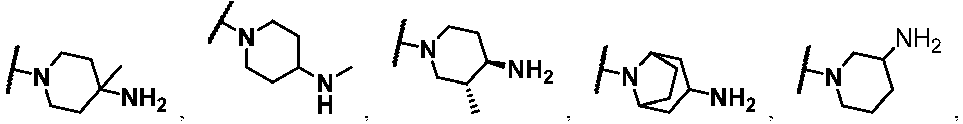

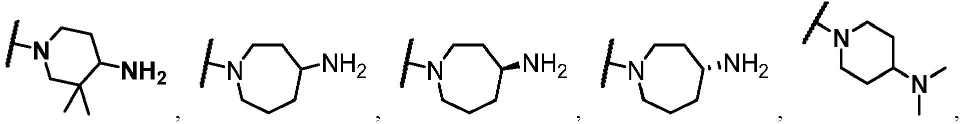

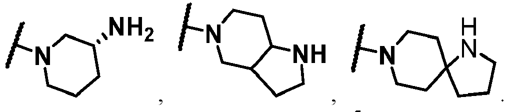

In a certain embodiment, R² is 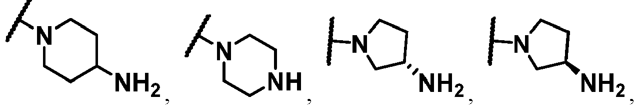 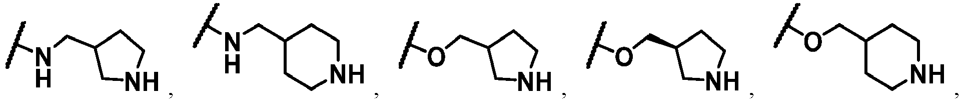 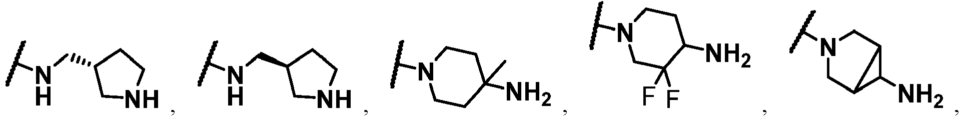 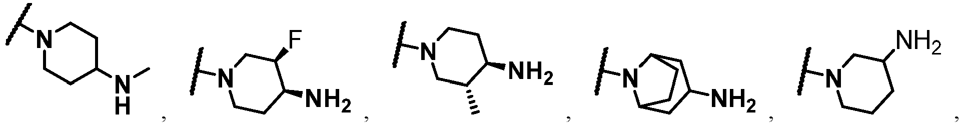 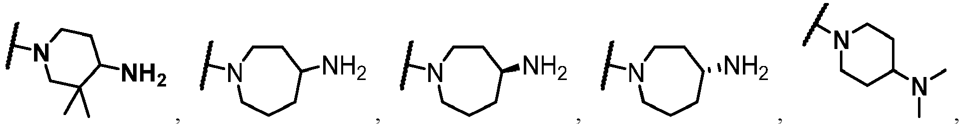 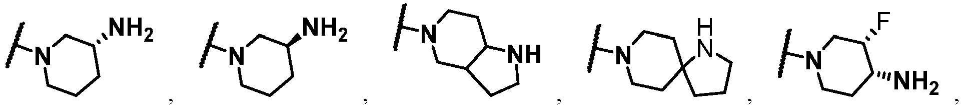 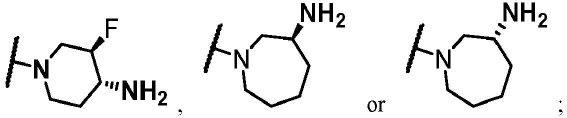 e.g., 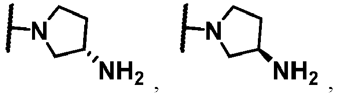 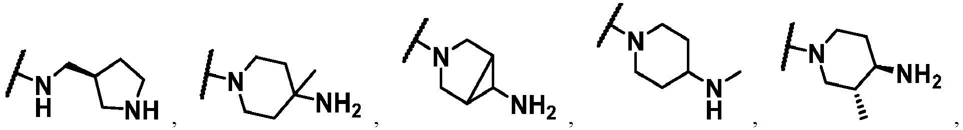 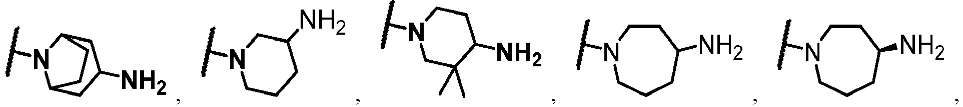 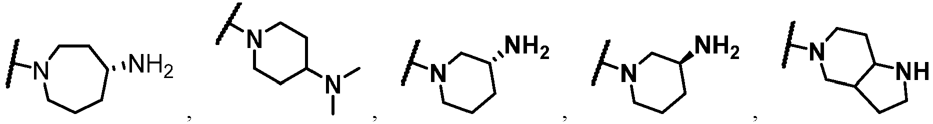 or 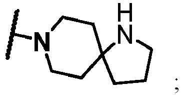 preferably

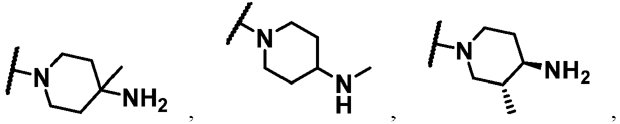

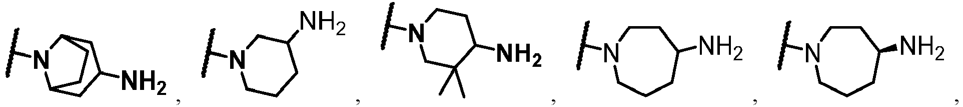

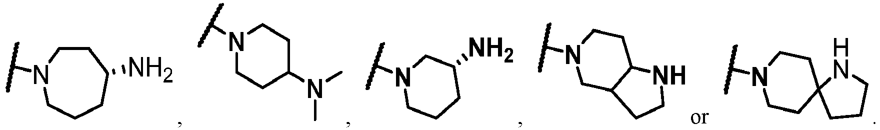

In a certain embodiment, the aromatic heterocyclic compound of Formula I, or pharmaceutically acceptable salts thereof are compounds with the following structure, or hydrochloride thereof (Table 1):

**Table 1:**

| | | | |
|---|---|---|---|
| 1 | 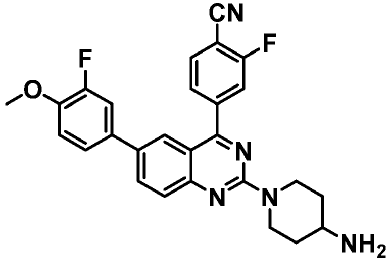 | 2 | 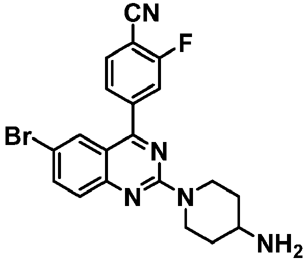 |
| 3 | 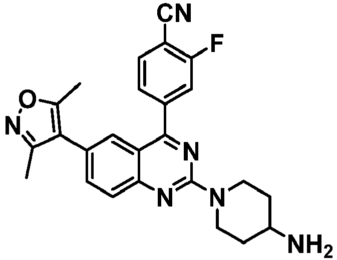 | 4 | 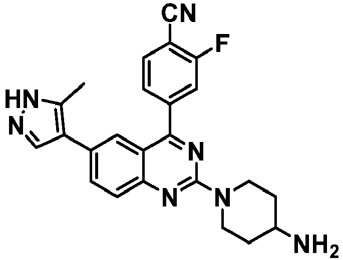 |
| 5 | 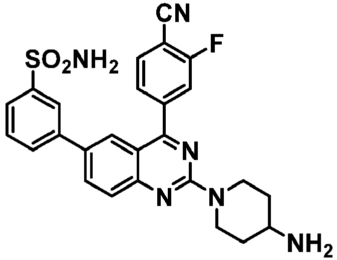 | 6 | 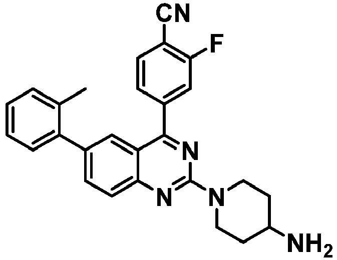 |
| 7 | 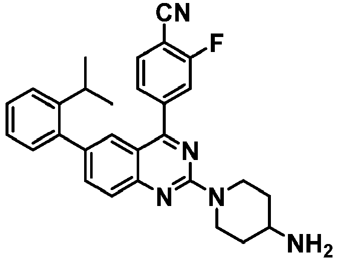 | 8 | 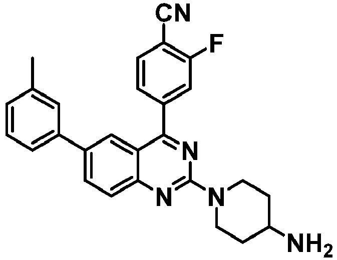 |
| 9 | 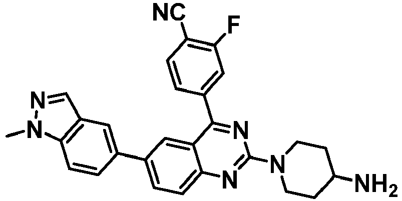 | 10 | 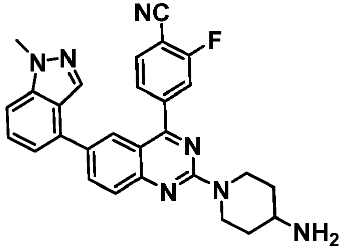 |
| 11 | 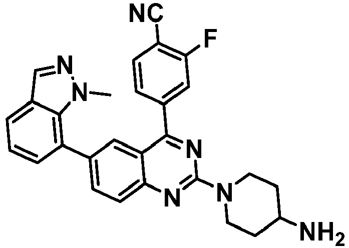 | 12 | 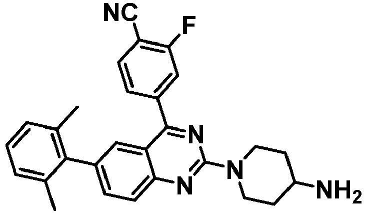 |
| 13 | 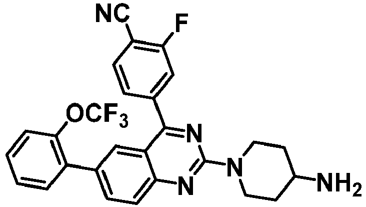 | 14 | 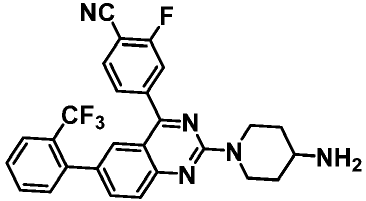 |
| 15 | 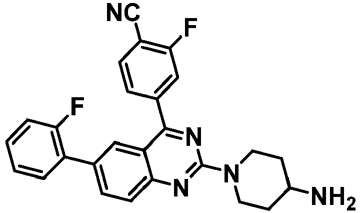 | 16 | 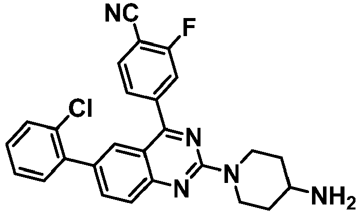 |
| 17 | 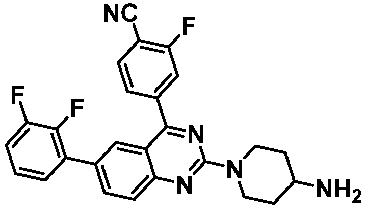 | 18 | 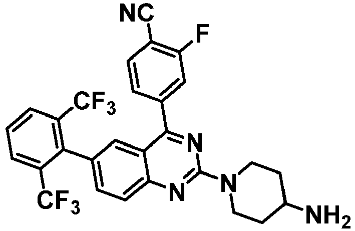 |
| 19 | 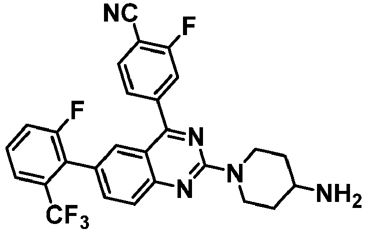 | 20 | 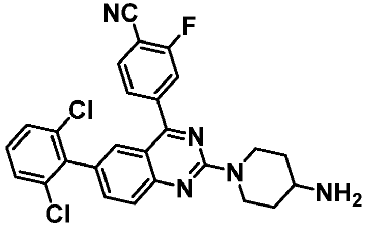 |
| 21 | 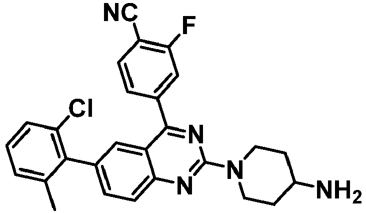 | 22 | 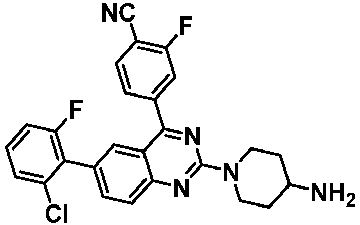 |
| 23 | 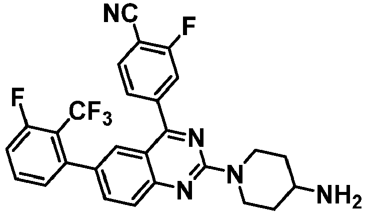 | 24 | 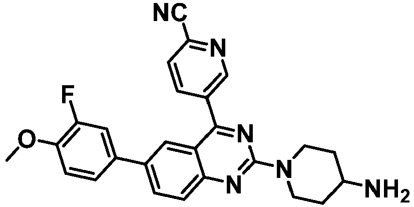 |
| 25 | 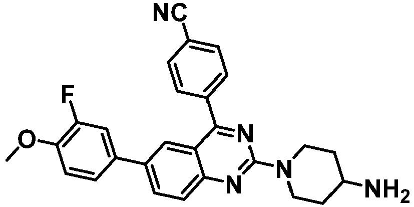 | 26 | 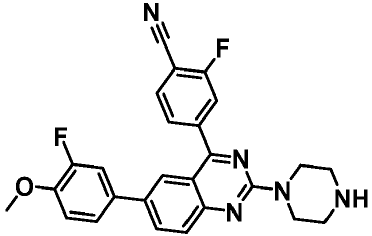 |
| 27 | 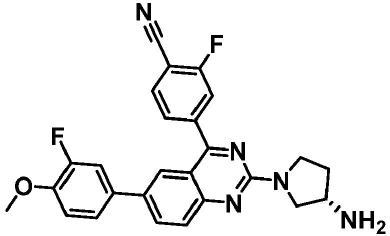 | 28 | 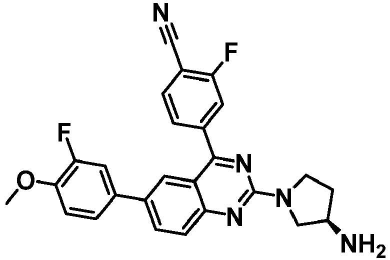 |
| 29 | 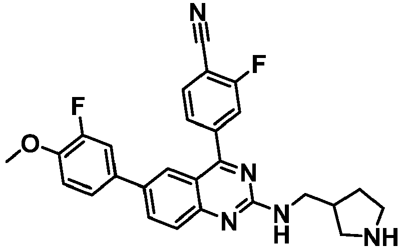 | 30 | 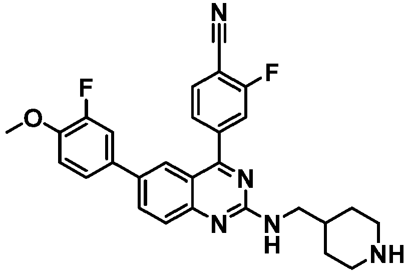 |
| 31 | 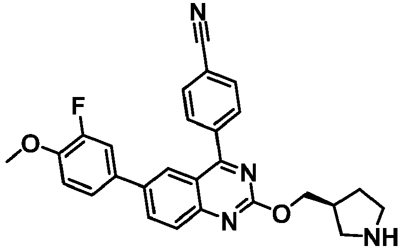 | 32 | 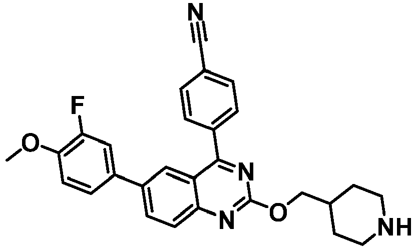 |
| 33 | 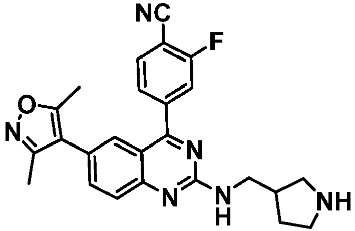 | 34 | 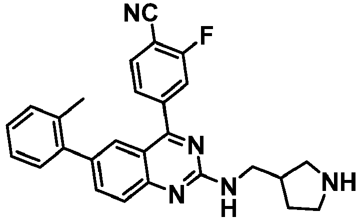 |
| 35 | 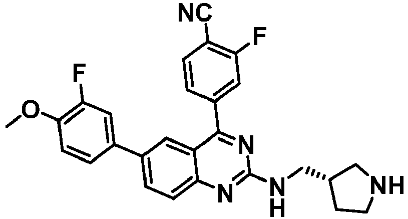 | 36 | 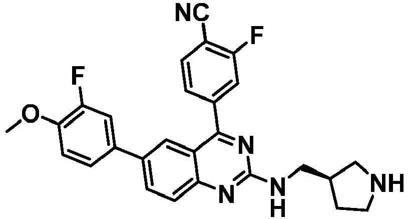 |
| 37 | 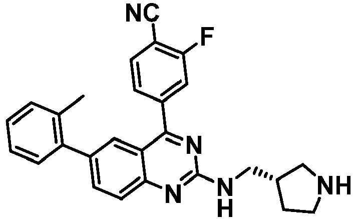 | 38 | 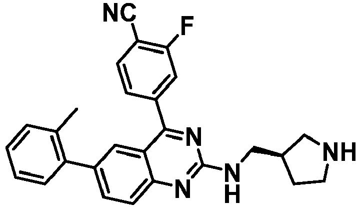 |
| 39 | 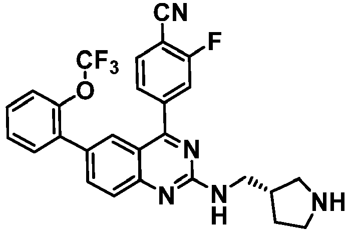 | 40 | 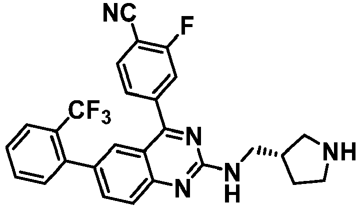 |
| 41 | 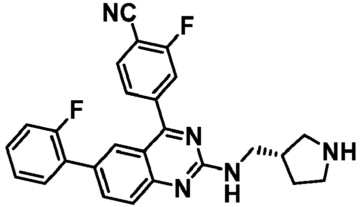 | 42 | 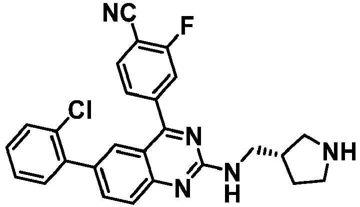 |
| 43 | 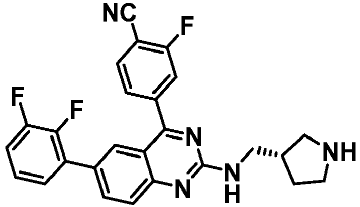 | 44 | 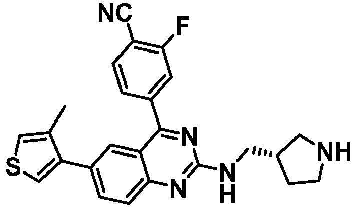 |
| 45 | 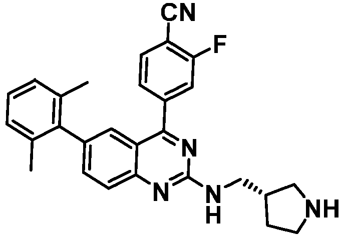 | 46 | 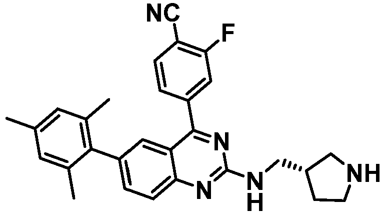 |
| 47 | 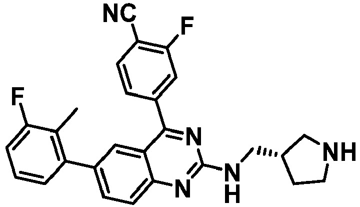 | 48 | 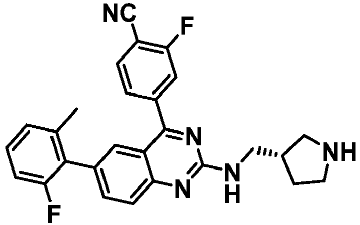 |
| 49 | 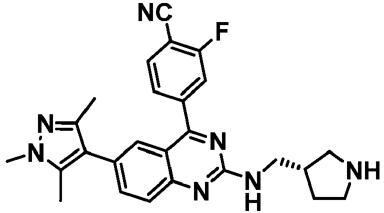 | 50 | 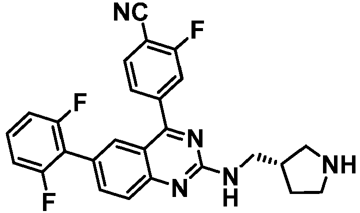 |
| 51 | 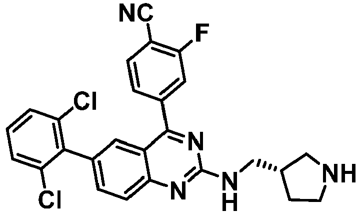 | 52 | 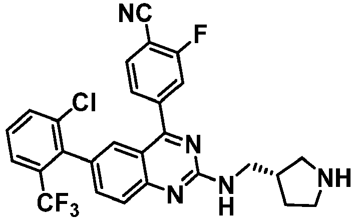 |
| 53 | 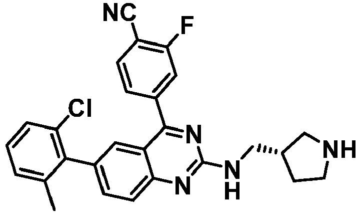 | 54 | 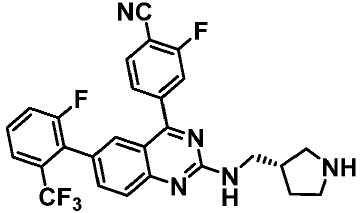 |
| 55 | 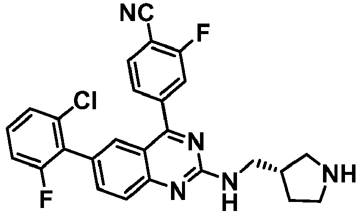 | 56 | 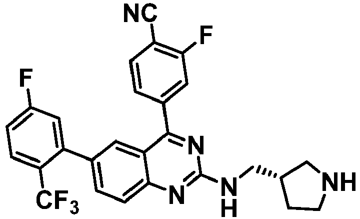 |
| 57 | 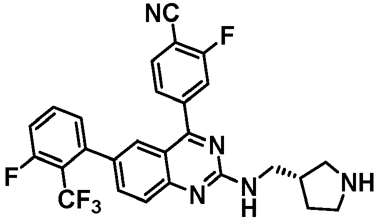 | 58 | 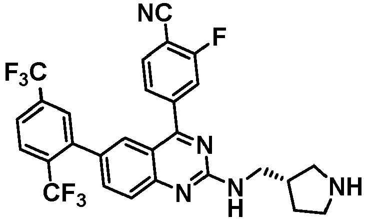 |
| 59 | 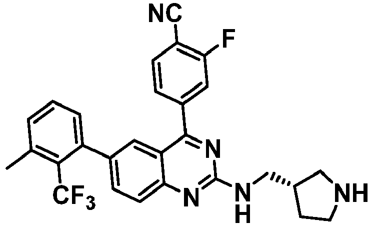 | 60 | 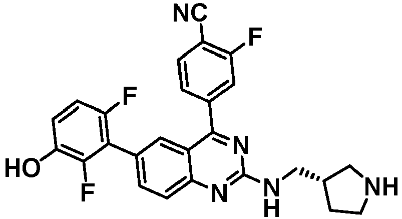 |
| 61 | 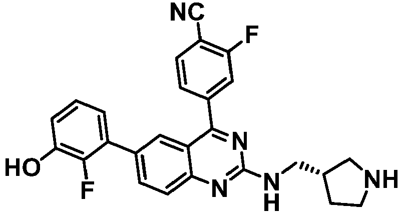 | 62 | 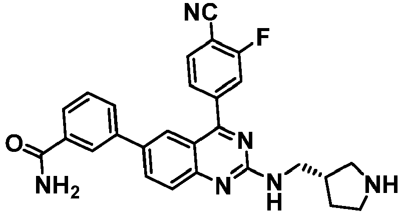 |
| 63 | 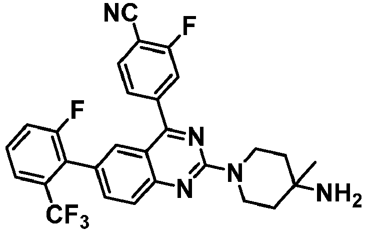 | 64 | 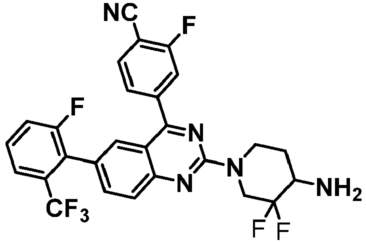 |
| 65 | 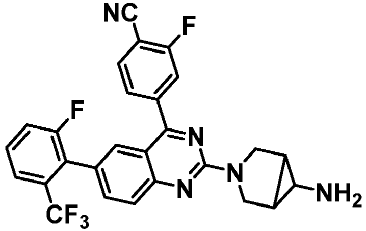 | 66 | 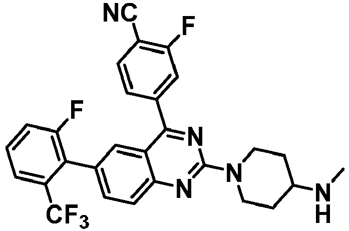 |
| 67 | 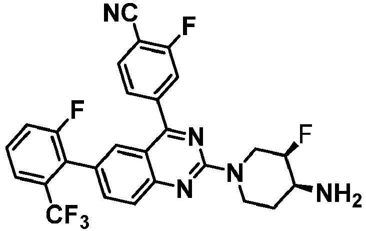 | 68 | 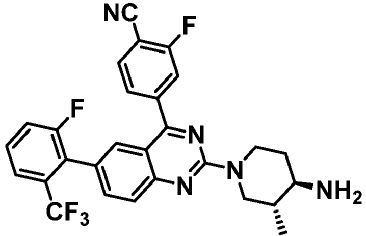 |
| 69 | 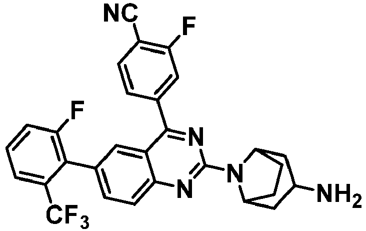 | 70 | 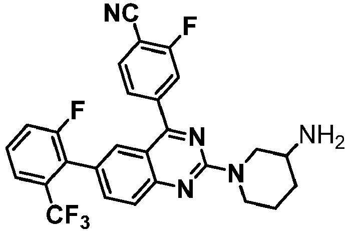 |
| 71 | 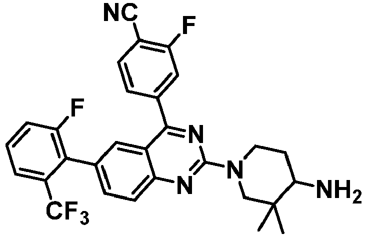 | 72 | 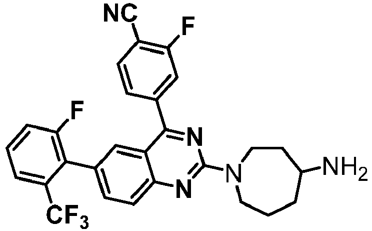 |
| 73 | 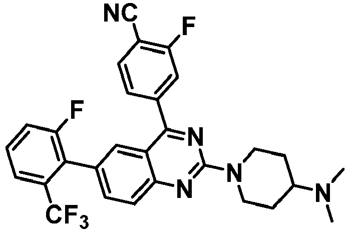 | 74 | 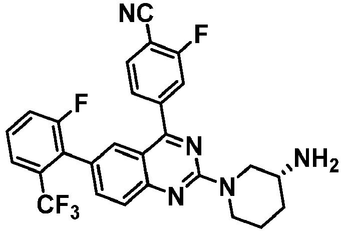 |
| 75 | 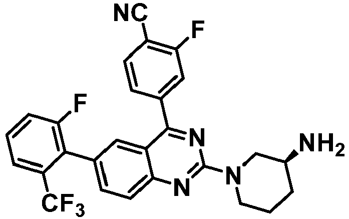 | 76 | 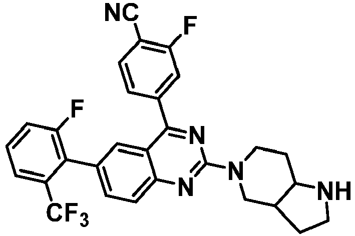 |
| 77 | 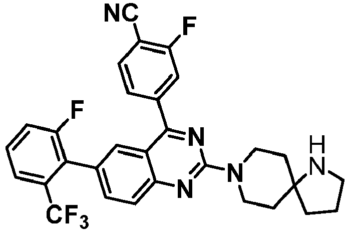 | 78 | 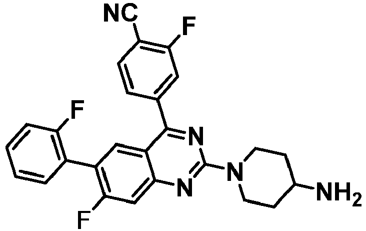 |
| 79 | 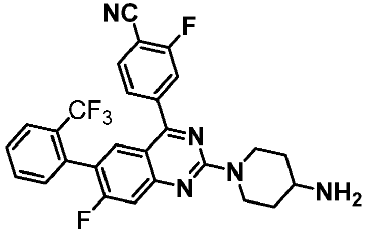 | 80 | 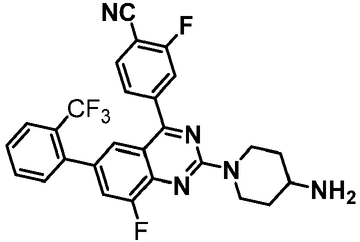 |
| 81 | 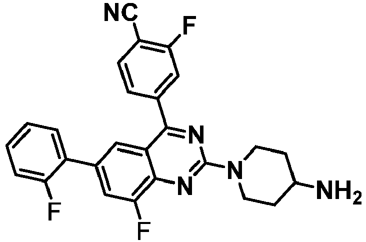 | 82 | 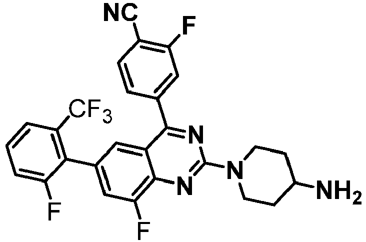 |
| 83 | 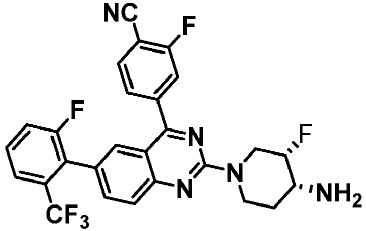 | 84 | 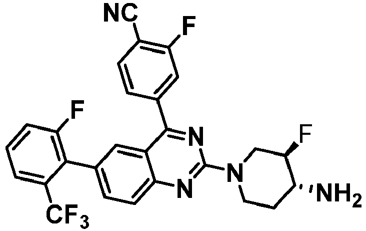 |
| 85 | 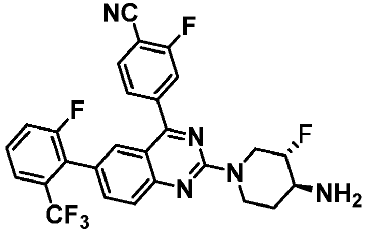 | 86 | 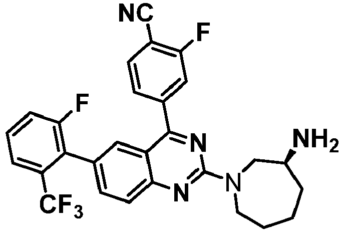 |
| 87 | 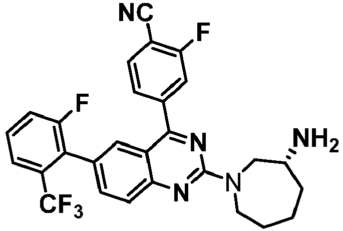 | 88 | 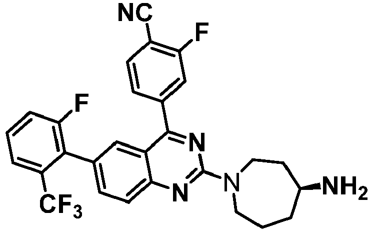 |
| 89 | 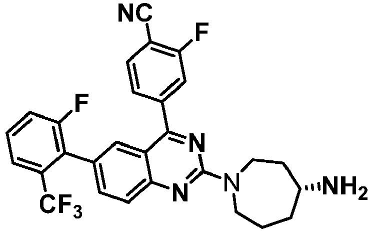 | 90 | 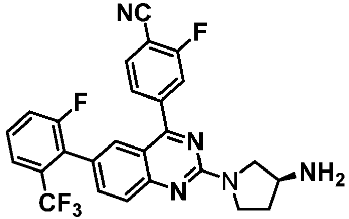 |
| 91 | 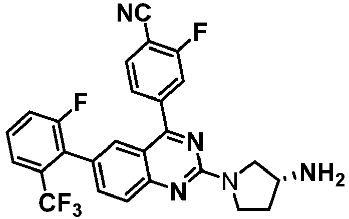 | 92 | 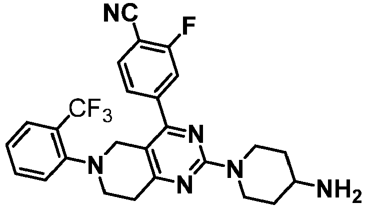 |
| 93 | 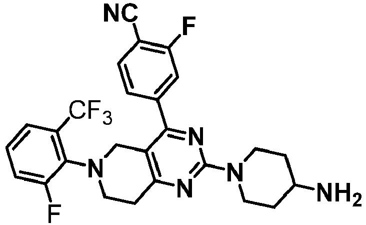 | 94 | 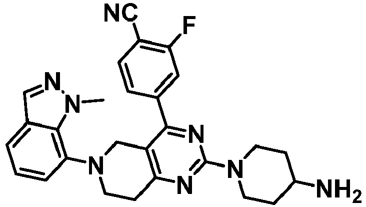 |
| 95 | 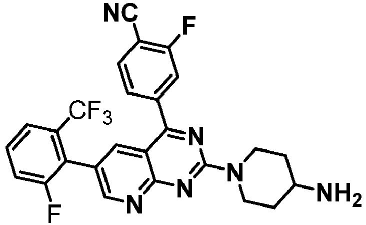 | 96 | 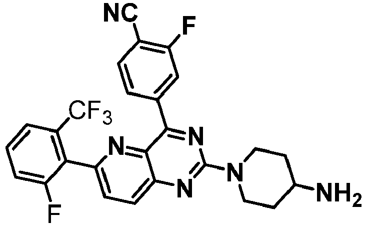 |
| 97 | 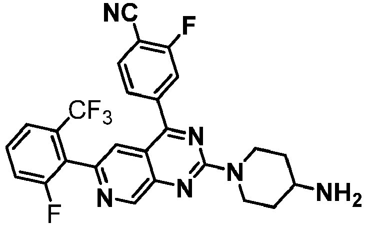 | 98 | 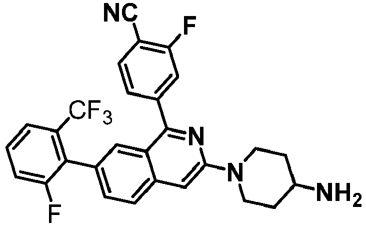 |
| 99 | 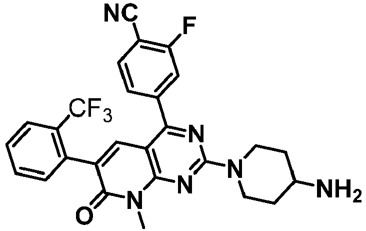 | | |

The present invention provides a pharmaceutical composition comprising a therapeutically effective amount of substance A and pharmaceutically acceptable carriers (pharmaceutical excipients); wherein the substance A is one or more of the aromatic heterocyclic compound of Formula I as described above, a pharmaceutically acceptable salt thereof, a stereoisomer, a racemate, a prodrug, a co-crystalline complex, a hydrate, or a solvate thereof.

The present invention also providesa use of substance A in the preparation of a LSD1 inhibitor, wherein the substance A is one or more of the aromatic heterocyclic compounds of Formula I as described above, a pharmaceutically acceptable salt thereof, a stereoisomer, a racemate, a prodrug, a co-crystalline complex, a hydrate, or a solvate thereof. In the use, the LSD1 inhibitor can be used in mammalian organisms in vivo; it can also be used in in vitro of the organisms, mainly for experimental purposes, e.g.: as a standard or control sample to provide comparison, or manufactured as a kit according to conventional method in the field, to provide rapid assay of the inhibitory effect against LSD1.

The present invention also provides a use of substance A in the preparation of a drug, wherein the substance A is one or more of the aromatic heterocyclic compounds of Formula I as described above, a pharmaceutically acceptable salt thereof, a stereoisomer, a racemate, a prodrug, a co-crystalline complex, a hydrate, or a solvate thereof; the substance A is ofa therapeutically effective amount; and the drug is used for the treatment or prevention of LSD1-mediated disease.

The present invention also provides a use of substance A in the preparation of a drug, and the drug is used for the treatment or prevention of cancer; wherein the substance A is one or more of the aromatic heterocyclic compounds of Formula I as described above, a pharmaceutically acceptable salt thereof, a stereoisomer, a racemate, a prodrug, a co-crystalline complex, a hydrate, or a solvate thereof; and the substance A is ofa therapeutically effective amount

The present invention also provides a method for inhibiting LSD1, comprising administering to a patient a therapeutically effective amount of substance A; wherein the substance A is one or more of the aromatic heterocyclic compounds of Formula I as described above, a pharmaceutically acceptable salt thereof, a stereoisomer, a racemate, a prodrug, a co-crystalline complex, a hydrate, or a solvate thereof.

The present invention also provides a method for treating or preventing LSD1-mediated diseases, comprising administering to a patient a therapeutically effective amount of substance A; wherein the substance A is one or more of the aromatic heterocyclic compounds of Formula I as described above, a pharmaceutically acceptable salt thereof, a stereoisomer, a racemate, a prodrug, a co-crystalline complex, a hydrate, or a solvate thereof.

The present invention also provides a method fort reating or preventing cancer, comprising administering to a patient a therapeutically effective amount of substance A; wherein the substance A is one or more of the aromatic heterocyclic compounds of Formula I as described above, a pharmaceutically acceptable salt thereof, a stereoisomer, a racemate, a prodrug, a co-crystalline complex, a hydrate, or a solvate thereof.

The LSD1-mediated disease is cancer.

The cancer as described above isnon-limitingly selected from one or more of bladder cancer, breast cancer, prostate cancer, pancreatic cancer, thyroid cancer, liver cancer, colon cancer, rectal cancer, esophageal cancer, cervical cancer, ovarian cancer, acute leukemia, acute lymphoblastic leukemia, acute myeloid leukemia (mononucleated, myeloblastic, hemangiosarcoma, astrocytoma, myelomonocyte, and promyelocytic), acute T-cell leukemia, chondrosarcoma, chronic lymphocytic leukemia, chronic myeloid (myelogenous) leukemia, large cell lymphoma, fibrosarcoma, testicular germ cell carcinoma, neuroglioma, lymphoma (hodgkin's or non-hodgkin's), multiple myeloma, lymph cancer, myeloma, neuroblastic tumor, neuroblastoma, non-small cell lung cancer, and small cell lung cancer.

In addition to the foregoing, when used in the specification and claims of this application, the following terms have the meanings shown below, unless otherwise specifically indicated.

In the present application, when the carbon atom with "*" is a chiral carbon atom, it is in R configuration, S configuration, or a mixture thereof.

The term "more" means 2, 3, 4 or 5.

The term "pharmaceutically acceptable salt" refers to a salt prepared from compounds of the present invention with relatively non-toxic, pharmaceutically acceptable acids or bases. When the compound of the present invention contains a relatively acidic functional group, base addition salts can be obtained by contacting the neutral form of such compound with a sufficient amount of pharmaceutically acceptable base in pure solution or in a suitable inert solvent. Pharmaceutically acceptable base addition salts include, but are not limited to: lithium salts, sodium salts, potassium salts, calcium salts, aluminum salts, magnesium salts, zinc salts, bismuth salts, ammonium salts, diethanolamine salts. When the compound of the present invention contains relatively basic functional groups, acid addition salts can be obtained by contacting the neutral form of such compound with a sufficient amount of pharmaceutically acceptable acid in a pure solution or in a suitable inert solvent. Pharmaceutically acceptable acids include inorganic acids, organic acids (e.g., trifluoroacetic acid, hydrochloric acid). Please refer, in particular, Berge et al., "Pharmaceutical Salts", Journal of Pharmaceutical Science 66: 1-19 (1977), or, Handbook of Pharmaceutical Salts: Properties, Selection, and Use (P. Heinrich Stahl and Camille G. Wermuth, ed ., Wiley-VCH, 2002).

As used herein, the compound of Formula I of the present invention may contain one or more chiral centers and can be present in different optically active forms. When a compound contains a chiral center, the compound includes enantiomers. The present invention includes both isomers and mixtures of the two, such as racemic mixtures. The enantiomers can be resoluted by methods known in the art, such as crystallization as well as methods such as chiral chromatography. Diastereoisomers can exist when the compound of formula I contains more than one chiral center. The present invention includes resoluted optically pure specific isomers as well as mixtures of diastereoisomers. The diastereoisomer can be resoluted by methods known in the art, such as crystallization and preparative chromatography. The term "stereoisomer" includes both conformational and configurational isomer, wherein configurational isomers include primarily cis-trans isomers and optical isomers. The compound of the present invention may exist in the form of stereoisomers and thus cover all possible stereoisomeric forms including, but not limited to, cis-trans isomers, enantiomers, diastereoisomers, atropisomers and the like, and the compound of the present invention may also exist in the form of any combinations or any mixtures of the foregoing stereoisomers, for example, a mixture of mesomer, racemate, andatropisomer in the same amount, and the like. Examples include single enantiomer, single diastereoisomer or a mixture of the above, or single atropisomer or a mixture thereof. When the compound of the present invention contains an olefinic double bond, they include cis and trans isomers, as well as any combinations thereof, unless otherwise specified. The atropisomers of the present invention are stereoisomers based on axial or planar chirality resulting from restricted intramolecular rotation. With respect to atropisomers of the present invention, wherein the compound has the structure of Formula I, or wherein the compound of Formula I has an isomer arising from asymmetric carbon, etc., which denotes eitherof thepair of atropisomers present in each isomeric compound. And as a drug, the atropisomerwithsuperior activity is preferred. These stereoisomers can be isolated, purified and enriched by asymmetric synthesis methods or chiral separation methods (including, but not limited to, thin-layer chromatography, rotary chromatography, column chromatography, gas chromatography, high-pressure liquid chromatography, etc.), and can be obtained by chiral resolution, such as by bonding or salting with other chiral compounds. The term "single stereoisomer" means that one stereoisomer of the compound of the invention contains no less than 95% by mass of all stereoisomers of the compound. The compounds of Formula I have optical isomers originating from asymmetric carbons, axial asymmetry, etc., and the single isomer can be obtained by methods known in the art, such as crystallization or chromatography (e.g., chiral chromatography), if necessary.

As previously described, the present invention providescompounds shown in the structures above-described, or cis-trans isomers, mesomers, racemates, enantiomers, diastereomers, atropisomers, or mixtures thereof, wherein the "mixtures thereof" comprise a mixture in any form between any of the foregoing stereoisomers (e.g., cis-trans isomer, enantiomer, diastereoisomer, atropisomer) and/or a mixture (mesomers, racemate) of any of the foregoing mixture, such as a mixture of cis-trans isomers, a mixture of enantiomers and diastereoisomers, a mixture of diastereoisomers, a mixture of atropisomers, or a mixture of cis-trans isomers and racemates, a mixture of enantiomers and diastereoisomer mixture, a mixture of atropisomers and diastereoisomer mixture, etc.

The compounds of Formula I, stereoisomers thereof and pharmaceutically acceptable salts thereof are intended to cover any isotopically labeled (or "radiolabeled") variant of the compound of Formula I, a pharmaceutically acceptable salt thereof, a stereoisomer, a racemate, a prodrug, a co-crystalline complex, an hydrate or a solvate thereof. Such variants are compounds of formula I, pharmaceutically acceptable salts thereof, stereoisomers, racemates, prodrugs, co-crystalline complexes, hydrates or solvates in which one or more atoms have been replaced by atoms having an atomic mass or mass number different from those normally found in nature. The radionuclide used will depend on the specific application of the radiolabeled variant. For example, for in vitro receptor labeling and competition assays, ³H or ¹⁴C are often useful. For radiographic applications,¹¹C or ¹⁸F are often useful.

Specific isotopic variants of the compound of the present invention, in particular those in which one or more radioisotopes have been incorporated, can be useful, for example, for examining the mechanism of action or the distribution of the active fraction in vivo; compounds labeled with the ³H or ¹⁴C isotopes are particularly suitable for this purpose due to their relative ease of manufactureand detectability. In addition, the incorporation of isotope such as deuterium can yield particular therapeutic benefits due to the better metabolic stability of the compound, e.g., by prolonging the half-life in vivo or by lowering the required effective dose; such modifications of the compounds of the present invention may therefore also constitute preferred embodiments of the present invention in a number of cases. Isotopic variants of the compound of the present invention may be prepared by methods known to those skilled in the art, e.g., by the methods described hereinafter and in the operational embodiments, or by the use of specific reagents and/or starting compounds with corresponding isotopic modifications.

For the purposes of this application, "pharmaceutical composition" means a formulation comprising a compound of the invention andmeida commonly accepted in the art for delivery of a biologically active compound to a mammal (e.g., a human). The meida includes pharmaceutically acceptable carriers. The purpose of the pharmaceutical composition is to facilitate the delivery of drugs to an organism and to facilitate the absorption of the active ingredient and thus producing its biological activity.

For the purposes of this application, "pharmaceutically acceptable" means a substance (e.g., a pharmaceutical excipient) that does not affect the biological activity or property of the compounds of the present invention and is relatively non-toxic, i.e., the substance can be administered to an individual without causing an adverse biological reaction or interacting in an undesirable manner with any of the components included in the composition.

The term "pharmaceutical excipients" or "pharmaceutically acceptable carriers" refers to excipients and additives used in the manufacture of pharmaceutical products and in the formulation of prescriptions, and are all substances, other than the active ingredient, that are included in a pharmaceutical preparation. See Pharmacopoeia of the People's Republic of China (2015 Edition), Part IV, or Handbook of Pharmaceutical Excipients (Raymond C Rowe, 2009 Sixth Edition). Excipients are mainly used to provide a safe, stable and functional pharmaceutical composition, and can also provide methods to enable the active ingredient to dissolve at a desired rate after the subject receives administration, or to promote the absorption of the active ingredient effectively after the subject receives the composition. The pharmaceutical excipient may be an inert filler or provide a function such as stabilizing the overall pH value of the composition or preventing degradation of the active ingredients of the composition. The pharmaceutical excipients may include one or more of the following excipients: binders, suspending agents, emulsifiers, diluents, fillers, granulating agents, adhesives, disintegrants, lubricants, anti-adhesive agents, glidants, wetting agents, gelling agents, absorption retardants, dissolution inhibitors, enhancers, adsorbents, buffers, chelating agents, preservatives, colorants, flavoring agents, and sweeteners.

The pharmaceutical composition of the present invention may be prepared according to the disclosure using any method known to those skilled in the art. For example, conventional mixing, dissolution, granulation, emulsification, grinding, encapsulation, embedding or freeze-drying processes.

When used as drug, the compound of Formula I, a pharmaceutically acceptable salt thereof, a stereoisomer, a racemate, a prodrug, a co-crystalline complex, a hydrate, or a solvate thereof may be administered in any form of a pharmaceutical composition. These compositions may be prepared according to methods well known in the field of pharmacy and may be administered in a variety of routes, depending on the needs for local or systemic treatment and the area to be treated. Administration may be topical (including epidermal and transdermal, ocular and mucosal, including intranasal, vaginal and rectal delivery), pulmonary (e.g., inhalation or blowing in via powder or aerosol, including via a nebulizer; endotracheal or intranasal), oral (solid and liquid formulations), or parenteral administration forms. Examples of solid oral formulations include, but are not limited to, powders, capsules, caplets, soft capsule formulations, and tablets. Examples of liquid formulations for oral or mucosal administration include, but are not limited to, suspensions, emulsions, elixirs and solutions.Examplesof formulations for topical administration include, but are not limited to, emulsions, gels, ointments, creams, patches, pastes, foams, lotions, drops, or serum formulations. Examples of formulations for parenteral administration include, but are not limited to, solutions for injection, dry formulations that can be dissolved or suspended in a pharmaceutically acceptable carrier, suspensions for injection, and emulsions for injection. Pharmaceutical compositions and formulations for external administration may include transdermal patches, ointments, lotions, ointments, gels, drops, suppositories, sprays, liquids and powders. Examples of other suitable formulations of the described pharmaceutical compositions include, but are not limited to, eye drops and other ophthalmic formulations; aerosols: such as nasal sprays or inhalers. Oral administration may include dosage forms formulated for once daily or twice daily (BID) administration. Parenteral administration includes intravenous, intra-arterial, subcutaneous, intraperitoneal, intramuscular or injection or infusion; or intracranial such as intrathecal or intracardiac administration. Parenteral administration may be in the form of a single push dose or may be by a continuous infusion pump. Conventional pharmaceutical carriers, water, powdered or oily bases, thickeners, and the like may be necessary or desired. Pharmaceutical compositions comprising the present invention may also be controlled or delayed release dosage forms (e.g., liposomes or microspheres).

The term "treatment" refers to curative therapies or palliative measures. When involving a specific condition, treatment means: (1) alleviating one or more biological manifestations of a disease or condition, (2) interfering with (a) one or more points in a biological cascade that causes or contributes to a condition or (b) one or more biological manifestations of a condition, (3) ameliorating one or more symptoms, effects, or side-effects related to a condition, or to a condition or its treatment, or (4) slowing the progression of a condition or one or more biological manifestations of a condition. "Treatment" may also mean prolongation of survival compared to the expected survival without treatment.

The term "prevention" refers to a reduction in the risk of acquiring or developing a disease or disorder.

The term "therapeutically effective amount" refers to an amount of a compound that, when administered to a patient, is sufficient to effectively treat the disease or condition described herein. The "therapeutically effective amount" will vary depending on the compound, the condition and its severity, and the age of the patient to be treated, but can be adjusted as needed by those skilled in the art.

The term "patient" refers to any animal, preferably mammal, more preferably human, that is to be or has already been administered the compound or composition according to embodiments of the invention. The term "mammal" includes any mammal. Examples of mammal include, but are not limited to, cows, horses, sheep, pigs, cats, dogs, mice, rats, rabbits, guinea pigs, monkeys, humans, etc., with humans being the most preferred.

Unless otherwise indicated, the following definitions used herein should be applied. For the purposes of this invention, the chemical elements are consistent with the CAS version of the Periodic Table, and the Handbook of Chemistry and Physics, 75th Edition, 1994. In addition, general principles of organic chemistry may be found in "Organic Chemistry", Thomas Sorrell, University Science Books, Sausalito:1999, and "March's Advanced Organic Chemistry "by Michael B. Smith and Jerry March, John Wiley & Sons, New York: 2007, the entire contents of which are incorporated herein by reference.

In this specification, the group and its substituents can be selected by the skilled person in the art to provide a stable structural moiety and compound. When the substituent is described by the conventional chemical formula written from left to right, the substituent also includes the chemically equivalent substituent obtained when written from right to left.

Some chemical groups defined in this article are preceded with a simplified notation to represent the total number of carbon atoms present in this group. For example, C₁-C₆ alkyl refers to an alkyl having a total of 1, 2, 3, 4, 5 or 6 carbon atoms as defined below. The total number of carbon atoms in the simplified notation does not include carbon that may be present in the substituents of the group.

In this article, the numerical ranges defined in the substituent, such as 0 to 4, 1-4, 1 to 3, etc., indicate integers in this range, for example, 1-6 is 1, 2, 3, 4, 5, 6.

In addition to the foregoing, when used in the specification and claims of this application, the following terms have the meanings indicated below, unless otherwise specifically indicated.

The term "include" is an open-ended expression, i.e., it includes what is specified in the present invention, but does not exclude other aspects.

The term "substituted" means that any one or more hydrogen atoms on a particular atom are substituted by substituents, as long as the valence state of the particular atom is normal and the substituted compound is stable.

In general, the term "substituted" means that one or more hydrogen atoms in a given structure are substituted by a specific substituent. Further, when the group is substituted by one or more substituents, the substituents are independent of each other, that is, the one or more of the substituents may be different from each other or the same. Unless otherwise indicated, a substituent group can be substituted at each of the substitutable positions of the group being substituted. When more than one position in the given structural formula can be substituted by one or more substituents selected from specific groups, then the substituents can be substituted equally or differently at each position.

In each part of this specification, the substituents of the compounds disclosed in the present invention are disclosed according to the group type or range. In particular, the present invention includes each independent secondary combination of each member of these group types and ranges. The term "Cₓ-C_{y} alkyl" refers to straight or branched saturated hydrocarbons containing x to y carbon atoms. For example, the terms "C₁-C₆ alkyl" or "C₁₋₆ alkyl" refer specifically to the independently disclosed methyl, ethyl, C₃ alkyl, C₄ alkyl,C₅ alkyl, and C₆ alkyl; "C₁₋₄ alkyl" refers specifically to the independently disclosed methyl, ethyl, C₃alkyl (i.e., propyl, including n-propyl and isopropyl), C₄ alkyl (i.e., butyl, including n-butyl, isobutyl, sec-butyl and tert-butyl).

As used herein, the terms "moiety", "structural moiety", "chemical moiety", "group", "chemical group" refer to a specific fragment or functional group in a molecule. Chemical moiety are usually considered as chemical entities embedded or attached to molecules.

When no indication is given in the enumerated substituents as to the atom through which they are attached to the general formula of the chemical structure (including, but not specifically mentioned, in the compounds), such substituents may be bonded through any of their atom. Combinations of substituent groups and/or variants thereof are permitted only if such combination results in stable compounds.

When avariable (e.g., R^{1a}) occurs multiple times in the definition of a compound, the definition of that variable at each position where it occurs is independent of the definitions of the remaining positions where it occurs, and their meanings are independent of each other and do not affect each other. Thus, if a group is substituted by 1, 2 or 3 R^{1a} groups, i.e., the group may be substituted by up to 3 R^{1a}, the definition of R^{1a} at that position is independent of the definitions of R^{1a} at the remaining positions. Furthermore, combinations of substituents and/or variables are only allowed when the combination results in a stable compound.

When a listed group is not explicitly identified as having a substituent, such group is referred to only as unsubstituted. For example, when "C₁-C₄ alkyl" is not preceded by the limitation "substituted or unsubstituted", it refers only to "C₁-C₄ alkyl" itself or "unsubstituted C₁-C₄ alkyl".

In various parts of the present invention, the linking substituents are described. When the structure clearly requires a linking group, the Markush variable enumerated for the group should be understood as a linking group. For example, if the structure requires a linking group and the definition of the Markush group for this variable enumerates "alkyl", it should be understood that the "alkyl" represents an alkylene group for linkage.

In some specific structures, when an alkyl group is clearly represented as a linking group, the alkyl group represents a linking alkylidene group, e.g., the C₁-C₆ alkyl in the group "halogenated-C₁₋C₆ alkyl" should be understood as C₁₋C₆alkylidene.

The term "halogen" means fluorine, chlorine, bromine or iodine, in particular F or Cl.

For the purposes of the present application, the term "hydrocarbyl", as a group or a part of another group (e.g., as used in groups such as halogenated hydrocarbyl), means a saturated or unsaturated branched and straight aliphatic hydrocarbon group comprising a specified number of carbon atoms, consisting only of carbon and hydrogen atoms, and connected to the rest of the molecule through a single bond. Examples include "alkyl", "alkenyl" or "alkynyl".

For the purposes of the present application, the term "alkyl", as a group or a part of another group (e.g., as used in groups such as halogenated alkyl), means a saturated branched and straight aliphatic hydrocarbon group comprising a specified number of carbon atoms, consisting only of carbon and hydrogen atoms, and connected to the rest of the molecule by a single bond, for example, having 1 to 10(preferably, 1 to 6, more preferably 1 to 4) carbon atoms. Wherein propyl is C₃ alkyl (including isomers, e.g., n-propyl or isopropyl); butyl is C₄ alkyl (including isomers, e.g., n-butyl, sec-butyl, isobutyl, or tert-butyl); pentyl is C₅ alkyl (including isomers, e.g., n-pentyl, 1 -methyl-butyl, 1- ethyl-propyl, 2-methyl-1 -butyl, 3-methyl-1-butyl, isoamyl, tert-amyl, or neo-amyl); hexyl is C₆ alkyl (including isomers such as n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl,2-methylpentyl, 3-methylpentyl, 4-methylpentyl, 2,3-dimethylbutyl). Examples include, but are not limited to, methyl, ethyl, n-propyl, isopropyl, n-butyl, isobutyl, sec-butyl, tert-butyl, n-pentyl, 2-methylbutyl, 2,2-dimethylpropyl, n-hexyl, n-heptyl, 2-methylhexyl, 3-methylhexyl, n-octyl, nonyl and decyl, and other similar alkyls thereof.

For the purposes of this application, the term "alkylidene", as a group or a part of another group, denotes a saturated divalent hydrocarbon group obtained by removing two hydrogen atoms from a saturated straight or branched hydrocarbon group; i.e., one of the hydrogens in the alkyl group is replaced, and the alkyl group is defined as described above. Examples of alkylidene group include methylene (-CH₂-), ethylene {including-CH₂CH₂- or-CH(CH₃)-}, isopropylene {including-CH(CH₃)CH₂- or-C(CH₃)₂-}, and the like.

For the purposes of this application, the term "alkenyl", as a group or a part of another group, refers to a straight or branched hydrocarbon chain group having at least one double bond, comprising only of carbon and hydrogen atoms, having, for example, from 2 to 12 (preferably from 2 to 8, more preferably from 2 to 6, and most preferably from 2 to 4) carbon atoms and connected to the rest of the molecule by a single bond, including for example, but not limited to, vinyl, n-propenyl, isopropenyl, n-butenyl, isobutenyl, sec-butenyl, tert-butenyl, n-pentenyl, 2-methylbutenyl, 2,2-dimethylpropenyl, n-hexenyl, heptenyl, 2-methylhexenyl, 3-methylhexenyl, octenyl, nonenyl, and decenyl, and the like.

For the purposes of this application, the term "alkynyl", as a group or a part of another group, means a straight or branched hydrocarbon chain group having at least one triple bond, comprising only of carbon and hydrogen atoms, having, for example, from 2 to 12 (preferably from 2 to 8, more preferably from 2 to 6, and most preferably from 2 to 4) carbon atoms and connected to the rest of the molecule by a single bond, including, for example, but not limited to, ethynyl, n-propynyl, isopropynyl, n-butynyl, isobutynyl, sec-butynyl, tert-butynyl, n-pentynyl, 2-methylbutynyl, 2,2-dimethylpropynyl, n-hexynyl, heptynyl, 2-methylhexynyl, 3-methylhexynyl, octynyl, nonynyl, and decynyl, and the like.

For the purposes of this application, the term "cyclic hydrocarbyl", as a group or as a part of anothergroup, means a saturated or unsaturated (non-aromatic) monocyclic or polycyclic(e.g., a bicyclic, tricyclic or more cyclic bridged, fused (fuse-ring), or spiro ring system) carbocyclic substituent, which linked to the rest of the molecule througha single bond via any suitable carbon atom. Examples include 3-12-membered cyclic hydrocarbylhaving from 3 to 12 carbon atoms, another example is 3-12-membered cycloalkyl or cycloalkenyl, and further examples include C₃₋₇ monocyclic hydrocarbyl, C₄₋₁₂ bridged or fused cyclichydrocarbyl, and C₇₋₁₂spirocyclic hydrocarbyl. In some embodiments, a 3-10-membered cyclic hydrocarbylhaving 3 to 10 carbon atoms is preferred, also for example a 3-10-membered cycloalkyl having 3 to 10 carbon atoms, preferably a 3-6-membered cycloalkyl having 3 to 6 carbon atoms; or a 3-10-membered cycloalkenyl having 3 to 10 carbon atoms, preferably a 3-6-membered cycloalkenyl having 3 to 6 carbon atoms.In a certain embodiment, exemplary monocyclic cycloalkyl includescyclopropyl, cyclobutyl, cyclopentyl, cyclohexyl or cycloheptyl.In some embodiments, "cycloalkenyl" is a monocyclic, unsaturated carbocycloalkenyl having 5 to 6 ring atoms ("5- to 6-membered cycloalkenyl"). The term includes, but is not limited to, cyclopentenyl (e.g., 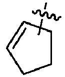 ), cyclopentadienyl (e.g., 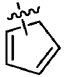 ), cyclohexenyl (e.g., 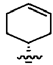 ), or cyclohexadienyl, and stereoisomers thereof.

For the purposes of this application, the term "heterocyclyl", as a group or as a part of another group, means a stable, saturated or unsaturated (non-aromatic) heterocyclic hydrocarbylwith a monocyclic or polycyclic ring (e.g., a bicyclic, tricyclic or polycyclic bridged, fused (fuse-ring), or spiro ring system), consisting of carbon atoms, as well as one or more heteroatoms selected from O, S, or N or heteroatom groups (e.g., C(=O), S(=O), S(=O)₂), and is connected to the rest of the molecule through a single bond via any suitable carbon atom.Examples include a3-10-membered heterocyclyl having 2 to 9 carbon atoms, preferably a 3-6-membered heterocyclyl having 3 to 5 carbon atoms, another example is a 3-10-membered heterocycloalkylhaving 2 to 9 carbon atoms, preferably a 3-6-membered heterocycloalkylhaving 3 to 5 carbon atoms, or a 3-10-membered heterocycloalkenyl having 2 to 9 carbon atoms, preferably a 3-6-membered heterocycloalkenyl having 3 to 5 carbon atoms. The ring system of the bicyclic heterocyclyl may include one or more heteroatoms in one or both rings; and is saturated or unsaturated. The heterocyclyl may be connected to the rest of the molecule through a single bond via carbon atom; in heterocyclyl comprising one or more nitrogen atoms, the point of attachment may be carbon or nitrogen atom; or, connected to the rest of the molecule ring in a paracyclic manner, as long as the valence permits.In some embodiments, "heterocycloalkyl" is 5- to 7-membered monocyclic heterocycloalkyl, 6- to 8-membered fused heterocycloalkyl, 6- to 8-membered bridgdheterocycloalkyl, or 7- to 10-membered spiroheterocycloalkyl. Exemplary 3-membered heterocycloalkyl includes, but isnot limited to, aziridinyl, oxirane group, and thiiranyl, or stereoisomers thereof; exemplary 4-membered heterocycloalkyl includes, but isnot limited to, azetidinyl, epoxy propanoxy, and thietanyl, or structural isomers and stereoisomers thereof; and exemplary 5-membered heterocycloalkyl includes, but isnot limited to, tetrahydrofuryl, tetrahydrothienyl, pyrrolidinyl 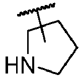 (e.g., 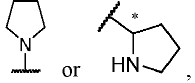 and e.g., 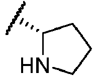 ), thiazolidinyl, isothiazolidinyl, oxazolidinyl, isoxazolidinyl, imidazolidinyl, pyrazolidinyl, dioxolan, oxathiofuranyl, dithiofuryl, or structural isomers and stereoisomers thereof. Exemplary 6-membered heterocycloalkyl includes, but isnot limited to, piperidinyl 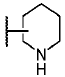 (e.g., 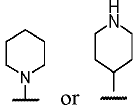 ), tetrahydropyranyl, sulfurized cyclopentyl, morpholinyl, thiomorpholinyl, dithiane, dioxane, piperazinyl 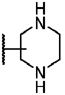 (e.g., 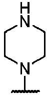 ), triazinyl, 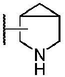 (e.g., 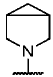 ), or structural isomers and stereoisomers thereof. Exemplary 7-membered heterocycloalkyl includes, but isnot limited to, 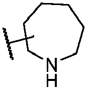 (e.g., 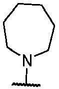 ), 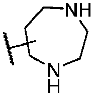 (e.g., 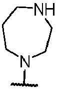 ), 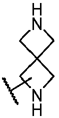 (e.g., 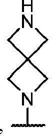 ), 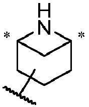 (e.g., 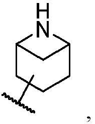 also e.g., 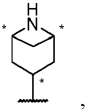 also e.g., 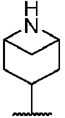 ), or 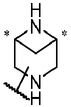 (e.g., 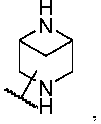 also e.g., 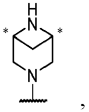 also e.g., 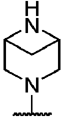 ), or isomersand stereoisomers thereof. Exemplary 8-membered heterocycloalkyl includes, but isnot limited to, 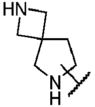 (e.g., 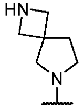 ), 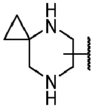 (e.g., 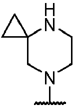 ), 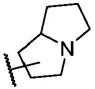 (e.g., 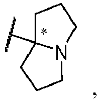 and e.g., 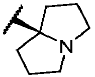 ), 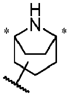 (e.g., 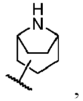 and also e.g., 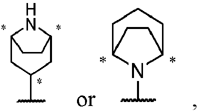 and also e.g., 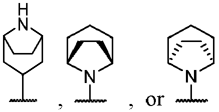 ), or 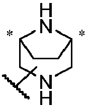 (e.g., 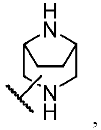 and also e.g., 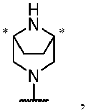 and also e.g., 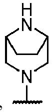 ), or isomersand stereoisomers thereof. Exemplary 9-membered heterocycloalkyl includes, but isnot limited to, 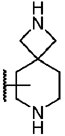 (e.g., 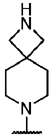 ) or 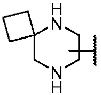 (e.g., 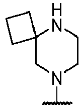 ), 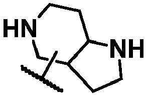 (e.g., 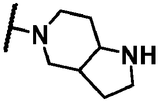 ) or isomersand stereoisomers thereof. Exemplary 10-membered heterocycloalkyl includes, but isnot limited to, 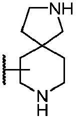 (e.g., 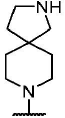 ) or 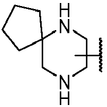 (e.g., 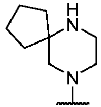 ), or isomersand stereoisomers thereof. ("*" indicates that the carbon atom with "*" is a chiral carbon atom, and it is in R configuration, S configuration, or a mixture thereof). In some embodiments, "heterocycloalkenyl" is 5- to 7-membered monocyclic heterocycloalkenyl, 6- to 8-membered fused heterocycloalkenyl, 6- to 8-membered bridged heterocycloalkenyl, or 7- to 10-membered spiroheterocycloalkenyl. Examples of heterocycloalkenyl include, but are not limited to, pyranyl, 2,3-dihydropyrrolyl, 2,3-dihydrofuryl, 1,2,3,4-tetrahydropyridyl, 1,2,3,6-tetrahydropyridyl, 3,4-dihydro-2H-pyranyl, 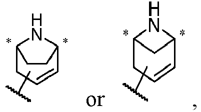 e.g., 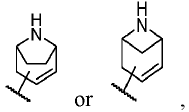 and further e.g., 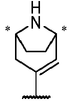 or 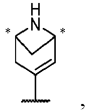 and further e.g., 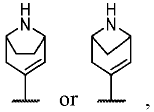 wherein "*" indicates that the carbon atom with "*" is a chiral carbon atom, and it is in R configuration, S configuration, or a mixture thereof.

In the present application, the term "aryl", as a group or a part of another group, means a conjugated hydrocarbon ring system group satisfying the 4n+2 rule. For example, a conjugated hydrocarbon ring group with 6 to 20 carbon atoms (preferably with 6 to 10 carbon atoms) satisfying the 4n+2 rule. For the purposes of the present invention, the aryl may be monocyclic, bicyclic, tricyclic or polycyclic ring system, and may also be fused with cycloalkyl, cycloalkenyl, heterocycloalkyl, or heterocycloalkenyl, as defined above, provided that the aryl is connected to the rest of the molecule via the atoms on the aromatic ring througha single bond. In a certain embodiment, the term "aryl" refers to an aromatic group consisting of carbon atoms, with each ring being aromatic. Examples of aryl group include, but are not limited to, phenyl, naphthyl (e.g., 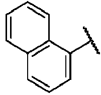 ).

In the present application, the term "heteroaryl", as a group or a part of another group, means a conjugated cyclic group having carbon atoms and at least one heteroatom selected from O, S, and N on the ring. For example, a 5- to 14-membered conjugated ring group having from 1 to 13 carbon atoms (preferably from 2 to 9 carbon atoms) and from 1 to 6 heteroatoms selected from O, N and S on the ring. Unless otherwise specifically indicated in this specification, the heteroaryl may be monocyclic, bicyclic, tricyclic or polycyclic ring system, and may also be fused with cycloalkyl, cycloalkenyl, heterocycloalkyl, or heterocycloalkenyl, as defined above, provided that the heteroaryl is connected to the rest of the molecule via the atoms on the aromatic ring througha single bond. For the purposes of the present invention, the heteroaryl preferably includes a stable 5- to 14-membered aromatic group comprising from 1 to 5 heteroatoms selected from nitrogen, oxygen and sulfur, and further preferably a stable 5- to 10-membered aromatic group comprising from 1 to 5 heteroatoms selected from nitrogen, oxygen and sulfur. In a certain embodiment, the term "heteroaryl" refers to an aromatic group containing heteroatoms, with each ring being aromatic; preferably, an aromatic 5- to 6-membered monocyclic ring or 9- to 10-membered bicyclic ring containing 1, 2 or 3 heteroatoms independently selected from nitrogen, oxygen and sulfur. Examples of heteroaryl include, but are not limited to, thienyl, imidazolyl, pyrazolyl, thiazolyl, oxazolyl, diazolyl, oxadiazolyl, isoxazolyl, pyridinyl, pyrimidinyl, pyrazinyl,pyridazinyl, benzimidazolyl, benzopyrazolyl, indolyl, furanyl, pyrrolyl, triazolyl, tetrazolyl, triazinyl, indolizinyl, isoxazolyl, thiadiazolyl, isoindolyl, indazolyl, isoindazolyl, purinyl, quinolinyl, isoquinolinyl, diazanaphthalene, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzisothiazolyl, benzothiophenyl, oxatriazolyl, cinnolyl, quinazolinyl, indolizinyl, o-diazaphenanthridinyl, isoxazolyl, phenoxazinyl, phenothiazinyl, benzooxazolyl, or benzoisoxazolyl.

It should be understood that the singular form used in the present invention, such as "a", includes the plural designation unless otherwise specified.

The terms "one (or more)" or "one or more than two" mean one, two, three, four, five, six, seven, eight, nine or more.

Unless otherwise specified, the present invention uses conventional methods of mass spectrometry and elemental analysis, and the steps and conditions may refer to the conventional operation steps and conditions in the field.

Unless otherwise specified, the present invention uses standard nomenclature and standard laboratory steps and techniques for analytical chemistry, organic synthetic chemistry, and optics.In some cases, standard techniques are used for chemical synthesis, chemical analysis, and luminescent device performance testing.

Furthermore, it should be noted that, unless otherwise expressly stated, the description "...independently" as used in the present invention should be understood in a broad sense to mean that the individuals described are independent of each other and can be independently of each other for the same or different specific moieties.In more detail, the description "...independently" can either mean that the specific options expressed between the same symbols in different groups do not affect each other, or that the specific options expressed between the same symbols in the same groups do not affect each other.

It will be understood by those skilled in the art that, in accordance with the practice used in the art, the " 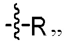 and 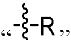 used in the structural formula of the group described herein means that the corresponding group R links to other fragments, groups in the compound through that site.

It will be understood by those skilled in the art that "=" as used in the structural formula of the groups described in this application denotes a single or double bond, according to the conventions used in the art.

Unless otherwise specified, all technical and scientific terms used herein have the standard meanings in the field of the claimed subject matter. Where more than one definition exists for a certain term, the definition herein shall prevail.

On the basis of not violating the common knowledge in the field, each of the above preferred conditions can be arbitrarily combined to obtain each preferred embodimentof the present invention.

The reagents and raw materials used in the present invention are commercially available.

The positive and progressive effects of the present invention lie in: As reversible LSD 1 inhibitors with a novel structural backbone, the compounds of the present invention have significant inhibitory activity against LSD1.

### Detailed Description of the Invention

Hereinafter, the present invention is further described by way of embodiments, but the present invention is not limited to the scope of the described embodiments. In the following examples, the experimental methods without specific conditions are usually in accordance with conventional conditions and methods, or in accordance with the commodity instructions.

In the present invention, room temperature refers to the ambient temperature, which is 10°C-35°C. Overnight means 8-15 hours. Reflux means solvent reflux temperature at atmospheric pressure.

The structure of the compoundisdetermined by nuclear magnetic resonance (NMR) or/and mass spectrometry (MS).The NMR shifts (δ) are given in units of 10-6 (ppm).The NMR determination is performed using a Bruker AVANCE-400 NMR instrument andthe solvents for determination aredeuterated dimethylsulfoxide (DMSO-J6), deuterated chloroform (CDCl₃), and deuterated methanol (CD₃OD), and the internal standard is tetramethylsilane (TMS). MS is determined using a Finnigan LCQ/Deca (ESI) mass spectrometer. High performance liquid chromatography (HPLC) analysis is performed using a Gilson-215 high pressure liquid chromatograph.

Thin Layer Chromatography silica gel plates use Yantai Huanghai HSGF 254 or Qingdao GF 254 silica gel plates, the size of silica gel plates used in Thin Layer Chromatography (TLC) is 0.15 mm -0.2 mm, and the size of silica gel plates of Thin Layer Chromatography (TLC) for the purification of the products is 0.4 mm-0.5 mm. Silica gel column chromatography generally uses 200-300 mesh silica gel from Yantai Huanghai as the carrier.

The known starting materials of the present disclosure can be synthesized using or according to methods known in the art, or can be purchased from companies such as Shanghai Haohong Biomedical Technology Co. and Bide Pharmatech Ltd.. The reactions are capable of being carried out under argon atmosphere or nitrogen atmosphere without special instructions in the examples.

In the examples, the reaction process is monitored by thin layer chromatography (TLC), the spreading agents used for the reaction, the system of eluents for column chromatography for the purification of the compounds and the system of spreading agents for TLC used include dichloromethane/methanol and petroleum ether/ethyl acetate, and the volume ratio of the solvents is adjusted according to the polarity of the compounds, and can also be adjusted by adding small amounts of basic or acidic reagents, such as triethylamine and acetic acid.

### Example 1

### Preparation of 4-(2-(4-aminopiperidin-1-yl)-6-(3-fluoro-4-methoxyphenyl)quinazolin-4-yl)-2-fluorobenzonitrile 1

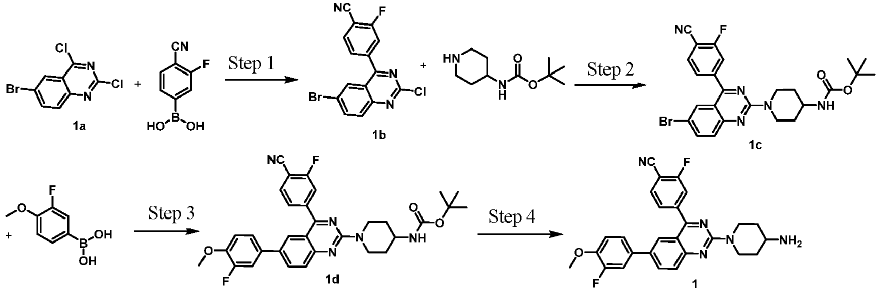

### Step 1

### 4-(6-bromo-2-chloroquinazolin-4-yl)-2-fluorobenzonitrile 1b

To a mixture of compound 6-bromo-2,4-dichloroquinazoline **1a** (4.0 g, 11.03 mmol, Shanghai Haohong Biomedical Technology Co., Ltd.), bis(triphenylphosphine)palladium(II) chloride (0.77 g, 1.10 mmol), aqueous sodium carbonate (2.0 M, 5 mL), and acetonitrile (40 mL) was added (4-cyano-3-fluorophenyl)boronic acid (1.82 g, 11.03 mmol Shanghai Haohong Biomedical Technology Co.). The reaction mixture was heated at 100 °C for 15 min with stirring under nitrogen protection. The reaction solution was then cooled and concentrated under reduced pressure, pulped with ethyl acetate, filtered and dried to give the title compound **1b** (3.74 g) (white solid), yield: 72%.

¹H NMR (400 MHz, DMSO-d₆) δ 8.28 (dd, *J*= 9.0, 2.1 Hz, 1H), 8.23 - 8.17 (m, 2H), 8.05 (d, *J*= 9.0 Hz, 1H), 7.97 (d, *J*= 9.8 Hz, 1H), 7.80 (d, *J*= 8.0 Hz, 1H).

### Step 2

### tert-butyl(1-(6-bromo-4-(4-cyano-3-fluorophenyl)quinazolin-2-yl)piperidin-4-yl)carbamate1c

Compound 4-(6-bromo-2-chloroquinazolin-4-yl)-2-fluorobenzonitrile **1b** (2.0 g, 5.5 mmol), diisopropylethylamine (1.82 mL, 11 mmol), and tert-butyl piperidin-4-ylcarbamate (1.65 g, 8.25 mmol Bide Pharmatech Ltd.) were dissolved in N,N-dimethylformamide (20 ml). The reaction mixture was stirred at room temperature overnight. The reaction solution was then concentrated under reduced pressure, added with 50mL of water and 50mL of ethyl acetate, mixed homogeneously and allowed to separate into layers. The aqueous phase was extracted with ethyl acetate (20mLX 3). The organic phases were combined, washed with saturated sodium chloride solution, dried over anhydrous sodium sulfate, and filtered; the filtrate was concentrated, and purified by silica gel column chromatography (EA:PE; 1:10 to 1:5) to give the title compound **1c** (2.48 g) (yellow solid), yield: 85%.¹H NMR (400 MHz, Chloroform-d) δ 7.81 (dd, J = 8.1, 6.3 Hz, 1H), 7.75 (d, J = 2.2 Hz, 1H), 7.70 (dd, J = 9.0, 2.2 Hz, 1H), 7.61 - 7.55 (m, 2H), 7.51 (d, J = 9.0 Hz, 1H), 4.84 (dt, J = 13.6, 3.9 Hz, 2H), 4.52 (d, J = 7.9 Hz, 1H), 3.76 (s, 1H), 3.14 (t, J = 12.5 Hz, 2H), 2.07 (dd, J = 13.1, 3.7 Hz, 2H), 1.45 (s, 9H), 1.39 (qd, J = 11.8, 5.4 Hz, 2H).

MS m/z (ESI):526.01 [M +1]⁺.

### Step 3

### tert-butyl

### (1-(4-(4-cyano-3-fluorophenyl)-6-(3-fluoro-4-methoxyphenyl)quinazolin-2-yl)piperidin-4-yl)carbatnate 1d

To a mixture of compound tert-butyl (1-(6-bromo-4-(4-cyano-3-fluorophenyl)quinazolin-2-yl)piperidin-4-yl)carbamate **1c** (50 mg, 0.09 mmol), [1,1'-bis(diphenylphosphino)ferrocene]palladium dichloride dichloromethane complex (7.3 mg, 0.009 mol), sodium carbonate (28.6 mg, 0.27 mmol) and tetrahydrofuran/water (5:1) (2 mL) wasadded (3-fluoro-4-methoxyphenyl)boronic acid (30.6 mg, 0.18 mmol Shanghai Haohong Biomedical Technology Co.). The reaction mixture was stirred at 70 °C for 16 h under nitrogen protection. The reaction solutionwas then cooled,concentrated under reduced pressure, added with 15 mL of water and 15 mL of ethyl acetate, mixed homogeneously, and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:8 to 1:4) to give the title compound **1d** (42 mg) as yellow solid, yield: 77%.¹HNMR (400 MHz, Chloroform-d) δ 7.87 (dd, J = 8.8, 2.1 Hz, 1H), 7.82 (dd, J = 8.1, 6.3 Hz, 1H), 7.74 - 7.68 (m, 2H), 7.68 - 7.59 (m, 2H), 7.30 - 7.22 (m, 2H), 7.02 (t, J = 8.8 Hz, 1H), 4.88 (d, J = 13.4 Hz, 2H), 4.52 (d, J = 7.9 Hz, 1H), 3.92 (s, 3H), 3.77 (s, 1H), 3.16 (t, J = 12.4 Hz, 2H), 2.09 (dd, J = 12.9, 3.8 Hz, 2H), 1.46 (s, 9H), 1.39 (qd, J = 11.7, 4.0 Hz, 2H).

MS m/z (ESI):571.15 [M + 1]⁺.

### Step 4

### 4-(2-(4-aminopiperidin-1-yl)-6-(3-fluoro-4-methoxyphenyl)quinazolin-4-yl)-2-fluorobenzonitrile 1

Compound tert-butyl (1-(4-(4-cyano-3-fluorophenyl)-6-(3-fluoro-4-methoxyphenyl)quinazolin-2-yl)piperidin-4-yl)carbatnate **1d** (42 mg) was dissolved in 2 mL of dichloromethane, and 2 mL of a solution of 4 N hydrogen chloride-1,4-dioxane was added. The reaction mixture was stirred at room temperature for 2 hours. The reaction solutionwas then concentrated under reduced pressure to give the title compound 1 hydrochloride (30 mg), as yellow solid, yield: 88%.¹H NMR (400 MHz, DMSO-*d*₆) δ 8.21 - 8.13 (m, 2H), 8.00 (d, J = 9.7 Hz, 2H), 7.84 (dd, J = 10.2, 1.9 Hz, 2H), 7.59 (dd, J = 12.9, 2.2 Hz, 1H), 7.47 (dd, J = 8.5, 2.2 Hz, 1H), 7.23 (t, J = 8.8 Hz, 1H), 4.93 (d, J = 13.3 Hz, 2H), 3.46 - 3.34 (m, 1H), 3.24 (t, J = 13.0 Hz, 2H), 2.11 (d, J = 12.1 Hz, 2H), 1.64 (q, J = 12.2 Hz, 2H).

### Example 2

### 4-(2-(4-Aminopiperidin-1-yl)-6-bromo-quinazofin-4-yl)-2-fluorobenzonitrile2

The second step product **1c** from the synthetic route of Example 1 was used as the raw material, and a method similar to the reaction of step 4 in Example 1 was used to give the title compound **2** hydrochloride (20 mg).MS m/z (ESI):426.05 [M + 1]⁺.

### Example 3

### 4-(2-(4-atninopiperidin-1-yl)-6-(3,5-dimethyfisoxazol-4-yl)quinazolin-4-yl)-2-fluorobenzonitrile 3

The title compound **3**hydrochloride (29 mg) was prepared using the synthetic route of Example 1 by replacing the raw material (3-fluoro-4-methoxyphenyl)boronic acid of step 3 with (3,5-dimethylisoxazol-4-yl)boronic acid. ¹H NMR (400 MHz, DMSO-*d*₆) δ 8.16 (t, J = 7.3 Hz, 1H), 8.00 - 7.93 (m, 1H), 7.88 (dd, J = 8.7, 1.9 Hz, 1H), 7.84 - 7.78 (m, 2H), 7.65 (d, J = 1.8 Hz, 1H), 4.20 (d, J = 5.2 Hz, 4H), 3.26 - 3.18 (m, 4H), 2.40 (s, 3H), 2.21 (s, 3H).

### Example 4

### 4-(2-(4-aminopiperidin-1-yl)-6-(5-methyl-1H-pyrazol-4-yl)quinazolin-4-yl)-2-fluorobenzonitrile 4

The title compound **4**hydrochloride (31mg) was prepared using the synthetic route of Example 1 by replacing the raw material (3-fluoro-4-methoxyphenyl)boronic acid of step 3 with (5-methyl-1H-pyrazol-4-yl)boronic acid.¹H NMR (400 MHz, DMSO-*d*₆) δ 8.17 (t, J = 7.3 Hz, 1H), 8.07 (s, 1H), 8.01 (t, J = 8.7 Hz, 2H), 7.90 (d, J = 8.8 Hz, 1H), 7.84 (d, J = 7.9 Hz, 1H), 7.71 (s, 1H), 4.91 (d, J = 13.2 Hz, 2H), 3.44 - 3.32 (m, 1H), 3.19 (t, J = 12.6 Hz, 2H), 2.37 (s, 3H), 2.09 (d, J = 12.2 Hz, 2H), 1.69 - 1.55 (m, 2H).

### Example 5

### 3-(2-(4-aminopiperidin-1-yl)-4-(4-cyano-3-fluorophenyl)quinazolin-6-yl)benzenesulfonamide 5

The title compound **5**hydrochloride (28mg) was prepared using the synthetic route of Example 1 by replacing the raw material (3-fluoro-4-methoxyphenyl)boronic acid of step 3 with 3-(aminosulfonyl)phenyl boronic acid.MS m/z (ESI):503.16 [M + 1]⁺.

### Example 6

### 4-(2-(4-aminopiperidin-1-yl)-6-(o-methylphenyl)quinazolin-4-yl)-2-fluorobenzonitrile 6

The title compound 6hydrochloride (26mg) was prepared using the synthetic route of Example 1 by replacing the raw material (3-fluoro-4-methoxyphenyl)boronic acid of step 3 witho-methylphenylboronic acid.¹H NMR (400 MHz, DMSO-*d*₆) δ 8.14 (t, J = 7.2 Hz, 1H), 8.07 (d, J = 8.6 Hz, 1H), 7.95 (d, J = 9.7 Hz, 1H), 7.92 - 7.89 (m, 1H), 7.79 (d, J = 7.8 Hz, 1H), 7.60 (d, J = 1.7 Hz, 1H), 7.32 - 7.21 (m, 4H), 4.95 (d, J = 13.1 Hz, 2H), 3.43 - 3.39 (m, 1H), 3.28 (t, J = 12.8 Hz, 2H), 2.22 (s, 3H), 2.12 (d, J = 12.2 Hz, 2H), 1.66 (d, J = 11.9 Hz, 2H).

### Example 7

### 4-(2-(4-Aminopiperidin-1-yl)-6-(2-isopropylphenyl)quinazolin-4-yl)-2-fluorobenzonitrile 7

The title compound 7 hydrochloride (26mg) was prepared using the synthetic route of Example 1 by replacing the raw material (3-fluoro-4-methoxyphenyl)boronic acid of step 3 with2-isopropylphenylboronic acid.¹H NMR (400 MHz, DMSO-d₆) δ 8.12 (t, J = 6.9 Hz, 1H), 8.00 (d, J = 7.7 Hz, 1H), 7.94 (d, J = 9.6 Hz, 1H), 7.79 (t, J = 8.2 Hz, 2H), 7.52 (s, 1H), 7.41 (d, J = 7.5 Hz, 1H), 7.34 (t, J = 7.0 Hz, 1H), 7.19 (q, J = 7.9 Hz, 2H), 4.93 (d, J = 12.5 Hz, 2H), 3.43 - 3.34 (m, 1H), 3.22 (d, J = 12.5 Hz, 2H), 3.06 - 2.88 (m, 1H), 2.11 (d, J = 11.9 Hz, 2H), 1.64 (t, J = 11.8 Hz, 2H), 1.08 (d, J = 6.4 Hz, 6H).

### Example 8

### 4-(2-(4-atninopiperidin-1-yl)-6-(m-methylphenyl)quinazolin-4-yl)-2-fluorobenzonitrile 8

The title compound 8 hydrochloride (16 mg) was prepared using the synthetic route of Example 1 by replacing the raw material (3-fluoro-4-methoxyphenyl)boronic acid of step 3 with 3-methylphenylboronic acid.¹H NMR (400 MHz, DMSO-d₆) δ 8.21 - 8.12 (m, 2H), 8.01 (d, J = 9.8 Hz, 1H), 7.93 - 7.81 (m, 3H), 7.49 - 7.41 (m, 2H), 7.34 (t, J = 7.5 Hz, 1H), 7.18 (d, J = 7.3 Hz, 1H), 4.91 (d, J = 13.2 Hz, 2H), 3.40 - 3.36 (m, 1H), 3.19 (t, J = 12.6 Hz, 2H), 2.35 (s, 3H), 2.09 (d, J = 12.1 Hz, 2H), 1.61 (q, J = 12.4, 11.2 Hz, 2H).

### Example 9

### 4-(2-(4-aminopiperidin-1-yl)-6-(1-methyl-1H-indazol-5-yl)quinazolin-4-yl)-2-fluorobenzonitrile 9

The title compound 9 hydrochloride (26 mg) was prepared using the synthetic route of Example 1 by replacing the raw material (3-fluoro-4-methoxyphenyl)boronic acid of step 3 with 1-methylindazole-5-boronic acid.¹H NMR (400 MHz, Methanol-*d*₄) δ 8.40 (d, J = 8.5 Hz, 1H), 8.15 - 8.03 (m, 5H), 7.91 (d, J = 7.8 Hz, 1H), 7.73 (d, J = 8.7 Hz, 1H), 7.66 (d, J = 8.7 Hz, 1H), 5.07 - 5.02 (m, 2H), 4.09 (s, 3H), 3.67 - 3.59 (m, 1H), 3.52 (d, J = 11.5 Hz, 2H), 2.30 (d, J = 11.3 Hz, 2H), 1.88 (d, J = 12.2 Hz, 2H).

### Example 10

### 4-(2-(4-aminopiperidin-1-yl)-6-(1-methyl-1H-indazol-4-yl)quinazolin-4-yl)-2-fluorobenzonitrile 10

The title compound 36 hydrochloride (22 mg) was prepared using the synthetic route of Example 1 by replacing the raw material (3-fluoro-4-methoxyphenyl)boronic acid of step 3 with 1-methylindazol-4-boronic acid.¹H NMR (400 MHz, Methanol-*d*₄) δ 8.43 (dd, J = 8.7, 1.8 Hz, 1H), 8.17 (d, J = 1.7 Hz, 1H), 8.13 (d, J = 8.7 Hz, 1H), 8.07 (dd, J = 7.9, 6.4 Hz, 1H), 8.03 (s, 1H), 7.98 (d, J = 9.5 Hz, 1H), 7.92 (d, J = 7.9 Hz, 1H), 7.88 - 7.81 (m, 2H), 7.44 (d, J = 8.4 Hz, 1H), 5.05 (d, J = 11.6 Hz, 2H), 4.11 (s, 3H), 3.64 (d, J = 12.4 Hz, 1H), 3.50 (t, J = 12.9 Hz, 2H), 2.34 - 2.27 (m, 2H), 1.88 (d, J = 12.5 Hz, 2H).

### Example 11

### 4-(2-(4-aminopiperidin-1-yl)-6-(1-methyl-1H-indazol-7-yl)quinazolin-4-yl)-2-fluorobenzonitrile 11

The title compound 11 hydrochloride (23 mg) was prepared using the synthetic route of Example 1 by replacing the raw material (3-fluoro-4-methoxyphenyl)boronic acid of step 3 with 1-methylindazol-7-boronic acid.¹H NMR (400 MHz, Methanol-*d*₄) δ 8.25 - 8.17 (m, 2H), 8.09 (d, J = 1.5 Hz, 1H), 8.06 - 7.92 (m, 3H), 7.88 (d, J = 7.9 Hz, 1H), 7.83 (dd, J = 7.9, 1.5 Hz, 1H), 7.31 (d, J = 6.9 Hz, 1H), 7.23 (t, J = 7.5 Hz, 1H), 5.10 - 5.02 (m, 2H), 3.70 - 3.55 (m, 6H), 2.33 (d, J = 11.2 Hz, 2H), 2.01 - 1.83 (m, 2H).

### Example 12

### 4-(2-(4-Aminopiperidin-1 -yl)-6-(2,6-dimethylphenyl)quinazolin-4-yl)-2-fluorobenzonitrile 12

The title compound 12 hydrochloride (18 mg) was prepared using the synthetic route of Example 1 by replacing the raw material (3-fluoro-4-methoxyphenyl)boronic acid of step 3 with 2,6-dimethylphenylboronic acid.¹H NMR (400 MHz, Methanol-*d*₄) δ 8.18 (d, J = 8.2 Hz, 1H), 7.98 (d, J = 6.3 Hz, 1H), 7.89 - 7.76 (m, 3H), 7.65 (s, 1H), 7.19 - 7.05 (m, 3H), 5.10 - 5.03 (m, 2H), 3.70 - 3.64 (m, 1H), 3.56 - 3.50 (m, 2H), 2.33 (d, J = 11.3 Hz, 2H), 2.01 (s, 6H), 1.96 - 1.86 (m, 2H).

### Example 13

### 4-(2-(4-aminopiperidin-1-yl)-6-(2-(trifluoromethoxy)phenyl)quinazolin-4-yl)-2-fluorobenzonitrile 13

The title compound 13 hydrochloride (16 mg) was prepared using the synthetic route of Example 1 by replacing the raw material (3-fluoro-4-methoxyphenyl)boronic acid of step 3 with 2-(trifluoromethoxy)phenylboronic acid.¹H NMR (400 MHz, CDCl*3*) δ 4 (s, 1H), 8.20 (s, 1H), 8.07 (t, J = 7.1 Hz, 1H), 8.03 (t, J = 8.6 Hz, 1H), 3.71 (s, 2H), 7.88 (d, J = 8.1 Hz, 1H), 7.62 - 2.84 (m, 2H), 2.61 (s, 1H), 7.59 - 7.44 (m, 3H), 5.10 - 5.03 (m, 2H), 3.68 (s, 4H), 3.57 (s, 2H), 2.34 (t, J = 12.0 Hz, 4H), 2.01 - 1.83 (m, 2H).

### Example 14

### 4-(2-(4-aminopiperidin-1 -yl)-6-(2-(trifluoromethyl)phenyl)quinazolin-4-yl)-2-fluorobenzonitrile 14

The title compound 14 hydrochloride (20 mg) was prepared using the synthetic route of Example 1 by replacing the raw material (3-fluoro-4-methoxyphenyl)boronic acid of step 3 with 2-(trifluoromethyl)phenylboronic acid.¹H NMR (400 MHz, Methanol-d₄) δ 8.13 (d, J = 8.5 Hz, 1H), 8.03 (t, J = 6.9 Hz, 2H), 7.88 (dd, J = 16.5, 8.4 Hz, 4H), 7.73 (t, J = 7.4 Hz, 1H), 7.65 (t, J = 7.7 Hz, 1H), 7.48 (d, J = 7.4 Hz, 1H), 5.10 - 5.03 (m, 2H), 3.71 - 3.64 (m, 1H), 3.53 (d, J = 13.3 Hz, 2H), 2.39 - 2.27 (m, 2H), 1.92 (d, J = 12.7 Hz, 2H).

### Example 15

### 4-(2-(4-aminopiperidin-1-yl)-6-(2-fluorophenyl)quinazolin-4-yl)-2-fluorobenzonitrile 15

The title compound 15 hydrochloride (30 mg) was prepared using the synthetic route of Example 1 by replacing the raw material (3-fluoro-4-methoxyphenyl)boronic acid of step 3 with 2-fluorophenylboronic acid.¹H NMR (400 MHz, Methanol-*d*₄) δ 8.24 (d, J = 9.2 Hz, 1H), 8.13 - 8.01 (m, 3H), 7.93 (d, J = 9.5 Hz, 1H), 7.87 (d, J = 7.9 Hz, 1H), 7.57 (td, J = 7.9, 1.7 Hz, 1H), 7.45 (tdd, J = 7.2, 5.0, 1.7 Hz, 1H), 7.35 - 7.20 (m, 2H), 5.05 (d, J = 12.5 Hz, 2H), 3.66 -3.60 (m, 1H), 3.55 - 3.39 (m, 2H), 2.29 (d, J = 12.0 Hz, 2H), 1.95 - 1.77 (m, 2H).

### Example 16

### 4-(2-(4-aminopiperidin-1 -yl)-6-(2-chlorophenyl)quinazolin-4-yl)-2-fluorobenzonitrile 16

The title compound 16 hydrochloride (32 mg) was prepared using the synthetic route of Example 1 by replacing the raw material (3-fluoro-4-methoxyphenyl)boronic acid of step 3 with 2-chlorophenylboronic acid.¹H NMR (400 MHz, Methanol-d₄) δ 8.13 (q, J = 8.7 Hz, 2H), 8.04 (t, J = 7.0 Hz, 1H), 7.98 (s, 1H), 7.93 (d, J = 9.5 Hz, 1H), 7.87 (d, J = 7.8 Hz, 1H), 7.58 - 7.52 (m, 1H), 7.50 - 7.37 (m, 3H), 5.08 - 5.02 (m, 2H), 3.72 - 3.60 (m, 1H), 3.58 - 3.41 (m, 2H), 2.31 (d, J = 11.0 Hz, 2H), 1.93 - 1.86 (m, 2H).

### Example 17

### 4-(2-(4-atninopiperidin-1-yl)-6-(2,3-difluorophenyl)quinazolin-4-yl)-2-fluorobenzonitrile 17

The title compound 17 hydrochloride (19 mg) was prepared using the synthetic route of Example 1 by replacing the raw material (3-fluoro-4-methoxyphenyl)boronic acid of step 3 with 2,3-fluorophenylboronic acid.¹H NMR (400 MHz, Methanol-d₄) δ 8.26 (d, J = 8.8 Hz, 1H), 8.17 - 8.02 (m, 3H), 7.96 (d, J = 9.5 Hz, 1H), 7.90 (d, J = 8.2 Hz, 1H), 7.43 - 7.25 (m, 3H), 5.12 - 5.03 (m, 2H), 3.71 - 3.58 (m, 1H), 3.57 - 3.40 (m, 2H), 2.32 (d, J = 12.3 Hz, 2H), 1.97 - 1.78 (m, 2H).

### Example 18

4-(2-(4-aminopiperidin-1-yl)-6-(2,6-bis(trifluoromethyl)phenyl)quinazolin-4-yl)-2-fluorobenzonitrile **18**The title compound 18 hydrochloride (14 mg) was prepared using the synthetic route of Example 1 by replacing the raw material (3-fluoro-4-methoxyphenyl)boric acid of step 3 with 2, 6-bis(trifluoromethyl)phenylboric acid.MS m/z (ESI):560.23 [M + 1]⁺.

### Example 19

### 4-(2-(4-atninopiperidin-1-yl)-6-(2-fluoro-6-(trifluoromethyl)phenyl)quinazolin-4-yl)-2-fluorobenzonitri le19

The title compound 19 hydrochloride (23 mg) was prepared using the synthetic route of Example 1 by replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.13 (t, J = 7.3 Hz, 1H), 7.95 (d, J = 8.7 Hz, 1H), 7.84 - 7.76 (m, 2H), 7.73 - 7.61 (m, 5H), 4.93 (d, J = 13.5 Hz, 2H), 3.46 - 3.37 (m, 1H), 3.24 (t, J = 12.7 Hz, 2H), 2.11 (dd, J = 13.0, 3.9 Hz, 2H), 1.63 (d, J = 12.5 Hz, 2H).

### Example 20

### 4-(2-(4-aminopiperidin-1-yl)-6-(2,6-dichlorophenyl)quinazolin-4-yl)-2-fluorobenzonitrile 20

The title compound 20 hydrochloride (32 mg) was prepared using the synthetic route of Example 1 by replacing the raw material (3-fluoro-4-methoxyphenyl)boronic acid of step 3 with 2,6-dichlorophenylboronic acid.¹H NMR (400 MHz, DMSO-*d*₆) δ 8.15 (dd, J = 8.0, 6.6 Hz, 1H), 7.99 (d, J = 8.7 Hz, 1H), 7.86 (dd, J = 9.8, 1.5 Hz, 1H), 7.77 (dd, J = 8.7, 1.9 Hz, 1H), 7.73 (dd, J = 8.0, 1.5 Hz, 1H), 7.67 (d, J = 1.9 Hz, 1H), 7.59 (d, J = 8.0 Hz, 2H), 7.44 (dd, J = 8.7, 7.5 Hz, 1H), 4.94 (d, J = 13.4 Hz, 2H), 3.43 - 3.34 (m, 1H), 3.25 (t, J = 12.7 Hz, 2H), 2.14 - 2.09 (m, 2H), 1.64 (qd, J = 12.5, 4.1 Hz, 2H).

### Example 21

### 4-(2-(4-Aminopiperidin-1 -yl)-6-(2-chloro-6-methylphenyl)quinazolin-4-yl)-2-fluorobenzonitrile 21

The title compound 21 hydrochloride (22 mg) was prepared using the synthetic route of Example 1 by replacing the raw material (3-fluoro-4-methoxyphenyl)boronic acid of step 3 with 2-chloro-6-methylphenylboronic acid.¹H NMR (400 MHz, DMSO-*d*₆) δ 8.13 (dd, J = 8.0, 6.7 Hz, 1H), 7.88 (dd, J = 9.8, 1.5 Hz, 1H), 7.80 (d, J = 8.7 Hz, 1H), 7.72 (dd, J = 8.0, 1.5 Hz, 1H), 7.66 (dd, J = 8.7, 1.9 Hz, 1H), 7.50 (d, J = 1.9 Hz, 1H), 7.39 (dd, J = 5.4, 3.9 Hz, 1H), 7.35 - 7.27 (m, 2H), 4.90 (d, J = 13.4 Hz, 2H), 3.44 - 3.32 (m, 1H), 3.16 (t, J = 12.7 Hz, 2H), 2.07 - 2.05 (s, 5H), 1.64 - 1.51 (m, 2H).

### Example 22

### 4-(2-(4-Aminopiperidin-1 -yl)-6-(2-chloro-6-fluorophenyl)quinazolin-4-yl)-2-fluorobenzonitrile 22

The title compound 22 hydrochloride (12 mg) was prepared using the synthetic route of Example 1 by replacing the raw material (3-fluoro-4-methoxyphenyl)boronic acid of step 3 with 2-chloro-6-fluorophenylboronic acid.¹H NMR (400 MHz, DMSO-*d*₆) δ 8.15 (t, J = 7.3 Hz, 1H), 7.97 (d, J = 8.7 Hz, 1H), 7.87 (dd, J = 15.4, 9.3 Hz, 2H), 7.78 - 7.71 (m, 2H), 7.47 (t, J = 5.3 Hz, 2H), 7.39 - 7.30 (m, 1H), 4.94 (d, J = 13.2 Hz, 2H), 3.50 - 3.33 (m, 1H), 3.24 (t, J = 12.6 Hz, 2H), 2.11 (dd, J = 13.0, 3.9 Hz, 2H), 1.64 (qd, J = 12.0, 4.0 Hz, 2H).

### Example 23

### 4-(2-(4-Aminopiperidin-1-yl)-6-(3-fluoro-2-(trifluoromethyl)phenyl)quinazolin-4-yl)-2-fluorobenzonitr ile 23

The title compound 23 hydrochloride (32 mg) was prepared using the synthetic route of Example 1 by replacing the raw material (3-fluoro-4-methoxyphenyl)boronic acid of step 3 with 3-fluoro-2-(trifluoromethyl)phenylboronic acid.¹H NMR (400 MHz, DMSO-*d*₆) δ 8.18 - 8.10 (m, 1H), 7.96 - 7.88 (m, 2H), 7.78 (ddt, J = 13.5, 10.3, 5.3 Hz, 3H), 7.66 (d, J = 1.9 Hz, 1H), 7.54 (dd, J = 11.6, 8.5 Hz, 1H), 7.31 (d, J = 7.7 Hz, 1H), 4.93 (d, J = 13.5 Hz, 2H), 3.40 (dt, J = 13.5, 4.9 Hz, 1H), 3.23 (t, J = 12.7 Hz, 2H), 2.14 - 2.08 (m, 2H), 1.64 (qd, J = 12.2, 4.1 Hz, 2H).

### Example 24

### 5-(2-(4-aminopiperidin-1-yl)-6-(3-fluoro-4-methoxyphenyl)quinazolin-4-yl)pyridinecarbonitrile 24

The title compound 24 hydrochloride (10 mg) was prepared using the synthetic route of Example 1 by replacing the raw material (4-cyano-3-fluorophenyl)boronic acid of step 1 with (6-cyanopyridin-3-yl)boronic acid.¹H NMR (400 MHz, Methanol-*d*₄) δ 9.21 (d, J = 2.1 Hz, 1H), 8.55 (dd, J = 8.0, 2.2 Hz, 1H), 8.31 (dd, J = 8.8, 2.0 Hz, 1H), 8.17 (d, J = 8.0 Hz, 1H), 8.08 (dd, J = 8.8, 2.2 Hz, 1H), 8.04 (d, J = 2.0 Hz, 1H), 7.51 - 7.42 (m, 2H), 7.20 (t, J = 8.9 Hz, 1H), 5.03 (d, J = 12.7 Hz, 2H), 3.92 (s, 3H), 3.61 (d, J = 11.1 Hz, 1H), 3.49 (t, J = 13.0 Hz, 2H), 2.29 (d, J = 12.4 Hz, 2H), 1.86 (q, J = 12.1 Hz, 2H).

### Example 25

### 4-(2-(4-atninopiperidin-1-yl)-6-(3-fluoro-4-methoxyphenyl)quinazolin-4-yl)benzonitrile 25

The title compound 25hydrochloride (24 mg) was prepared using the synthetic route of Example 1 by replacing the raw material (4-cyano-3-fluorophenyl)boronic acid of step 1 with (4-cyanophenyl)boronic acid.¹H NMR (400 MHz, Methanol-*d*₄) δ 8.27 (dd, J = 8.8, 2.0 Hz, 1H), 8.11 - 8.00 (m, 6H), 7.48 - 7.38 (m, 2H), 7.20 (t, J = 8.9 Hz, 1H), 5.04 (d, J = 14.3 Hz, 2H), 3.92 (s, 3H), 3.64 - 3.55 (m, 2H), 3.44 (t, J = 13.2 Hz, 2H), 2.27 (d, J = 12.6 Hz, 2H), 1.82 (qd, J = 12.3, 10.4, 2.2 Hz, 2H)..

### Example 26

### 2-Fluoro-4-(6-(3-fluoro-4-methoxyphenyl)-2-(piperazin-1-yl)quinazolin-4-yl)benzonitrile 26

The title compound 24 hydrochloride (29 mg) was prepared using the synthetic route of Example 1 by replacing the raw materialtert-butyl piperidin-4-ylcarbamate of step 2 with tert-butyl piperazin-1-carboxylate.¹H NMR (400 MHz, DMSO*-d*₆) δ 8.21 - 8.12 (m, 2H), 8.01 (dd, J = 9.9, 1.4 Hz, 1H), 7.89 (d, J = 2.0 Hz, 1H), 7.85 (dd, J = 7.9, 1.4 Hz, 1H), 7.80 (d, J = 8.8 Hz, 1H), 7.60 (dd, J = 12.9, 2.2 Hz, 1H), 7.47 (dd, J = 8.4, 2.2 Hz, 1H), 7.23 (t, J = 8.8 Hz, 1H), 4.18 (t, J = 5.1 Hz, 4H), 3.26 - 3.19 (m, 4H).

### Example 27

### (S) -4-(2-(3-aminopyrrolidin-1-yl)-6-(3-fluoro-4-methoxyphenyl)quinazolin-4-yl)-2-fluorobenzonitrile 27

The title compound 27 hydrochloride (32 mg) was prepared using the synthetic route of Example 1 by replacing the raw materialtert-butyl piperidin-4-ylcarbamate of step 2 with (S)-3-tert-butoxycarbonylaminopyrrolidine.¹H NMR (400 MHz, DMSO-*d*₆) δ 8.24 - 8.15 (m, 2H), 8.01 (d, J = 9.7 Hz, 2H), 7.90 (s, 1H), 7.85 (d, J = 7.7 Hz, 1H), 7.61 (d, J = 12.6 Hz, 1H), 7.48 (d, J = 8.2 Hz, 1H), 7.24 (t, J = 8.7 Hz, 1H), 4.03 - 3.85 (m, 5H), 2.43 - 2.35 (m, 1H), 2.28 - 2.17 (m, 1H).

### Example 28

### (R) -4-(2-(3-aminopyrrolidin-1-yl)-6-(3-fluoro-4-methoxyphenyl)quinazolin-4-yl)-2-fluorobenzonitrile 28

The title compound 28 hydrochloride (20 mg) was prepared using the synthetic route of Example 1 by replacing the raw materialtert-butyl piperidin-4-ylcarbamate of step 2 with (R)-3-tert-butoxycarbonylaminopyrrolidine.¹H NMR (400 MHz, DMSO-*d*₆) δ 8.20 (q, J = 7.5, 6.8 Hz, 2H), 8.01 (d, J = 9.7 Hz, 2H), 7.94 - 7.89 (m, 1H), 7.85 (d, J = 7.8 Hz, 1H), 7.62 (d, J = 12.7 Hz, 1H), 7.49 (d, J = 8.4 Hz, 1H), 7.24 (t, J = 8.7 Hz, 1H), 3.94 - 3.79 (m, 5H), 2.42 - 2.36 (m, 1H), 2.27 - 2.18 (m, 1H).

### Example 29

### 2-Fluoro-4-(6-(3-fluoro-4-methoxyphenyl)-2-((pyrrolidin-3-ylmethyl)amino)quinazolin-4-yl)benzonitril e 29

The title compound 29 hydrochloride (35 mg) was prepared using the synthetic route of Example 1 by replacing the raw materialtert-butyl piperidin-4-ylcarbamate of step 2 with N-tert-butoxycarbonyl-3-(aminomethyl)pyrrolidine.¹HNMR (400 MHz, Methanol-*d*₄) δ 8.21 (s, 1H), 8.05 (t, J = 7.2 Hz, 1H), 7.97 - 7.83 (m, 3H), 7.46 - 7.37 (m, 2H), 7.18 (t, J = 8.9 Hz, 1H), 3.91 (s, 3H), 3.80 - 3.76 (m, 2H), 3.59 - 3.41 (m, 2H), 3.36-3.32 (m, 1H), 3.20 - 3.12 (m, 1H), 2.92 - 2.84 (m, 1H), 2.32 -2.28 (m, 1H), 1.96 - 1.87 (m, 1H).

### Example 30

### 2-Fluoro-4-(6-(3-fluoro-4-methoxyphenyl)-2-((piperidin-4-ylmethyl)atnino)quinazolin-4-yl)benzonitril e 30

The title compound 30 hydrochloride (29 mg) was prepared using the synthetic route of Example 1 by replacing the raw materialtert-butyl piperidin-4-ylcarbamate of step 2 with 1-tert-butoxycarbonyl-4-aminomethylpiperidine.¹H NMR (400 MHz, DMSO-*d*₆) δ 8.40 - 8.22 (m, 2H), 8.21 (t, J = 7.2 Hz, 1H), 7.97 (d, J = 9.7 Hz, 1H), 7.92 - 7.79 (m, 2H), 7.63 (d, J = 12.7 Hz, 1H), 7.49 (d, J = 8.3 Hz, 1H), 7.25 (t, J = 8.8 Hz, 1H), 3.87 (s, 3H), 3.63 - 3.58 (m, 2H), 3.54 - 3.50 (m, 1H), 3.28 (d, J = 12.2 Hz, 2H), 2.83 (q, J = 11.7 Hz, 2H), 2.05 - 1.99 (m, 2H), 1.57 - 1.41 (m, 2H).

### Example 31

### (S) -4-(6-(3-fluoro-4-methoxyphenyl)-2-(pyrrolidin-3-ylmethoxy)quinazolin-4-yl)benzonitrile 31

The title compound 31 hydrochloride (20 mg) was prepared using the synthetic route of Example 1 by replacing the raw material (4-cyano-3-fluorophenyl)boronic acid of step 1 with (4-cyanophenyl)boronic acid, and replacing the raw material tert-butyl piperidin-4-ylcarbamate of step 2 with (S)-1-tert-butoxycarbonyl-3-hydroxymethylpyrrolidine.¹H NMR (400 MHz, Methanol-*d*₄) δ 8.22 (dd, J = 8.8, 2.0 Hz, 1H), 8.01 (s, 5H), 7.96 (d, J = 8.8 Hz, 1H), 7.44 - 7.40 (m, 1H), 7.39 (d, J = 1.7 Hz, 1H), 7.17 (t, J = 8.6 Hz, 1H), 4.69 (dd, J = 10.9, 5.1 Hz, 1H), 4.62 (dd, J = 10.9, 6.0 Hz, 1H), 3.91 (s, 3H), 3.68 - 3.44 (m, 2H), 3.42 - 3.32 (m, 2H), 2.35 (ddt, J = 21.6, 14.7, 7.6 Hz, 1H), 2.18 - 1.99 (m, 2H).

### Example 32

### 4-(6-(3-fluoro-4-methoxyphenyl)-2-(piperidin-4-ylmethoxy)quinazolin-4-yl)benzonitrile32

The title compound 32 hydrochloride (13 mg) was prepared using the synthetic route of Example 1 by replacing the raw material (4-cyano-3-fluorophenyl)boronic acid of step 1 with (4-cyanophenyl)boronic acid, and replacing the raw material tert-butyl piperidin-4-ylcarbamate of step 2 with 1-tert-butoxycarbonyl-4-piperidinemethanol.¹H NMR (400 MHz, Methanol-*d*₄) δ 8.21 (dd, J = 8.8, 2.0 Hz, 1H), 8.05 - 7.96 (m, 5H), 7.96 (d, J = 8.9 Hz, 1H), 7.44 - 7.37 (m, 2H), 7.18 (t, J = 8.8 Hz, 1H), 5.34 (t, J = 4.8 Hz, 2H), 4.51 (d, J = 5.9 Hz, 2H), 3.91 (s, 3H), 3.48 (d, J = 12.3 Hz, 2H), 3.13-3.03 (m, 2H), 2.30 (q, J = 7.2 Hz, 1H), 1.80 - 1.65 (m, 2H).

### Example 33

### 4-(6-(3,5-dimethylisoxazol-4-yl)-2-(pyrrolidin-3-ylmethyl)amino)quinazolin-4-yl)-2-fluorobenzonitrile 33

The title compound 33 hydrochloride (20 mg) was prepared using the synthetic route of Example 1 by replacing the raw materialtert-butyl piperidin-4-ylcarbamate of step 2 with 1-tert-butoxycarbonyl-3-(aminomethyl)pyrrolidine, and replacing the raw material (3-fluoro-4-methoxyphenyl)boronic acid of step 3 with 3,5-dimethylisoxazol-4-boronic acid.MS m/z (ESI):443.31 [M + 1]⁺.

### Example 34

### 2-Fluoro-4-(2-((pyrrolidin-3-ylmethyl)amino)-6-(o-methylphenyl)quinazolin-4-yl)benzonitrile 34

The title compound 34 hydrochloride (18 mg) was prepared using the synthetic route of Example 1 by replacing the raw materialtert-butyl piperidin-4-ylcarbamate of step 2 with 1-tert-butoxycarbonyl-3-(aminomethyl)pyrrolidine, and replacing the raw material (3-fluoro-4-methoxyphenyl)boronic acid of step 3 with o-methylphenylboronic acid. H NMR (400 MHz, Methanol-d4) δ 8.26 - 7.70 (m, 6H), 7.38 - 7.14 (m, 4H), 3.94 - 3.82 (m, 2H), 3.66 - 3.43 (m, 2H), 3.37 - 3.32 (m, 1H), 3.22 - 3.15 (m, 1H), 3.01 - 2.86 (m, 1H), 2.41 - 2.25 (m, 1H), 2.26 (s, 3H), 1.97 - 1.87 (m, 1H).

### Example 35

### (S) -2-fluoro-4-(6-(3-fluoro-4-methoxyphenyl)-2-((pyrrolidin-3-ylmethyl)amino)quinazolin-4-yl)benzonitrile 35

The title compound 35 hydrochloride (15 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine.¹H NMR (400 MHz, Methanol-*d*₄) δ 8.21 (s, 1H), 8.05 (t, J = 7.2 Hz, 1H), 7.97 - 7.83 (m, 3H), 7.46 - 7.37 (m, 2H), 7.18 (t, J = 8.9 Hz, 1H), 3.91 (s, 3H), 3.81 - 3.77 (m, 2H), 3.59 - 3.41 (m, 2H), 3.36 - 3.32 (m, 1H), 3.20 - 3.12 (m, 1H), 2.92 - 2.84 (m, 1H), 2.32 - 2.27 (m, 1H), 1.96 - 1.87 (m, 1H).

### Example 36

### (R)-2-fluoro-4-(6-(3-fluoro-4-methoxyphenyl)-2-((pyrrolidin-3-ylmethyl)amino)quinazolin-4-yl)benzon itrile 36

The title compound 36 hydrochloride (30 mg) was prepared using the synthetic route of Example 1 by replacing the raw materialtert-butyl piperidin-4-ylcarbamate of step 2 with (S)-N-tert-butoxycarbonyl-3-(aminomethyl)pyrrolidine.¹H NMR (400 MHz, Methanol-*d*₄) δ 8.29 (s, 1H), 8.18 - 7.83 (m, 5H), 7.43 (t, J = 9.2 Hz, 2H), 7.19 (t, J = 8.6 Hz, 1H), 3.91 (s, 3H), 3.85 - 3.79 (m, 2H), 3.65 - 3.41 (m, 2H), 3.38 - 3.33 (m, 1H), 3.20 - 3.13 (m, 1H), 2.92 - 2.84 (m, 1H), 2.35 - 2.29 (m, 1H), 1.96 - 1.88 (m, 1H).

### Example 37

### (S)-2-fluoro-4-(2-((pyrrolidin-3-ylmethyl)amino)-6-(o-methylphenyl)quinazolin-4-yl)benzonitrile 37

The title compound 37 hydrochloride (25 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with o-methylphenylboronic acid.¹H NMR (400 MHz, Methanol-*d*₄) δ 8.28 - 7.63 (m, 6H), 7.39 - 7.17 (m, 4H), 3.87 (t, J = 7.7 Hz, 2H), 3.71 - 3.32 (m, 3H), 3.26 - 3.10 (m, 1H), 3.02 - 2.82 (m, 1H), 2.46 - 2.30 (m, 1H), 2.27 (s, 3H), 2.01 - 1.86 (m, 1H).

### Example 38

### (R) -2-fluoro-4-(2-((pyrrolidin-3-ylmethyl)amino)-6-(o-methylphenyl)quinazolin-4-yl)benzonitrile 38

The title compound 38 hydrochloride (16 mg) was prepared using the synthetic route of Example 1 by replacing the raw materialtert-butyl piperidin-4-ylcarbamate of step 2 with (S)-N-tert-butoxycarbonyl-3-(aminomethyl)pyrrolidine, and replacing the raw material (3-fluoro-4-methoxyphenyl)boronic acid of step 3 with o-methylphenylboronic acid.MS m/z (ESI):438.45 [M + 1]⁺.

### Example 39

### (S)-2-fluoro-4-(2-((pyrrolidin-3-ylmethyl)amino)-6-(2-(trifluoromethoxy)phenyl)quinazolin-4-yl)benzo nitrile 39

The title compound 39 hydrochloride (36 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-(trifluoromethoxy)phenylboronic acid.¹H NMR (400 MHz, Methanol-*d*₄) δ 8.31 - 7.78 (m, 6H), 7.67 - 7.40 (m, 4H), 3.97 - 3.79 (m, 2H), 3.67 - 3.40 (m, 3H), 3.25 - 3.08 (m, 1H), 3.01 - 2.81 (m, 1H), 2.45 - 2.24 (m, 1H), 2.00 - 1.83 (m, 1H).

### Example 40

### (S)-2-fluoro-4-(2-((pyrrolidin-3-ylmethyl)amino)-6-(2-(trifluoromethyl)phenyl)quinazolin-4-yl)-2-fluor obenzonitrile 40

The title compound 40 hydrochloride (16 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-(trifluoromethyl)phenylboronic acid.¹H NMR (400 MHz, Methanol-*d*₄) δ 8.20 - 8.17 (m, 1H), 8.05 - 8.01 (m, 2H), 7.95 - 7.77 (m, 4H), 7.71 (t, J = 7.3 Hz, 1H), 7.63 (t, J = 7.7 Hz, 1H), 7.46 (d, J = 7.6 Hz, 1H), 3.98 - 3.78 (m, 2H), 3.70 - 3.32 (m, 3H), 3.26 - 3.08 (m, 1H), 3.02 - 2.83 (m, 1H), 2.48 - 2.23 (m, 1H), 2.00 - 1.84 (m, 1H).

### Example 41

### (S)-2-fluoro-4-(2-((pyrrolidin-3-ylmethyl)amino)-6-(2-fluorophenyl)quinazolin-4-yl)-2-fluorobenzonitri le 41

The title compound 41 hydrochloride (30 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-fluorophenylboronic acid. H NMR (400 MHz, Methanol-*d*₄) δ 8.32 - 7.84 (m, 6H), 7.57 (t, J = 7.7 Hz, 1H), 7.45 (q, J = 7.5 Hz, 1H), 7.31 (t, J = 7.5 Hz, 1H), 7.24 (dd, J = 11.1, 8.2 Hz, 1H), 3.94 - 3.87 (m, 2H), 3.68 - 3.43 (m, 2H), 3.41 - 3.33 (m, 1H), 3.27 - 3.12 (m, 1H), 2.99 - 2.87 (m, 1H), 2.45 - 2.25 (m, 1H), 2.06 - 1.87 (m, 1H).

### Example 42

### (S)-2-fluoro-4-(2-((pyrrolidin-3-ylmethyl)amino)-6-(2-chlorophenyl)quinazolin-4-yl)-2-fluorobenzonitr ile 42

The title compound 42 hydrochloride (18 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-chlorophenylboronic acid.¹H NMR (400 MHz, Methanol-*d*₄) δ 8.29 - 7.82 (m, 6H), 7.54 (dq, J = 6.6, 3.9, 3.2 Hz, 1H), 7.50 - 7.40 (m, 3H), 3.93 - 3.87 (m, 2H), 3.68 - 3.43 (m, 2H), 3.40 - 3.32 (m, 1H), 3.26 - 3.11 (m, 1H), 2.98 - 2.87 (m, 1H), 2.44 - 2.24 (m, 1H), 2.05 - 1.86 (m, 1H).

### Example 43

### (S)-2-fluoro-4-(2-((pyrrolidin-3-ylmethyl)amino)-6-(2,3-difluorophenyl)quinazolin-4-yl)-2-fluorobenzo nitrile 43

The title compound 43 hydrochloride (20 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2,3-difluorophenylboronic acid.¹H NMR (400 MHz, Methanol-*d*₄) δ 8.33 - 7.86 (m, 6H), 7.41 - 7.23 (m, 3H), 3.94 - 3.88 (m, 2H), 3.68 - 3.43 (m, 2H), 3.40 - 3.33 (m, 1H), 3.25 - 3.13 (m, 1H), 2.98 - 2.88 (m, 1H), 2.43 - 2.25 (m, 1H), 2.05 - 1.86 (m, 1H).

### Example 44

### (S)-2-fluoro-4-(6-(4-methylthiophen-3-yl)-2-((pyrrolidin-3-ylmethyl)amino)quinazolin-4-yl)benzonitril e 44

The title compound 44 hydrochloride (24 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 4-methyl-3-thiopheneboronic acid.¹H NMR (400 MHz, Methanol-*d*₄) δ 8.29 - 7.79 (m, 6H), 7.48 (s, 1H), 7.18 (s, 1H), 3.90 - 3.84 (m, 2H), 3.70 - 3.41 (m, 2H), 3.36 - 3.30 (m, 1H), 3.22 - 3.16 (m, 1H), 2.98 - 2.89 (m, 1H), 2.48 - 2.28 (m, 1H), 2.27 (s, 3H), 2.07 - 1.87 (m, 1H).

### Example 45

### (S)-4-(6-(2,6-dimethylphenyl)-2-((pyrrolidin-3-ylmethyl)amino)quinazolin-4-yl)-2-fluorobenzonitrile 45

The title compound 45 hydrochloride (25 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2,6-dimethylphenylboronic acid.¹H NMR (400 MHz, Methanol-*d*₄) δ 8.36 - 7.57 (m, 6H), 7.20 - 7.07 (m, 3H), 3.94 - 3.88 (m, 2H), 3.70 - 3.44 (m, 2H), 3.39 - 3.33 (m, 1H), 3.24 - 3.18 (m, 1H), 3.01 - 2.90 (m, 1H), 2.48 - 2.27 (m, 1H), 2.01 (s, 6H), 2.08 - 1.87 (m, 1H).

### Example 46

### (S)-4-(6-(2,4,6-trimethylphenyl)-2-((pyrrolidin-3-ylmethyl)amino)quinazolin-4-yl)-2-fluorobenzonitrile 46

The title compound 46 hydrochloride (20 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2,4,6-trimethylphenylboronic acid.¹H NMR (400 MHz, Methanol-*d*₄) δ 8.29 - 7.56 (m, 6H), 6.96 - 6.92 (m, 2H),3.92 - 3.86 (m, 2H), 3.59 - 3.42 (m, 2H), 3.39 - 3.32 (m, 1H), 3.23 - 3.16 (m, 1H), 3.02 - 2.90 (m, 1H), 2.48 - 2.23 (m, 1H), 2.28 (s, 3H), 1.98 (s, 6H), 2.08 - 1.88 (m, 1H).

### Example 47

### (S)-2-fluoro-4-(6-(3-fluoro-2-methylphenyl)-2-((pyrrolidin-3-ylmethyl)amino)quinazolin-4-yl)benzonit rile 47

The title compound 47 hydrochloride (22 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 3-fluoro-2-methylphenylboronic acid.¹H NMR (400 MHz, Methanol-*d*₄) δ 8.37 - 7.74 (m, 6H), 7.29 (q, J = 7.1 Hz, 1H), 7.12 (dt, J = 8.8, 5.2 Hz, 2H), 3.94 - 3.88 (m, 2H), 3.72 - 3.45 (m, 2H), 3.38 - 3.33 (m, 1H), 3.25 - 3.17 (m, 1H), 3.03 - 2.90 (m, 1H), 2.49 - 2.24 (m, 1H), 2.19 (d, J = 2.4 Hz, 3H), 2.08 - 1.87 (m, 1H).

### Example 48

### (S)-2-fluoro-4-(6-(2-fluoro-6-methylphenyl)-2-((pyrrolidin-3-ylmethyl)amino)quinazolin-4-yl)benzonit rile 48

The title compound 48 hydrochloride (12 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-fluoro-6-methylphenylboronic acid.¹H NMR (400 MHz, Methanol-*d*₄) δ 8.38 - 7.68 (m, 6H), 7.37 - 7.29 (m, 1H), 7.17 (d, J = 7.6 Hz, 1H), 7.04 (t, J = 9.0 Hz, 1H), 3.95 - 3.89 (m, 2H), 3.71 - 3.44 (m, 2H), 3.39 - 3.34 (m, 1H), 3.24 - 3.17 (m, 1H), 3.04 - 2.91 (m, 1H), 2.44 - 2.28 (m, 1H), 2.21 (s, 3H), 2.08 - 1.86 (m, 1H).

### Example 49

### (S)-2-fluoro-4-(2-((pyrrolidin-3-ylmethyl)amino)-6-(1,3,5-trimethyl-1H-pyrazol-4-yl)quinazolin-4-yl)b enzonitrile 49

The title compound 49 hydrochloride (15 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with (1,3,5-trimethyl-1H-pyrazol-4-yl)boronic acid.¹H NMR (400 MHz, Methanol-*d*₄) δ 8.43 - 7.72 (m, 6H), 4.00 (s, 3H), 3.96 - 3.87 (m, 2H), 3.72 - 3.41 (m, 2H), 3. 41- 3.35 (m, 1H), 3.25 - 3.17 (m, 1H), 3.05 - 2.91 (m, 1H), 2.50 - 2.26 (m, 7H), 2.06 - 1.86 (m, 1H).

### Example 50

### (S)-4-(6-(2,6-difluorophenyl)-2-((pyrrolidin-3-ylmethyl)amino)quinazolin-4-yl)-2-fluorobenzonitrile 50

The title compound 50 hydrochloride (29 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2,6-difluorophenylboronic acid. H NMR (400 MHz, Methanol-*d*₄) δ 8.33 - 7.78 (m, 6H), 7.47 (t, J = 7.1 Hz, 1H), 7.13 (t, J = 7.9 Hz, 2H), 3.96 - 3.88 (m, 2H), 3.69 - 3.44 (m, 2H), 3.38 - 3.32 (m, 1H), 3.25 - 3.14 (m, 1H), 3.02 - 2.88 (m, 1H), 2.45 - 2.25 (m, 1H), 2.04 - 1.89 (m, 1H).

### Example 51

### (S)-4-(6-(2,6-dichlorophenyl)-2-((pyrrolidin-3-ylmethyl)amino)quinazolin-4-yl)-2-fluorobenzonitrile 51

The title compound 51 hydrochloride (35 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2,6-dichlorophenylboronic acid. H NMR (400 MHz, Methanol-*d*₄) δ 8.10 - 7.78 (m, 6H), 7.53 (d, J = 8.0 Hz, 2H), 7.41 (t, J = 8.0 Hz, 1H), 3.97 - 3.88 (m, 2H), 3.68 - 3.42 (m, 2H), 3.38 - 3.32 (m, 1H), 3.26 - 3.14 (m, 1H), 3.02 - 2.89 (m, 1H), 2.45 - 2.25 (m, 1H), 2.03 - 1.87 (m, 1H).

### Example 52

### (S)-4-(6-(2-chloro-6-(trifluoromethyl)phenyl)-2-(pyrrolidin-3-ylmethyl)amino)quinazolin-4-yl)-2-fluor obenzonitrile 52

The title compound 52 hydrochloride (35 mg) was prepared using the synthetic route of Example 40 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-chloro-6-trifluoromethylphenylboronic acid.¹H NMR (400 MHz, Methanol-*d*₄) δ 8.36 - 7.85 (m, 6H), 7.74 (q, J = 8.2 Hz, 2H), 3.97 - 3.88 (m, 2H), 3.71 - 3.44 (m, 2H), 3.39 - 3.33 (m, 1H), 3.28 - 3.16 (m, 1H), 3.02 - 2.89 (m, 1H), 2.47 - 2.27 (m, 1H), 2.03 - 1.93 (m, 1H).

### Example 53

### (S)-4-(6-(2-chloro-6-methylphenyl)-2-(pyrrolidin-3-ylmethyl)amino)quinazolin-4-yl)-2-fluorobenzonitr ile 53

The title compound 53 hydrochloride (35 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-chloro-6-methylphenylboronic acid.¹H NMR (400 MHz, Methanol-*d*₄) δ 8.34 - 7.69 (m, 6H), 7.38 - 7.32 (m, 1H), 7.28 (d, J = 4.8 Hz, 2H), 3.95 - 3.86 (m, 2H), 3.70 - 3.44 (m, 2H), 3.38 - 3.32 (m, 1H), 3.27 - 3.13 (m, 1H), 3.01 - 2.87 (m, 1H), 2.45 - 2.25 (m, 1H), 2.11 (s, 3H), 2.03 - 1.87 (m, 1H).

### Example 54

### (S)-4-(6-(2-fluoro-6-(trifluoromethylphenyl)-2-(pyrrolidin-3-ylmethyl)amino)quinazolin-4-yl)-2-fluoro benzonitrile 54

The title compound 54 hydrochloride (36 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-fluoro-6-trifluoromethylphenylboronic acid. H NMR (400 MHz, Methanol-*d*₄) δ 8.32 - 7.74 (m, 6H), 7.69 (d, J = 3.5 Hz, 2H), 7.58 - 7.49 (m, 1H), 3.95 - 3.86 (m, 2H), 3.70 - 3.43 (m, 2H), 3.35 (d, J = 8.8 Hz, 1H), 3.25 - 3.14 (m, 1H), 3.00 -2.87 (m, 1H), 2.40 - 2.28 (m, 1H), 2.05 - 1.88 (m, 1H).

### Example 55

### (S)-4-(6-(2-chloro-6-fluorophenyl)-2-(pyrrolidin-3-ylmethyl)amino)quinazolin-4-yl)-2-fluorobenzonitri le 55

The title compound 55 hydrochloride (40 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2-chloro-6-fluorophenylboronic acid.MS m/z (ESI):476.48 [M + 1]⁺.

### Example 56

### (S)-4-(6-(5-fluoro-2-(tnfluoromethyl)-2-(pyrrolidin-3-ylmethyl)amino)quinazolin-4-yl)-2-fluorobenzon itrile 56

The title compound 56 hydrochloride (36 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 5-fluoro-2-trifluoromethylphenylboronic acid.¹H NMR (400 MHz, Methanol-*d*₄) δ 8.30 - 7.78 (m, 7H), 7.38 (td, J = 8.4, 2.4 Hz, 1H), 7.29 (dd, J = 9.0, 2.6 Hz, 1H), 3.95 - 3.86 (m, 2H), 3.69 - 3.42 (m, 2H), 3.37 (d, J = 8.6 Hz, 1H), 3.25 - 3.14 (m, 1H), 2.98 - 2.85 (m, 1H), 2.40 - 2.28 (m, 1H), 2.02 - 1.88 (m, 1H).

### Example 57

### (S)-4-(6-(3-fluoro-2-(trifluoromethylphenyl)-2-(pyrrolidin-3-ylmethyl)amino)quinazolin-4-yl)-2-fluoro benzonitrile 57

The title compound 57 hydrochloride (26 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 3-fluoro-2-trifluoromethylphenylboronic acid.¹H NMR (400 MHz, Methanol-d₄) δ 8.29 - 7.77 (m, 6H), 7.72 (d, J = 6.4 Hz, 1H), 7.41 (dd, J = 11.3, 8.1 Hz, 1H), 7.27 (d, J = 7.3 Hz, 1H), 3.96 - 3.87 (m, 2H), 3.70 - 3.43 (m, 2H), 3.35 (d, J = 7.9 Hz, 1H), 3.25 - 3.15 (m, 1H), 3.01 - 2.89 (m, 1H), 2.42 - 2.30 (m, 1H), 2.04 - 1.88 (m, 1H).

### Example 58

### (S)-4-(6-(2,5-bis(trifluoromethyl)phenyl)-2-((pyrrolidin-3-ylmethyl)amino)quinazolin-4-yl)-2-fluorobe nzonitrile 58

The title compound 58 hydrochloride (18 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 2,5-bis(trifluoromethyl)phenylboronic acid.¹H NMR (400 MHz, Methanol-d₄) δ 8.33 - 7.72 (m, 9H), 3.97 - 3.88 (m, 2H), 3.68 - 3.44 (m, 2H), 3.37 - 3.31 (m, 1H), 3.24 - 3.14 (m, 1H), 3.00 - 2.87 (m, 1H), 2.41 - 2.30 (m, 1H), 1.98 - 1.87 (m, 1H).

### Example 59

### (S)-2-fluoro-4-(6-(3-methyl-2-(trifluoromethyl)phenyl)-2-((pyrrolidin-3-ylmethyl)amino)quinazolin-4-y 1)benzonitrile 59

The title compound 59 hydrochloride (17 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 3-methyl-2-(trifluoromethyl)phenylboronic acid.¹H NMR (400 MHz, Methanol-*d*₄) δ 7.91 (d, J = 81.3 Hz, 6H), 7.52 (t, J = 6.5 Hz, 1H), 7.45 (d, J = 7.2 Hz, 1H), 7.21 (s, 1H), 3.96 - 3.87 (m, 2H), 3.68 - 3.41 (m, 2H), 3.38 - 3.32 (m, 1H), 3.25 - 3.15 (m, 1H), 3.01 - 2.88 (m, 1H), 2.56 (s, 3H), 2.41 - 2.30 (m, 1H), 1.99 - 1.89 (m, 1H).

### Example 60

### ((S)-4-(6-(2,6-difluoro-3-hydroxyphenyl)-2-((pyrrolidin-3-ylmethyl)amino)quinazolin-4-yl)-2-fluorobe nzonitrile 60

The title compound 60 hydrochloride (30 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 6-(2,6-difluoro-3-hydroxyphenyl)boronic acid.¹H NMR (400 MHz, Methanol-*d*₄) δ 8.23 - 7.80 (m, 6H), 7.05 - 6.88 (m, 2H), 3.88 (q, J = 6.5, 6.1 Hz, 2H), 3.65 - 3.42 (m, 2H), 3.36 - 3.31 (m, 1H), 3.26 - 3.16 (m, 1H), 3.02 - 2.89 (m, 1H), 2.43 - 2,29 (m, 1H), 2.00 - 1.89 (m, 1H).

### Example 61

### (S)-2-fluoro-4-(6-(2-fluoro-3-hydroxyphenyl)-2-((pyrrolidin-3-ylmethyl)amino)quinazolin-4-yl)benzoni trile 61

The title compound 61 hydrochloride (13 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 6-(2-fluoro-3-hydroxy)phenylboronic acid.¹H NMR (400 MHz, Methanol-*d*₄) δ 8.34 - 7.75 (m, 6H), 7.08 (t, J = 7.7 Hz, 1H), 7.02 - 6.93 (m, 2H), 3.95 - 3.86 (m, 2H), 3.69 - 3.43 (m, 2H), 3.39 - 3.33 (m, 1H), 3.24 - 3.14 (m, 1H), 3.02 - 2.89 (m, 1H), 2.41 - 2.29 (m, 1H), 2.01 - 1.91 (m, 1H).

### Example 62

### (S)-3-(4-(4-cyano-3-fluorophenyl)-2-((pyrrolidin-3-ylmethyl)amino)quinazolin-6-yl)benzamide 62

the title compound 62 hydrochloride (13 mg) was prepared using the synthetic route of Example 1 by replacing the raw materialtert-butyl piperidin-4-ylcarbamate of step 2 with (R)-N-tert-butoxycarbonyl-3-(aminomethyl)pyrrolidine, and replacing the raw material (3-fluoro-4-methoxyphenyl)boronic acid of step 3 with 3-aminocarbonylphenylboronic acid.MS m/z (ESI):467.46 [M + 1]⁺.

### Example 63

### 4-(2-(4-Amino-4-methylpiperidin-1-yl)-6-(2-fluoro-6-(trifluoromethyl)phenyl)quinazolin-4-yl)-2-fluoro benzonitrile 63

The title compound 63 hydrochloride (20 mg) was prepared using the synthetic route of Example 1 by replacing the raw materialtert-butyl piperidin-4-ylcarbamate of step 2 with tert-butyl (4-methylpiperidin-4-yl)carbamate, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.13 (t, J = 7.3 Hz, 1H), 7.89 (d, J = 8.7 Hz, 1H), 7.84 - 7.74 (m, 2H), 7.68 (dt, J = 11.3, 3.0 Hz, 5H), 4.43 (dt, J = 14.6, 5.1 Hz, 2H), 3.81 - 3.76 (m, 2H), 1.91 - 1.81 (m, 4H), 1.42 (s, 3H).

### Example 64

### 4-(2-(4-Amino-3,3-difluoropiperidin-1-yl)-6-(2-fluoro-6-(trifluoromethyl)phenyl)quinazolin-4-yl)-2-flu orobenzonitrile 64

The title compound 64 hydrochloride (26 mg) was prepared using the synthetic route of Example 1 by replacing the raw materialtert-butyl piperidin-4-ylcarbamate of step 2 with tert-butyl (3, 3-difluoropiperidin-4-yl)carbamate, and replacing the raw material (3-fluoro-4-methoxyphenyl)boronic acid of step 3 with 2-fluoro-6-(trifluoromethyl)phenylboronic acid.¹H NMR (400 MHz, DMSO-d₆) δ 8.15 (dd, J = 8.0, 6.7 Hz, 1H), 7.84 (dd, J = 9.8, 1.5 Hz, 1H), 7.78 (s, 2H), 7.74 - 7.62 (m, 5H), 5.32 - 5.21 (m, 1H), 4.93 (d, J = 13.6 Hz, 1H), 4.13 - 4.00 (m, 1H), 3.71 (dd, J = 31.2, 14.1 Hz, 1H), 3.38 - 3.29 (m, 1H), 2.30 (d, J = 12.9 Hz, 1H), 1.88 - 1.71 (m, 1H).

### Example 65

### 4-(2-(6-Amino-3-azabicyclo [3.1.0]hexan-3-yl)-6-(2-fluoro-6-(trifluoromethyl)phenyl)quinazolin-4-yl)-2-fluorobenzonitrile 65

The title compound 65 hydrochloride (13 mg) was prepared using the synthetic route of Example 1 by replacing the raw materialtert-butyl piperidin-4-ylcarbamate of step 2 with tert-butyl (3-azabicyclo[3.1.0]-6-hexyl)-carbamate, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.17 - 8.12 (m, 1H), 8.06 (d, J = 8.7 Hz, 1H), 7.84 (dd, J = 8.8, 1.8 Hz, 1H), 7.79 (d, J = 9.8 Hz, 1H), 7.74 - 7.62 (m, 5H), 4.13 (d, J = 11.9 Hz, 2H), 3.80 (dd, J = 11.9, 4.0 Hz, 2H), 2.51 - 2.44 (m, 1H), 2.33 - 2.19 (m, 2H).

### Example 66

### 2-Fluoro-4-(6-(2-fluoro-6-(trifluoromethyl)phenyl)-2-(4-(methylatnino)piperidin-1-yl)quinazolin-4-yl)b enzonitrile 66

The title compound 66 hydrochloride (16 mg) was prepared using the synthetic route of Example 1 by replacing the raw materialtert-butyl piperidin-4-ylcarbamate of step 2 with 4-N-tert-butoxycarbonyl-4-N-methylaminopiperidine, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.14 (dd, J = 8.0, 6.7 Hz, 1H), 7.85 - 7.80 (m, 2H), 7.76 (dd, J = 8.8, 1.8 Hz, 1H), 7.74 - 7.64 (m, 5H), 4.97 (d, J = 13.4 Hz, 2H), 3.38 - 3.27 (m, 1H), 3.13 (t, J = 12.2 Hz, 2H), 2.56 (t, J = 5.3 Hz, 3H), 2.18 (d, J = 12.2 Hz, 2H), 1.69 - 1.55 (m, 2H).

### Example 67

### 4-(2-((3R,4S)-4-amino-3-fluoropiperidin-1-yl)-6-(2-fluoro-6-(trifluoromethyl)phenyl)quinazofin-4-yl)-2-fluorobenzonitrile 67

The title compound 67 hydrochloride (20 mg) was prepared using the synthetic route of Example 1 by replacing the raw materialtert-butyl piperidin-4-ylcarbamate of step 2 with tert-butyl N-[(3R,4S)-3-fluoropiperidin-4-yl]carbamate, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.18 (d, J = 8.6 Hz, 1H), 8.05 - 7.97 (m, 2H), 7.91 (d, J = 1.8 Hz, 1H), 7.80 (dd, J = 15.6, 8.6 Hz, 2H), 7.68 (dd, J = 6.8, 3.8 Hz, 2H), 7.58 - 7.49 (m, 1H), 5.51 - 5.49 (m, 1H), 5.27 (d, J = 48.6 Hz, 1H), 5.17 - 5.14 (m, 1H), 4.00 - 3.68 (m, 2H), 3.65 - 3.52 (m, 1H), 2.30 - 2.12 (m, 2H).

### Example 68

### 4-(2-((3R,4R)-4-amino-3-methylpiperidin-1-yl)-6-(2-fluoro-6-(trifluoromethyl)phenyl)quinazolin-4-yl)-2-fluorobenzonitrile 68

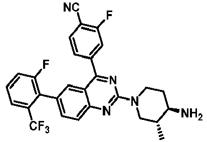

The title compound 68 hydrochloride (16 mg) was prepared using the synthetic route of Example 1 by replacing the raw materialtert-butyl piperidin-4-ylcarbamate of step 2 with tert-butyl N-[(3R,4R)-3-methylpiperidin-4-yl] carbamate, and replacing the raw material (3-fluoro-4-methoxyphenyl)boronic acid of step 3 with 2-fluoro-6-(trifluoromethyl)phenylboronic acid.MS m/z (ESI):524.28 [M + 1] ⁺.

### Example 69

### 4-(2-(3-amino-8-azabicyclo[3.2.1]octan-8-yl)-6-(2-fluoro-6-(trifluoromethyl)phenyl)quinazolin-4-yl)-2-fluorobenzonitrile 69

The title compound 69 hydrochloride (36 mg) was prepared using the synthetic route of Example 1 by replacing the raw materialtert-butyl piperidin-4-ylcarbamate of step 2 with tert-butyl 8-aza-bicyclo[3.2.1]octan-3-ylcarbamate, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.21 - 8.13 (m, 1H), 8.05 (d, J = 8.7 Hz, 2H), 7.85 (dd, J = 8.8, 1.8 Hz, 1H), 7.81 (d, J = 9.7 Hz, 1H), 7.76 - 7.63 (m, 4H), 5.17 - 5.07 (m, 1H), 5.06 - 4.90 (m, 1H), 3.69 (dq, J = 12.0, 6.7 Hz, 1H), 2.22 - 1.75 (m, 8H).

### Example 70

### 4-(2-(3-aminopiperidin-1-yl)-6-(2-fluoro-6-(trifluoromethyl)phenyl)quinazolin-4-yl)-2-fluorobenzonitri le 70

The title compound 70 hydrochloride (16 mg) was prepared using the synthetic route of Example 1 by replacing the raw materialtert-butyl piperidin-4-ylcarbamate of step 2 with 3-tert-butoxycarbonylaminopiperidine, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.18 - 8.10 (m, 1H), 7.86 (t, J = 9.9 Hz, 2H), 7.78 (dd, J = 8.8, 1.8 Hz, 1H), 7.69 (tdd, J = 11.0, 7.2, 2.3 Hz, 5H), 4.76 - 4.60 (m, 1H), 4.40 (d, J = 10.8 Hz, 1H), 3.75 - 3.41 (m, 3H), 3.29 (dd, J = 9.4, 4.9 Hz, 1H), 2.11 - 2.07 (m, 1H), 1.93 - 1.70 (m, 2H), 1.64 - 1.54 (m, 1H).

### Example 71

### 4-(2-(4-amino-3,3-dimethylpiperidin-1-yl)-6-(2-fluoro-6-(trifluoromethyl)phenyl)quinazolin-4-yl)-2-flu orobenzonitrile 71

The title compound 71 hydrochloride (18 mg) was prepared using the synthetic route of Example 1 by replacing the raw materialtert-butyl piperidin-4-ylcarbamate of step 2 with tert-butyl (3,3-dimethylpiperidin-4-yl)carbamate, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.13 (t, J = 7.3 Hz, 1H), 7.91 (d, J = 8.7 Hz, 1H), 7.77 (t, J = 8.4 Hz, 2H), 7.73 - 7.62 (m, 5H), 4.87 (d, J = 13.3 Hz, 1H), 4.64 - 4.61 (m, 1H), 3.27 (t, J = 13.0 Hz, 1H), 3.20 (p, J = 5.2 Hz, 1H), 3.11 (d, J = 13.5 Hz, 1H), 2.03 - 1.97 (m, 1H), 1.80 (tt, J = 12.1, 6.3 Hz, 1H), 1.13 (s, 3H), 0.93 (s, 3H).

### Example 72

### 4-(2-(4-atninoazepan-1-yl)-6-(2-fluoro-6-(trifluoromethyl)phenyl)quinazolin-4-yl)-2-fluorobenzonitrile 72

The title compound 72 hydrochloride (18 mg) was prepared using the synthetic route of Example 1 by replacing the raw materialtert-butyl piperidin-4-ylcarbamate of step 2 with 4-tert-butoxycarbonyl-1H-azepine, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.23 (d, J = 9.5 Hz, 1H), 8.02 (t, J = 7.2 Hz, 2H), 7.91 (s, 1H), 7.83 (dd, J = 17.1, 8.5 Hz, 2H), 7.73 - 7.63 (m, 2H), 7.54 (td, J = 8.8, 3.3 Hz, 1H), 4.60 (d, J = 14.5 Hz, 1H), 4.39 - 4.22 (m, 1H), 4.15 - 3.90 (m, 2H), 3.50 (d, J = 12.0 Hz, 1H), 2.58 - 1.77 (m, 6H).

### Example 73

### 4-(2-(4-(Dimethylamino)piperidin-1-yl)-6-(2-fluoro-6-(trifluoromethyl)phenyl)quinazolin-4-yl)-2-fluor obenzonitrile 73

The title compound 73 hydrochloride (16 mg) was prepared using the synthetic route of Example 1 by replacing the raw materialtert-butyl piperidin-4-ylcarbamate of step 2 with 4-dimethylaminopiperidine, and replacing the raw material (3-fluoro-4-methoxyphenyl)boronic acid of step 3 with 2-fluoro-6-(trifluoromethyl)phenylboronic acid.¹H NMR (400 MHz, DMSO-*d*₆) δ 8.14 (dd, J = 8.0, 6.7 Hz, 1H), 7.89 - 7.74 (m, 3H), 7.73 - 7.63 (m, 5H), 5.06 (d, J = 13.3 Hz, 2H), 3.58 - 3.48 (m, 1H), 3.12 - 3.03 (m, 2H), 2.70 (d, J = 4.9 Hz, 6H), 2.18 (d, J = 11.8 Hz, 2H), 1.75 - 1.61 (m, 2H).

### Example 74

### (R)-4-(2-(3-atninopiperidin-1-yl)-6-(2-fluoro-6-(trifluoromethyl)phenyl)quinazolin-4-yl)-2-fluorobenzo nitrile 74

The title compound 74 hydrochloride (26mg) was prepared using the synthetic route of Example 1 by replacing the raw materialtert-butyl piperidin-4-ylcarbamate of step 2 with (R)-3-tert-butoxycarbonylaminopiperidine, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.14 (t, J = 8.2 Hz, 1H), 8.00 (q, J = 8.9, 8.0 Hz, 2H), 7.92 - 7.84 (m, 2H), 7.81 (d, J = 7.6 Hz, 1H), 7.68 (dd, J = 7.3, 3.8 Hz, 2H), 7.61 - 7.46 (m, 1H), 4.87 - 4.76 (m, 1H), 4.45 - 4.27 (m, 1H), 4.10 - 3.98 (m, 1H), 3.94 - 3.84 (m, 1H), 3.67 - 3.58 (m, 1H), 2.31 - 2.25 (m, 1H), 2.17 - 2.06 (m, 1H), 1.97 - 1.88 (m, 2H).

### Example 75

### (S)-4-(2-(3-atninopiperidin-1-yl)-6-(2-fluoro-6-(trifluoromethyl)phenyl)quinazolin-4-yl)-2-fluorobenzo nitrile 75

The title compound 74 hydrochloride (15 mg) was prepared using the synthetic route of Example 1 by replacing the raw materialtert-butyl piperidin-4-ylcarbamate of step 2 with (S)-3-tert-butoxycarbonylaminopiperidine, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.14 (d, J = 8.2 Hz, 1H), 7.99 (t, J = 8.0 Hz, 2H), 7.93 - 7.85 (m, 2H), 7.81 (d, J = 6.7 Hz, 1H), 7.72 - 7.63 (m, 2H), 7.54 (d, J = 8.0 Hz, 1H), 5.08 - 5.02 (m, 1H), 4.43 - 4.35 (m, 1H), 4.07 - 3.98 (m, 1H), 3.93 - 3.84 (m, 1H), 3.69 - 3.55 (m, 1H), 2.37 - 2.19 (m, 1H), 2.14 - 2.06 (m, 1H), 2.00 - 1.85 (m, 2H).

### Example 76

### 2-Fluoro-4-(6-(2-fluoro-6-(trifluoromethyl)phenyl)-2-(octahydro-5H-pyrrolo[3,2-c]pyridin-5-yl)quinaz olin-4-yl)benzonitrile 76

The title compound 76 hydrochloride (28 mg) was prepared using the synthetic route of Example 1 by replacing the raw materialtert-butyl piperidin-4-ylcarbamate of step 2 with tert-butyl octahydro-1H-pyrrolo[3,2-C]pyridine-1-carboxylate, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.15 (t, J = 7.9 Hz, 1H), 8.04 - 7.94 (m, 2H), 7.89 (d, J = 5.3 Hz, 1H), 7.85 - 7.76 (m, 2H), 7.68 (t, J = 4.1 Hz, 2H), 7.54 (dt, J = 12.3, 5.2 Hz, 1H), 4.53 - 4.42 (m, 1H), 4.36 - 4.21 (m, 2H), 4.13 - 4.04 (m, 1H), 3.95 - 3.80 (m, 1H), 3.58 - 3.49 (m, 1H), 3.40 (d, J = 16.4 Hz, 1H), 2.95 - 2.84 (m, 1H), 2.45 - 2.17 (m, 3H), 2.07 - 1.94 (m, 1H).

### Example 77

### 2-Fluoro-4-(6-(2-fluoro-6-(trifluoromethyl)phenyl)-2-(1,8-diazaspiro[4.5]dec-8-yl)quinazolin-4-yl)benz onitrile 77

The title compound 77 hydrochloride (18 mg) was prepared using the synthetic route of Example 1 by replacing the raw materialtert-butyl piperidin-4-ylcarbamate of step 2 with tert-butyl 1,8-diazaspiro[4.5]decane-1-carboxylate, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.04 (d, J = 8.0 Hz, 1H), 7.97 (s, 1H), 7.91 - 7.71 (m, 3H), 7.67 - 7.64 (m, 1H), 7.51 (d, J = 7.4 Hz, 1H), 4.68 - 4.63 (m, 2H), 3.92 - 3.87 (m, 2H), 3.49 - 3.45 (m, 2H), 2.15 - 2.14 (m, 8H).

### Example 78

### 4-(2-(4-aminopiperidin-1-yl)-7-fluoro-6-(2-fluorophenyl)quinazolin-4-yl)-2-fluorobenzonitrile 78

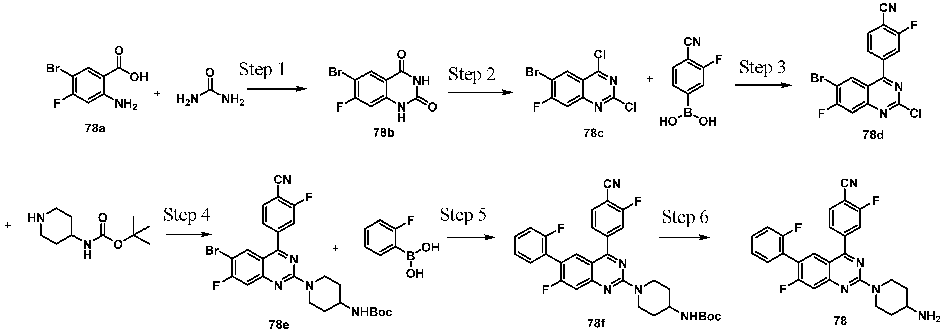

### Step 1

### 6-Bromo-7-fluoroquinazolin-2,4(1H,3H)-dione78b

Compound 2-amino-5-bromo-4-fluorobenzoic acid **78a** (1 g, 4.27 mmol, Bide Pharmatech Ltd.) andurea(2.56 g, 42.73 mmol) were ground to afford a homogeneous mixture, then heated to 180 °C and reacted for 3 hours under nitrogen protection. After cooling, the resulting solid mixture was ground with water, filtered, washed with water and dried to give the crude product **78b** (1.1 g) (light yellow solid), which was directly used inthe next step without purification.

### Step 2

### 6-Bromo-2,4-dichloro-7-fluoroquinazoline 78c

The crude product 78b (1.1 g, 4.25 mmol) was dissolved in phosphorus oxychloride (2.38 mL, 25.5 mmol) and then N,N-diethylaniline (2.03 mL, 12.75 mmol) was added. The reaction mixture was refluxed at 110°C for 3 hours. The reaction solution was cooled and concentrated under reduced pressure, then poured into cold water, and extracted with dichloromethanefor 3 times. The organic layers were combined, washed with saturated sodium chloride solution, dried, concentrated, and purified by silica gel column chromatography (EA:PE; 1:10 to 1:60) to give the title compound **78c** (0.56g) (yellow solid), yield: 45%.¹H NMR (400 MHz, Chloroform-*d*) δ 8.54 (d, J = 7.0 Hz, 1H), 7.70 (d, J = 8.4 Hz, 1H).

### Step 3

### 4-(6-bromo-2-chloro-7-fluoroquinazolin-4-yl)-2-fluorobenzonitrile 78d

To a mixture of compound 6-bromo-2,4-dichloro-7-fluoroquinazoline **78c** (300 mg, 1.01 mmol,), bis(triphenylphosphine)palladium(II) chloride (42.5 mg, 0.06 mmol), potassium phosphate (430 mg, 2.02 mmol) and MeCN/H2O (8:1) (40 mL) was added (4-cyano-3-fluorophenyl) boronic acid (167.2 mg, 1.01 mmol Shanghai Haohong Biomedical Technology Co.) The reaction mixture was heated at 100 °C for 15 min with stirring under nitrogen protection. The reaction solutionwas then cooled and concentrated under reduced pressure, pulped with ethyl acetate, filtered and dried to give compound **78d** (235mg) (white solid), yield: 61%.¹H NMR (400 MHz, Chloroform-*d*) δ 8.24 (d, J = 7.0 Hz, 1H), 7.90 (dd, J = 8.2, 6.3 Hz, 1H), 7.79 (d, J = 8.5 Hz, 1H), 7.69 - 7.67 (m, 1H), 7.66 (q, J = 1.6 Hz, 1H).

### Step 4

### Tert-butyl (1-(6-bromo-4-(4-cyano-3-fluorophenyl)-7-fluoroquinazolin-2-yl)piperidin-4-yl)carbamate 78e

Compound **78d** (235 mg, 0.62 mmol), diisopropylethylamine (0.216 mL, 1.24 mmol), and tert-butyl piperidin-4-ylcarbamate (186 mg, 0.93 mmol, Bide Pharmatech Ltd.) were dissolved in N,N-dimethylformamide (6 ml). The reaction mixture was stirred at room temperature overnight, the reaction solution was then concentrated under reduced pressure, added with 15mL of water and 15mL of ethyl acetate, mixed homogeneously and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:10 to 1:5) to give the title compound **78e** (214mg) (yellow solid), yield: 64%.¹H NMR (400 MHz, Chloroform-*d*) δ 7.83 (t, J = 3.3 Hz, 1H), 7.82 - 7.80 (m, 1H), 7.57 (dd, J = 4.4, 1.6 Hz, 1H), 7.55 (dd, J = 5.7, 1.3 Hz, 1H), 7.30 (d, J = 9.9 Hz, 1H), 4.83 (dd, J = 13.6, 3.9 Hz, 2H), 4.52 (d, J = 7.9 Hz, 1H), 3.76 (s, 1H), 3.14 (ddd, J = 14.0, 11.6, 2.7 Hz, 2H), 2.10 - 2.03 (m, 2H), 1.45 (s, 9H), 1.42 - 1.34 (m, 2H).

### Step 5

### Tert-butyl

### (1-(4-(4-cyano-3-fluorophenyl)-7-fluoro-6-(2-fluorophenyl)quinazolin-2-yl)piperidin-4-yl)carbatnate 78f

To a mixture of compound 78e (50 mg, 0.09 mmol), [1,1'-bis(diphenylphosphino)ferrocene]palladium(II) dichloride dichloromethane complex (7.3 mg, 0.009 mol), sodium carbonate (28.6 mg, 0.27 mmol) and tetrahydrofuran/water (5:1) (2 mL) was added (2-fluorophenyl)boronic acid (25.7 mg. 0.18 mmol, Shanghai Haohong Biomedical Technology Co.) The reaction mixture was stirred at 70 °C for 16 h under nitrogen protection. The reaction solution was then cooled and concentrated under reduced pressure, added with 15 mL of water and 15 mL of ethyl acetate, mixed homogeneously, and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:8 to 1:4) to give the title compound **78f** (34 mg) (yellow solid), yield: 66%.¹H NMR (400 MHz, Chloroform-d) δ 7.78 (dd, *J*= 8.1, 6.3 Hz, 1H), 7.68 (d, *J*= 7.9 Hz, 1H), 7.62 (dd, *J*= 4.9, 1.6 Hz, 1H), 7.60 (dd, *J*= 6.3, 1.4 Hz, 1H), 7.42 - 7.31 (m, 3H), 7.25 - 7.12 (m, 2H), 4.89 (d, *J*= 13.4 Hz, 2H), 4.49 (s, 1H), 3.78 (s, 1H), 3.18 (td, *J*= 12.5, 11.3, 2.7 Hz, 2H), 2.12 - 2.05 (m, 2H), 1.46 (s, 9H), 1.44 - 1.36 (m, 2H).

### Step 6

### 4-(2-(4-aminopiperidin-1-yl)-7-fluoro-6-(2-fluorophenyl)quinazolin-4-yl)-2-fluorobenzonitrile 78

To a solution of Compound **78f** (34 mg) in 2 mL of dichloromethane was added 2 mL of 4 N hydrogen chloride-1,4-dioxane solution. The reaction mixture was stirred at room temperature for 2 hours. The reaction solution was then concentrated under reduced pressure to give the title compound 78 hydrochloride (26 mg) (yellow solid), yield: 88%.¹H NMR (400 MHz, Methanol-d₄) δ 8.02 (t, J = 6.6 Hz, 1H), 7.96 (d, J = 7.1 Hz, 1H), 7.91 (d, J = 9.2 Hz, 1H), 7.85 (d, J = 7.0 Hz, 1H), 7.80 (d, J = 10.4 Hz, 1H), 7.50 (p, J = 6.6, 6.0 Hz, 2H), 7.31 (t, J = 7.2 Hz, 1H), 7.25 (dd, J = 10.3, 8.2 Hz, 1H), 5.09 (s, 2H), 3.71 - 3.59 (m, 1H), 3.46 (t, J = 12.4 Hz, 2H), 2.30 (d, J = 10.8 Hz, 2H), 1.88 (s, 2H).

### Example 79

### 4-(2-(4-Aminopiperidin-1-yl)-7-fluoro-6-(2-(trifluoromethyl)phenyl)quinazolin-4-yl)-2-fluorobenzonitr ile 79

The title compound 79hydrochloride (30 mg)was prepared using the synthetic route of Example 78 by replacing the raw material 2-fluorophenylboronic acid in step 5 with 2-trifluoromethylphenylboronic acid. ¹H NMR (400 MHz, Methanol-d₄) δ 7.96 (t, J = 6.9 Hz, 1H), 7.86 - 7.60 (m, 7H), 7.46 (d, J = 7.2 Hz, 1H), 5.08 (d, J = 12.8 Hz, 2H), 3.59 (t, J = 4.6 Hz, 1H), 3.35 (d, J = 12.6 Hz, 2H), 2.29 - 2.16 (m, 2H), 1.80 (d, J = 11.9 Hz, 2H).

### Example 80

### 4-(2-(4-aminopiperidin-1-yl)-7-fluoro-6-(2-fluorophenyl)quinazolin-4-yl)-2-fluorobenzonitrile 80

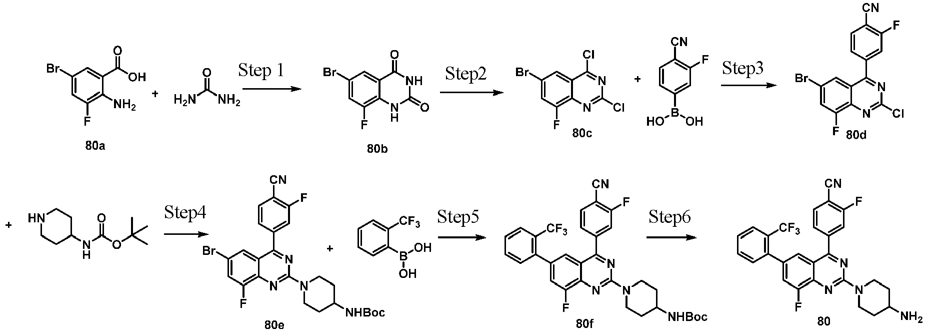

### Step 1

### 6-Bromo-8-fluoroquinazolin-2,4(1H,3H)-dione 80b

Compound 2-amino-5-bromo-3-fluorobenzoic acid **80a** (1 g, 4.27 mmol, Bide Pharmatech Ltd.) andurea(2.56 g, 42.73 mmol) were ground to afford a homogeneous mixture, then heated to 180 °C and reacted for 3 hours under nitrogen protection. After cooling,the resulting solid mixture was ground with water, filtered, washed with water and dried to give the crude product **80b** (1.05 g) (light yellow solid), which was directly used inthe next step without purification.

### Step 2

### 6-Bromo-2,4-dichloro-8-fluoroquinazoline 80c

To a solution of crude product **80b** (1.05 g, 4.05 mmol) in phosphorus oxychloride (2.27 mL, 24.32 mmol) was added N,N-diethylaniline (1.93 mL, 12.16 mmol). The reaction mixture was refluxed at 110°C for 3 hours. The reaction solution was cooled and concentrated under reduced pressure, then poured into cold water, and extracted with dichloromethane for 3 times. The organic layers were combined, washed with saturated sodium chloride solution, dried, concentrated, and purified by silica gel column chromatography (EA:PE; 1:100 to 1:60) to give the title compound **80c** (0.81g) (pale yellow solid), yield: 68%.¹H NMR (400 MHz, Chloroform-*d*) δ 8.18 (d, J = 2.0 Hz, 1H), 7.78 (dd, J = 8.8, 2.9 Hz, 1H).

### Step 3

### 4-(6-bromo-2-chloro-8-fluoroquinazolin-4-yl)-2-fluorobenzonitrile 80d

To a mixture of compound 6-bromo-2,4-dichloro-8-fluoroquinazoline **80c** (600mg, 2.02 mmol,), bis(triphenylphosphine)palladium(II) chloride (85mg, 0.12 mmol), potassium phosphate (860mg, 4.04 mmol) and MeCN/H₂O (8:1) (18 mL) was added (4-cyano-3-fluorophenyl) boronic acid (334.4mg, 2.02 mmol, Shanghai Haohong Biomedical Technology Co.). The reaction mixture was heated at 100 °C for 15 min with stirring under nitrogen protection. The reaction solitionwas then cooled and concentrated under reduced pressure, pulped with ethyl acetate, filtered and dried to give compound **80d** (490mg) (white solid), yield: 64%.¹H NMR (400 MHz, Chloroform-d) δ 7.95 (t, J = 1.6 Hz, 1H), 7.90 (dd, J = 8.1, 6.3 Hz, 1H), 7.82 (dd, J = 8.7, 1.9 Hz, 1H), 7.69 (s, 1H), 7.67 (s, 1H).

### Step 4

### Tert-butyl (1-(6-bromo-4-(4-cyano-3-fluorophenyl)-8-fluoroquinazolin-2-yl)piperidin-4-yl)carbamate 80e

Compound 4-(6-bromo-2-chloro-8-fluoroquinazolin-4-yl)-2-fluorobenzonitrile **80d** (300mg, 0.79mmol), diisopropylethylamine (0.274 mL, 1.58mmol), and tert-butyl piperidin-4-ylcarbamate (237mg, 1.18mmol, Bide Pharmatech Ltd.) were dissolved in N,N-dimethylformamide (8ml).The reaction mixture was stirred at room temperature overnight. The reacxtion solutionwas then concentrated under reduced pressure, added with 15mL of water and 15mL of ethyl acetate, mixed homogeneously and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:10 to 1:5) to give the title compound **80e** (210mg) (yellow solid), yield: 49%.¹H NMR (400 MHz, Chloroform-*d*) δ 7.82 (dd, J = 8.2, 6.3 Hz, 1H), 7.61 - 7.53 (m, 3H), 7.48 (dd, J = 9.6, 2.0 Hz, 1H), 4.86 (d, J = 13.5 Hz, 2H), 4.51 (s, 1H), 3.76 (s, 1H), 3.22 - 3.11 (m, 2H), 2.08 (dd, J = 13.0, 3.7 Hz, 2H), 1.45 (s, 9H), 1.43 - 1.34 (m, 2H).

### Step 5

### Tert-butyl

### (1-(4-(4-cyano-3-fluorophenyl)-8-fluoro-6-(2-trifluoromethylphenyl)quinazolin-2-yl)piperidin-4-yl)carbamat e 80f

To a mixture of compound tert-butyl (1-(6-bromo-4-(4-cyano-3-fluorophenyl)-8-fluoroquinazolin-2-yl)piperidin-4-yl)carbamate **80e** (50 mg, 0.09 mmol), [1,1'-bis(diphenylphosphino)ferrocene]palladium(II) dichloride dichloromethane complex (7.3 mg, 0.009 mol), sodium carbonate (28.6 mg, 0.27 mmol ) and tetrahydrofuran/water (5:1) (2 mL) was added (2-trifluoromethylfluorophenyl)boronic acid (34.9mg, 0.18 mmol, Shanghai Haohong Biomedical Science and Technology Co.). The reaction mixture was stirred at 70 °C for 16 h under nitrogen protection. The reaction solution was then cooled and concentrated under reduced pressure, added with 15 mL of water and 15 mL of ethyl acetate, mixed homogeneously, and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:8 to 1:4) to give the title compound **80f** (42 mg) (yellow solid), yield: 75%.¹H NMR (400 MHz, Chloroform-*d*) δ 7.80 - 7.71 (m, 2H), 7.70 - 7.47 (m, 3H), 7.43 - 7.36 (m, 2H), 7.31 (d, J = 7.6 Hz, 1H), 4.92 (d, J = 13.6 Hz, 2H), 4.52 (s, 1H), 3.77 (s, 1H), 3.25 - 3.15 (m, 2H), 2.09 (dd, J = 13.2, 3.8 Hz, 2H), 1.46 (s, 9H), 1.43 - 1.36 (m, 2H).

### Step 6

### 4-(2-(4-atninopiperidin-1-yl)-8-fluoro-6-(2-trifluoromethylphenyl)quinazolin-4-yl)-2-fluorobenzonitrile 80

Compound tert-butyl (1-(4-(4-cyano-3-fluorophenyl)-8-fluoro-6-(2-trifluoromethylphenyl)quinazofin-2-yl)piperidin-4-yl)carbamat e **80f** (42 mg) was dissolved in 2 mL of dichloromethane, and 2 mL ofa solution of 4N hydrogen chloride in 1,4-dioxane was added.The reaction mixture was stirred at room temperature for 2 hours. The reaction solution was then concentrated under reduced pressure to give the title compound 80 hydrochloride (34 mg) (yellow solid), yield: 97%.¹H NMR (400 MHz, Methanol-*d*₄) δ 7.93 (dd, J = 8.0, 6.5 Hz, 1H), 7.80 (dd, J = 7.9, 1.3 Hz, 1H), 7.74 (dd, J = 9.7, 1.4 Hz, 1H), 7.71 (dd, J = 8.0, 1.5 Hz, 1H), 7.67 (td, J = 7.7, 1.3 Hz, 1H), 7.58 (t, J = 7.7 Hz, 1H), 7.50 (dd, J = 11.0, 1.8 Hz, 1H), 7.47 - 7.42 (m, 2H), 5.15 (dt, J = 13.6, 2.7 Hz, 2H), 3.50 (tt, J = 11.6, 4.2 Hz, 1H), 3.17 (ddd, J = 14.2, 12.3, 2.6 Hz, 2H), 2.19 - 2.12 (m, 2H), 1.65 (qd, J = 12.3, 4.3 Hz, 2H).

### Example 81

### 4-(2-(4-aminopiperidin-1-yl)-8-fluoro-6-(2-fluoro)phenyl)quinazolin-4-yl)-2-fluorobenzonitrile 81

The title compound **81**hydrochloride (40 mg) was prepared using the synthetic route of Example **80** by replacing the raw material 2-trifluoromethylphenylboronic acid in step 5 with 2-fluorophenylboronic acid.¹H NMR (400 MHz, Methanol-*d*₄) δ 7.98 (t, J = 7.1 Hz, 1H), 7.84 - 7.74 (m, 3H), 7.73 (s, 1H), 7.51 (t, J = 7.8 Hz, 1H), 7.39 (t, J = 6.8 Hz, 1H), 7.26 (t, J = 7.6 Hz, 1H), 7.20 (dd, J = 11.3, 8.2 Hz, 1H), 5.12 (d, J = 13.4 Hz, 2H), 3.57 - 3.44 (m, 1H), 3.21 (t, J = 12.8 Hz, 2H), 2.18 (d, J = 12.3 Hz, 2H), 1.77 - 1.60 (m, 2H).

### Example 82

### 4-(2-(4-aminopiperidin-1-yl)-8-fluoro-6-(2-fluoro-6-trifluoromethyl)phenyl)quinazolin-4-yl)-2-fluorobe nzonitrile 82

The title compound 82 hydrochloride (24 mg) was prepared using the synthetic route of Example **80** by replacing the raw material 2-trifluoromethylphenylboronic acid ofstep 5 with 2-fluoro-6-trifluoromethylphenylboronic acid.¹H NMR (400 MHz, Methanol-*d*₄) δ 7.93 (ddd, J = 8.3, 6.5, 1.9 Hz, 1H), 7.74 - 7.58 (m, 4H), 7.53 - 7.44 (m, 3H), 5.15 (d, J = 13.7 Hz, 2H), 3.50 (dq, J = 11.7, 5.9, 4.3 Hz, 1H), 3.22 - 3.11 (m, 2H), 2.20 - 2.13 (m, 2H), 1.72 - 1.60 (m, 2H).

### Example 83

### 4-(2-((3S,4R)-4-amino-3-fluoropiperidin-1-yl)-6-(2-fhioro-6-(trifluoromethyl)phenyl)quinazolin-4-yl)-2-fluorobenzonitrile 83

The title compound 83 hydrochloride (33 mg) was prepared using the synthetic route of Example 1 by replacing the raw materialtert-butyl piperidin-4-ylcarbamate of step 2 with tert-butyl N-[(3S,4R)-3-fluoropiperidin-4-yl]carbamate, and replacing the raw material (3-fluoro-4-methoxyphenyl)boronic acid of step 3 with 2-fluoro-6-(trifluoromethyl)phenylboronic acid.

¹H NMR (400 MHz, DMSO-*d*₆) δ 8.13 (t, J = 7.3 Hz, 1H), 7.80 (d, J = 9.8 Hz, 1H), 7.74 (s, 2H), 7.72 - 7.61 (m, 5H), 5.37 - 5.25 (m, 1H), 5.16 (d, J = 49.1 Hz, 1H), 5.00 (d, J = 13.5 Hz, 1H), 3.40 (dd, J = 40.2, 14.9 Hz, 1H), 3.16 (t, J = 12.8 Hz, 1H), 1.99 (dq, J = 9.5, 6.1 Hz, 2H), 1.89 - 1.73 (m, 1H).

### Example 84

### 4-(2-((3R,4R)-4-amino-3-fluoropiperidin-1-yl)-6-(2-fluoro-6-(trifluoromethyl)phenyl)quinazolin-4-yl)-2-fluorobenzonitrile 84

The title compound 84 hydrochloride (36 mg) was prepared using the synthetic route of Example 1 by replacing the raw materialtert-butyl piperidin-4-ylcarbamate of step 2 with tert-butyl N-[(3R,4R)-3-fluoropiperidin-4-yl]carbamate, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.08 (d, J = 8.5 Hz, 1H), 7.99 (t, J = 7.0 Hz, 1H), 7.94 (d, J = 8.6 Hz, 1H), 7.87 (s, 1H), 7.82 (d, J = 9.5 Hz, 1H), 7.77 (d, J = 7.7 Hz, 1H), 7.67 (qd, J = 7.7, 3.3 Hz, 2H), 7.56 - 7.48 (m, 1H), 5.36 (d, J = 12.0 Hz, 1H), 5.05 (d, J = 12.8 Hz, 1H), 4.82 (s, 1H), 3.77 (d, J = 12.0 Hz, 1H), 3.55 - 3.38 (m, 2H), 2.38 (d, J = 12.4 Hz, 1H), 1.99 (d, J = 14.1 Hz, 1H).

### Example 85

### 4-(2-((3 S,4S)-4-amino-3-fluoropiperidin-1-yl)-6-(2-fluoro-6-(trifluoromethyl)phenyl)quinazofin-4-yl)-2 -fluorobenzonitrile 85

The title compound 85 hydrochloride (32 mg) was prepared using the synthetic route of Example 1 by replacing the raw materialtert-butyl piperidin-4-ylcarbamate of step 2 with tert-butyl N-[(3S,4S)-3-fluoropiperidin-4-yl]carbamate, and replacing the raw material (3-fluoro-4-methoxyphenyl)boronic acid of step 3 with 2-fluoro-6-(trifluoromethyl)phenylboronic acid.

¹H NMR (400 MHz, Methanol-*d*₄) δ 8.09 (d, J = 8.7 Hz, 1H), 7.99 (t, J = 7.0 Hz, 1H), 7.94 (d, J = 8.4 Hz, 1H), 7.87 (s, 1H), 7.82 (d, J = 9.5 Hz, 1H), 7.77 (d, J = 7.7 Hz, 1H), 7.67 (dd, J = 7.3, 3.7 Hz, 2H), 7.56 - 7.48 (m, 1H), 5.42 - 5.30 (m, 1H), 5.05 (d, J = 13.0 Hz, 1H), 4.83 (s, 1H), 3.83 - 3.72 (m, 1H), 3.48 (d, J = 12.7 Hz, 2H), 2.38 (s, 1H), 2.09 - 1.92 (m, 1H).

### Example 86

### (S)-4-(2-(3-atninoazepan-1-yl)-6-(2-fluoro-6-(trifluoromethyl)phenyl)quinazolin-4-yl)-2-fluorobenzonit rile 86

The title compound 86 hydrochloride (18 mg)was prepared using the synthetic route of Example 1 by replacing the raw materialtert-butyl piperidin-4-ylcarbamate of step 2 with tert-butyl (S)-azepan-3-ylcarbamate, and replacing the raw material (3-fluoro-4-methoxyphenyl)boronic acid of step 3 with 2-fluoro-6-(trifluoromethyl)phenylboronicacid.

¹H NMR (400 MHz, Methanol-*d*₄) δ 8.31 (d, J = 8.4 Hz, 1H), 8.10 - 8.00 (m, 2H), 7.91 (d, J = 11.5 Hz, 2H), 7.84 (d, J = 7.7 Hz, 1H), 7.72 - 7.63 (m, 2H), 7.54 (t, J = 7.2 Hz, 1H), 4.60 (d, J = 14.3 Hz, 1H), 4.32 (d, J = 14.0 Hz, 1H), 4.15 - 4.03 (m, 2H), 3.79 - 3.71 (m, 1H), 2.32 - 1.98 (m, 4H), 1.93 - 1.66 (m, 2H).

### Example 87

### (R)-4-(2-(3-atninoazepan-1-yl)-6-(2-fluoro-6-(trifluoromethyl)phenyl)quinazolin-4-yl)-2-fluorobenzonit rile 87

The title compound 87 hydrochloride (25 mg) was prepared using the synthetic route of Example 1 by replacing the raw materialtert-butyl piperidin-4-ylcarbamate of step 2 with tert-butyl (R)-azepan-3-ylcarbamate, and replacing the raw material (3-fluoro-4-methoxyphenyl)boronic acid of step 3 with 2-fluoro-6-(trifluoromethyl)phenylboronicacid.¹H NMR (400 MHz, DMSO-*d*₆) δ 8.37 (s, 1H), 8.13 (t, J = 7.3 Hz, 1H), 8.00 (t, J = 10.8 Hz, 1H), 7.91 - 7.57 (m, 6H), 4.41 - 4.31 (m, 1H), 4.18 - 3.75 (m, 3H), 3.57 (d, J = 46.6 Hz, 1H), 1.96 (d, J = 8.1 Hz, 2H), 1.92 - 1.61 (m, 3H), 1.48 - 1.43(m, 1H).

### Example 88

### (S)-4-(2-(4-atninoazepan-1-yl)-6-(2-fluoro-6-(trifluoromethyl)phenyl)quinazolin-4-yl)-2-fluorobenzonit rile 88

Tthe title compound 88 hydrochloride (22 mg) was prepared using the synthetic route of Example 1 by replacing the raw materialtert-butyl piperidin-4-ylcarbamate of step 2 with (S)-4-tert-butyloxycarbonyl-1H-azepine, and replacing the raw material (3-fluoro-4-methoxyphenyl)boronic acid of step 3 with 2-fluoro-6-(trifluoromethyl)phenylboronicacid.¹H NMR (400 MHz, Methanol-d₄) δ 8.19 (d, J = 8.4 Hz, 1H), 8.06 - 7.98 (m, 2H), 7.92 - 7.89 (m, 1H), 7.81 (dd, J = 16.7, 8.6 Hz, 2H), 7.68 (dd, J = 6.9, 3.7 Hz, 2H), 7.58 - 7.49 (m, 1H), 4.65 - 3.89 (m, 3H), 3.52 - 3.44 (m, 1H), 2.56 - 1.79 (m, 3H).

### Example 89

### 4-(2-(4-atninoazepan-1-yl)-6-(2-fluoro-6-(trifluoromethyl)phenyl)quinazolin-4-yl)-2-fluorobenzonitrile 89

The title compound 89 hydrochloride (59 mg) was prepared using the synthetic route of Example 1 by replacing the raw materialtert-butyl piperidin-4-ylcarbamate of step 2 with (R)-4-tert-butyloxycarbonyl-1H-azepine, and replacing the raw material (3-fluoro-4-methoxyphenyl)boronic acid of step 3 with 2-fluoro-6-(trifluoromethyl)phenylboronic acid.¹H NMR (400 MHz, Methanol-*d*₄) δ 8.30 - 8.16 (m, 1H), 8.06 - 7.98 (m, 2H), 7.93 - 7.90 (m, 1H), 7.83 (dd, J = 17.1, 8.5 Hz, 2H), 7.68 (dd, J = 6.2, 3.8 Hz, 2H), 7.54 (td, J = 9.0, 3.3 Hz, 1H), 4.69 - 3.89 (m, 4H), 2.56 - 1.78 (m, 2H).

### Example 90

### (S)-4-(2-(3-aminopyrrolidin-1-yl)-6-(2-fluoro-6-(trifluoromethyl)phenyl)quinazolin-4-yl)-2-fluorobenz onitrile 90

The title compound 90 (30 mg)was prepared using the synthetic route of Example 1 by replacing the raw materialtert-butyl piperidin-4-ylcarbamate of step 2 with (S)-3-tert-butoxycarbonylaminopyrrolidine, and replacing the raw material (3-fluoro-4-methoxyphenyl)boronic acid of step 3 with 2-fluoro-6-(trifluoromethyl)phenylboronicacid.¹H NMR (400 MHz, Chloroform-*d*) δ 7.74 (dd, J = 8.7, 6.0 Hz, 2H), 7.63 - 7.55 (m, 5H), 7.47 (td, J = 8.0, 5.1 Hz, 1H), 7.33 (t, J = 8.4 Hz, 1H), 3.94 (ddd, J = 19.1, 15.3, 6.5 Hz, 2H), 3.79 (q, J = 11.2, 9.0 Hz, 2H), 3.62 - 3.43 (m, 1H), 2.23 (dt, J = 19.8, 7.0 Hz, 2H).

### Example 91

### (R)-4-(2-(3-aminopyrrolidin-1-yl)-6-(2-fluoro-6-(trifluoromethyl)phenyl)quinazolin-4-yl)-2-fluorobenz onitrile 91

The title compound 91 (38 mg)was prepared using the synthetic route of Example 1 by replacing the raw materialtert-butyl piperidin-4-ylcarbamate of step 2 with (R)-3-tert-butoxycarbonylaminopyrrolidine, and replacing the raw material (3-fluoro-4-methoxyphenyl)boronic acid of step 3 with 2-fluoro-6-(trifluoromethyl)phenylboronicacid.¹H NMR (400 MHz, Chloroform-*d*) δ 7.77 - 7.72 (m, 2H), 7.62 (q, J = 1.5 Hz, 1H), 7.60 (dd, J = 4.3, 1.4 Hz, 2H), 7.58 - 7.56 (m, 2H), 7.47 (td, J = 8.1, 5.2 Hz, 1H), 7.33 (t, J = 8.5 Hz, 1H), 3.94 (ddd, J = 18.9, 12.6, 6.4 Hz, 2H), 3.79 (td, J = 11.9, 9.9, 6.5 Hz, 2H), 3.50 (dd, J = 11.4, 4.6 Hz, 1H), 2.24 (dq, J = 15.0, 7.8, 7.1 Hz, 1H), 1.86 (dq, J = 12.7, 6.1 Hz, 1H).

### Example 92

### 4-(2-(4-atninopiperidin-1-yl)-6-(2-(trifluoromethyl)phenyl)-5,6,7, 8-tetrahydropyrido [4,3-d]pyrimidin-4-yl)-2-fluorobenzonitrile 92

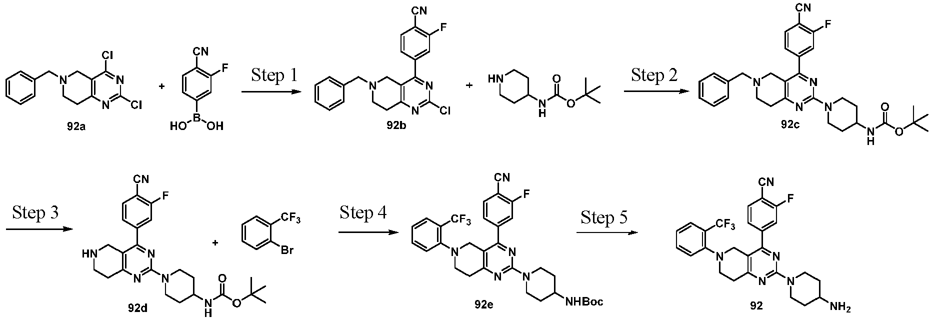

### Step 1

### 4-(6-Benzyl-2-chloro-5,6,7,8-tetrahydropyrido[4,3-d]pyrimidin-4-yl)-2-fluorobenzonitrile 92b

To a mixture of compound 6-benzyl-2,4-dichloro-5,6,7,8-tetrahydropyrido[4,3-d]pyrimidine **92a**(1 g, 3.38 mmol), bis(triphenylphosphine)palladium(II) chloride (237mg, 0.34 mmol), potassium phosphate (1.43g, 6.75 mmol) and MeCN/H₂O (8:1) (18 mL) was added (4-cyano-3-fluorophenyl) boronic acid (612mg, 3.71 mmol, Shanghai Haohong Biomedical Technology Co.). The reaction mixture was heated at 100 °C for 15 min with stirring under nitrogen protection. The reaction solution was then cooled and concentrated under reduced pressure, pulped with ethyl acetate, filtered and dried to give compound **92b** (908 mg) (white solid), yield: 70.6%.

### Step 2

### Tert-butyl

### (1-(6-benzyl-4-(4-cyano-3-fluorophenyl)-5,6,7,8-tetrahydropyrido[4,3-d]pyrimidin-2-yl)piperidin-4-ylcarba mate 92c

Compound 4-(6-benzyl-2-chloro-5,6,7,8-tetrahydropyrido[4,3-d]pyrimidin-4-yl)-2-fluorobenzonitrile **92b** (600 mg, 1.58 mmol), diisopropylethylamine (0.55 mL, 3.15 mmol), and tert-butyl piperidin-4-ylcarbamate (473mg, 2.36mmol, Bide Pharmatech Ltd.) were dissolved in N,N-dimethylformamide (8ml). The reaction mixture was stirred at room temperature overnight.The reaction solution was then concentrated under reduced pressure, added with 15mL of water and 15 mL of ethyl acetate, mixed homogeneously and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:10 to 1:5) to give the title compound **92c** (760mg) (white solid), yield: 88.9%.¹H NMR (400 MHz, Chloroform-d) δ 7.66 (t, J = 7.1 Hz, 1H), 7.39 (d, J = 8.8 Hz, 2H), 7.35 - 7.25 (m, 5H), 4.66 (d, J = 13.3 Hz, 2H), 4.46 (s, 1H), 3.74 - 3.66 (m, 1H), 3.64 (s, 2H), 3.49 (s, 2H), 3.01 (t, J = 11.9 Hz, 2H), 2.85 (d, J = 5.9 Hz, 2H), 2.78 (d, J = 6.1 Hz, 2H), 2.00 (d, J = 12.4 Hz, 2H), 1.45 (s, 9H), 1.32 (dtt, J = 12.1, 8.7, 4.5 Hz, 2H).

### Step 3

### Tert-butyl

### (1-(4-(4-cyano-3-fluorophenyl)-5,6,7,8-tetrahydropyrido[4,3-d]pyrimidin-2-yl)piperidin-4-yl)carbamate 92d

A mixture of compound tert-butyl (1-(6-benzyl-4-(4-cyano-3-fluorophenyl)-5,6,7,8-tetrahydropyrido[4,3-d]pyrimidin-2-yl)piperidin-4-ylcarba mate **92c** (600 mg, 1.11 mmol), 10% Pd-C catalyst (60 mg) and methanol (10 mL) was stirred at room temperature under hydrogen until the hydrogen absorption stoped.The catalyst was filtered out, and the filtrate was evaporated and purified by silica gel column chromatography (DCM:MeOH; 1:20 to 1:15) to give the title compound **92d** (210 mg) (white solid), yield: 42.0%.¹H NMR (400 MHz, Chloroform-d) δ 7.68 (t, J = 7.1 Hz, 1H), 7.39 (d, J = 8.7 Hz, 2H), 4.65 (d, J = 13.6 Hz, 1H), 4.51 (d, J = 7.7 Hz, 2H), 3.83 (s, 2H), 3.69 (s, 1H), 3.19 (t, J = 6.2 Hz, 2H), 3.05 - 2.96 (m, 2H), 2.80 (t, J = 6.1 Hz, 2H), 2.03 - 1.96 (m, 2H), 1.44 (s, 9H), 1.33 (qd, J = 11.8, 4.3 Hz, 2H).

### Step 4

### Tert-butyl

### (1-(4-(4-cyano-3-fluorophenyl)-6-(2-(trifluoromethyl)phenyl)-4a,5,6,7,8,8a-hexahydroquinazolin-2-yl)piperi din-4-yl)carbamate 92e

To a mixture of compound tert-butyl (1-(4-(4-cyano-3-fluorophenyl)-5,6,7,8-tetrahydropyrido[4,3-d]pyrimidin-2-yl)piperidin-4-yl)carbamate **92d** (41 mg, 0.09 mmol), bis(dibenzylideneacetone)palladium (2.73 mg, 4.75 µmol), 4,5-Bis(diphenylphosphino)-9,9-dimethylxanthene(5.5 mg, 9.50 µmol), cesium carbonate (61.89 mg, 0.27 mmol) and dried dioxane (2 mL) was added 2-bromobenzotrifluoride (30.6 mg, 0.18 mmol).The reaction mixture was stirred at 100°C for 17 h under nitrogen protection. The reaction solutionwas then cooled and concentrated under reduced pressure, added with 15 mL of water and 15 mL of ethyl acetate, mixed homogeneously, and allowed to separate into layers. The aqueous phase was extracted with ethyl acetate (10mLX 3). The organic phases were combined, washed with saturated sodium chloride solution, dried over anhydrous sodium sulfate, and filtered; the filtrate was concentrated, and purified by silica gel column chromatography (EA:PE; 1:8 to 1:4) to give the title compound **92e** (21mg) (yellow solid), yield: 36.9%.¹H NMR (400 MHz, Chloroform-*d*) δ 7.67 - 7.60 (m, 2H), 7.54 (t, J = 8.1 Hz, 1H), 7.43 - 7.34 (m, 3H), 7.31 - 7.21 (m, 1H), 4.70 (d, J = 13.3 Hz, 2H), 4.51 - 4.48 (m, 1H), 3.90 - 3.87 (m, 2H), 3.74 - 3.69 (m, 1H), 3.28 (t, J = 5.9 Hz, 2H), 3.09 - 2.97 (m, 4H), 2.03 (d, J = 11.3 Hz, 2H), 1.46 (s, 9H), 1.43 - 1.30 (m, 2H).

### Step 5

### 4-(2-(4-atninopiperidin-1-yl)-6-(2-(trifluoromethyl)phenyl)-5,6,7, 8-tetrahydropyrido [4,3-d]pyrimidin-4-yl)-2-fluorobenzonitrile 92

Compound tert-butyl (1-(4-(4-cyano-3-fluorophenyl)-6-(2-(trifluoromethyl)phenyl)-4a,5,6,7,8,8a-hexahydroquinazolin-2-yl)piperi din-4-yl)carbamate **92e** (21mg) was dissolved in 2 mL of dichloromethane, and 2 mL ofa solution of 4N hydrogen chloride in 1,4-dioxane was added.The reaction mixture was stirred at room temperature for 2 hours. The reaction solutionwas then concentrated under reduced pressure to give the title compound **92**hydrochloride (15 mg) (yellow solid), yield: 85.8%.¹H NMR (600 MHz, Methanol-*d*₄) δ 7.99 - 7.92 (m, 1H), 7.87 (t, J = 6.9 Hz, 1H), 7.63 (p, J = 9.7, 8.2 Hz, 4H), 7.34 (t, J = 7.3 Hz, 1H), 4.01 - 3.97 (m, 2H), 3.52 - 3.48 (m, 1H), 3.34 (d, J = 6.4 Hz, 2H), 3.19 (t, J = 12.7 Hz, 2H), 3.15 - 3.11 (m, 2H), 2.30 - 2.19 (m, 1H), 2.14 (d, J = 12.0 Hz, 2H), 2.05 - 1.99 (m, 1H), 1.72 - 1.67 (m, 2H).

### Example 93

### 4-(2-(4-aminopiperidin-1-yl)-6-(2-fluoro-6-(trifluoromethyl)phenyl)-5,6,7,8-tetrahydropyrido[4,3-d]pyr imidin-4-yl)-2-fluorobenzonitrile 93

The title compound **93**hydrochloride(8 mg) was prepared using the synthetic route of Example 92 by replacing the raw material 2-bromobenzotrifluoride of step 4 with 2-bromo-3-fluorobenzotrifluoride.MS m/z (ESI):515.20 [M + 1]⁺.

### Example 94

### 4-(2-(4-aminopiperidin-1-yl)-6-(1-methyl-1H-indazol-7-yl)-5,6,7,8-tetrahydropyrido[4,3-d]pyrimidin-4 -yl)-2-fluorobenzonitrile 94

The title compound **94**hydrochloride (10 mg) was prepared using the synthetic route of Example **92** by replacing the raw material 2-bromobenzotrifluorideofstep 4 with 7-bromo-1-methylindazole.MS m/z (ESI):482.52 [M + 1]⁺.

### Example 95

### 4-(3-(4-Aminopiperidin-1-yl)-7-(2-fluoro-6-(trifluoromethyl)phenyl)isoquinolin-1-yl)-2-fluorobenzonit rile 98

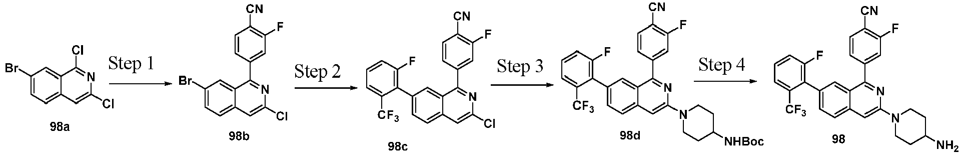

### Step 1

### 4-(7-Bromo-3-chloroisoquinolin-1-yl)-2-fluorobenzonitrile 98b

To a mixture of compound 7-bromo-1,3-dichloroisoquinoline **98a** (1.00 g, 3.61 mmol), bis(triphenylphosphine)palladium(II) chloride (127 mg, 180.54 µmol), potassium phosphate (1.53 g, 7.22 mmol) and acetonitrile/water (8:1) (18 mL) was added (4-cyano-3-fluorophenyl)boronic acid (596 mg, 3.61 mmol, Shanghai Haohong Biomedical Technology Co.).The reaction mixture was heated at 100 °C for 15 min with stirring under nitrogen protection. After the completion of the reaction was monitored by TLC, the reaction solution was then cooled and concentrated under reduced pressure, pulped with ethyl acetate, filtered and dried to give the title compound **98b** (1.10 g) (white solid), yield: 84.3%.¹H NMR (400 MHz, Chloroform-*d*) δ 8.11 (s, 1H), 7.84 (td, *J*= 5.9, 5.3, 2.6 Hz, 2H), 7.80 - 7.74 (m, 2H), 7.61 (s, 1H), 7.59 (s, 1H).

### Step 2

### 4-(3-Chloro-7-(2-fluoro-6-(trifluoromethyl)phenyl)isoquinolin-1-yl)-2-fluorobenzonitrile 98c

To a mixture of compound 4-(7-bromo-3-chloroisoquinolin-1-yl)-2-fluorobenzonitrile **98b** (500 mg, 1.38 mmol), [1,1'-bis(diphenylphosphino)ferrocene]palladium(II) dichloride dichloromethane complex (56 mg, 0.069 mol), sodium carbonate (293 mg, 2.77 mmol) and tetrahydrofuran/water (5:1) (35 mL) was added 2-fluoro-6-trifluoromethylphenylboronic acid (288 mg, 1.38 mmol, Shanghai Haohong Biomedical Technology Co.).The reaction mixture was stirred at 70 °C for 16 h under nitrogen protection. After the completion of the reaction was monitored by TLC, the reaction solution cooled and concentrated under reduced pressure, added with 35 mL of water and 35 mL of ethyl acetate, mixed homogeneously, and allowed to separate into layers. The aqueous phase was extracted with ethyl acetate (20mLX 3). The organic phases were combined, washed with saturated sodium chloride solution, dried over anhydrous sodium sulfate, and filtered. The filtrate was concentrated, and purified by silica gel column chromatography (EA:PE; 1:8 to:4) to give the title compound **98c** (420 mg) as pale yellow solid, yield: 68.3%.MS m/z (ESI):445.35 [M+1]⁺.

### Step 3

### Tert-butyl

### (1-(1-(4-cyano-3-fluorophenyl)-7-(2-fluoro-6-(trifluoromethyl)phenyl)isoquinolin-3-yl)piperidin-4-yl)carba mate 98d

To a mixture of compound 4-(3-chloro-7-(2-fluoro-6-(trifluoromethyl)phenyl)isoquinofin-1-yl)-2-fluorobenzonitrile **98c** (100 mg, 224.83 µmol), 4-tert-butoxycarbonylaminopiperidine (90 mg, 449.65 µmol), cesium carbonate (147 mg, 449.65 µmol), and dried 1,4-dioxane (4 mL) was added palladium acetate (5 mg, 22.48 µmol) and BINAP (28 mg, 44.97 µmol). The reaction mixture was stirred at 110°C for 12 h under nitrogen protection. After the completion of the reaction was monitored by TLC, the reaction solution cooled and concentrated under reduced pressure, added with 15 mL of water and 15 mL of ethyl acetate, mixed homogeneously, and allowed to separate into layers. The aqueous phase was extracted with ethyl acetate (10mLX 3). The organic phases were combined, washed with saturated sodium chloride solution, dried over anhydrous sodium sulfate, and filtered. The filtrate was concentrated, and purified by silica gel column chromatography (EA:PE; 1:10 to 1:5) to give the title compound **98d** (57 mg) as yellow solid, yield: 41.7%.MS m/z (ESI):609.13 [M + 1]⁺.

### Step 4

### 4-(3-(4-Aminopiperidin-1-yl)-7-(2-fluoro-6-(trifluoromethyl)phenyl)isoquinolin-1-yl)-2-fluorobenzonit rile 98

Compound tert-butyl (1-(1-(4-cyano-3-fluorophenyl)-7-(2-fluoro-6-(trifluoromethyl)phenyl)isoquinolin-3-yl)piperidin-4-yl)carba mate **98d** (57 mg) was dissolved in 2 mL of dichloromethane, and 2 mL of asolution of 4N hydrogen chloride in 1,4-dioxane was added.The reaction mixture was stirred at room temperature for 3 hours. The reaction solutionwas concentrated under reduced pressure and washed with ethyl acetate solution to give the title compound **98**hydrochloride (41 mg) (yellow solid), yield: 80.6%.MS m/z (ESI):509.21 [M+1]⁺.

### Example 96

### 4-(2-(4-atninopiperidin-1-yl)-8-methyl-7-oxo-6-(2-trifluoromethylphenyl)-7, 8-dihydropyrido [2,3-d]pyri midin-4-yl)-2-fluorobenzonitrile 99

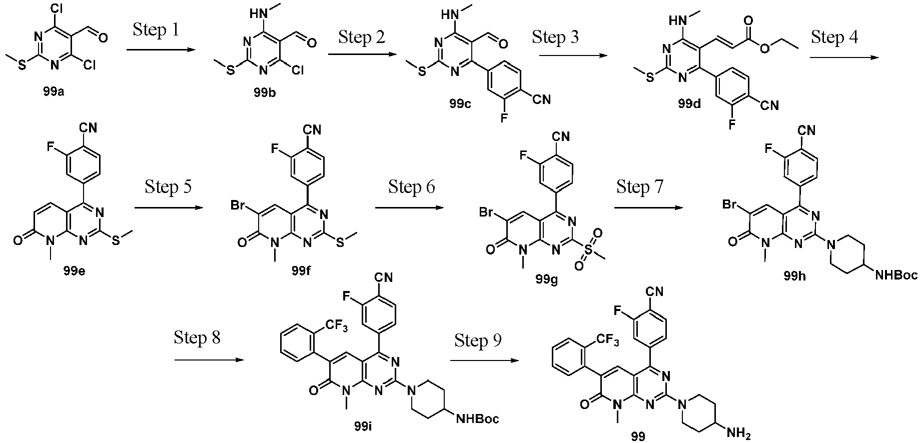

### Step 1

### 4-Chloro-6-methylamino-2-(methylthio)pyrimidin-5-carbaldehyde 99b

Compound 4,6-dichloro-2-methylthiopyrimidin-5-carboxaldehyde **99a** (5.00 g, 22.41 mmol, Shanghai Haohong Biomedical Technology Co., Ltd.) was dissolved in chloroform (40 mL), and methylamine (40% aqueous solution) (1.83 g, 23.54 mmol) and triethylamine (4.67 mL, 33.62 mmol) were added dropwise.The reaction was stirred for 1 hour at room temperature. After the completion of the reaction was monitored by TLC, the reaction solution was concentrated under reduced pressure, added with 60mL of water and 60 mL of ethyl acetate, mixed homogeneously and allowed to separate into layers. The aqueous phase was extracted with ethyl acetate (30mLX 3). The organic phases were combined, washed with saturated sodium chloride solution, dried over anhydrous sodium sulfate, and filtered; the filtrate was concentrated, and purified by silica gel column chromatography (EA:PE; 1:10 to 1:5) to give the title compound **96b** (4.50 g) (white solid), yield: 90.2%.¹H NMR (400 MHz, DMSO-*d*₆) δ 9.81 (s, 1H), 3.04 (d, J = 4.9 Hz, 3H), 2.53 (s, 3H).

### Step 2

### 2-Fluoro-4-(5-formyl-6-(methylamino)-2-(methylthio)pyrimidin-4-yl)benzonitrile 99c

To a mixture of compound 4-chloro-6-methylamino-2-methylthiopyrimidin-5-carbaldehyde **99b** (4.50 g, 20.67 mmol), tetrakis(triphenylphosphine)palladium (477 mg, 413.47 µmol), potassium carbonate (5.71 g, 41.35 mmol) and 1,4-dioxane/water (4:1) (40 mL) was added (4-cyano-3 -fluorophenyl)boronic acid (4.43 g, 26.88 mmol, Shanghai Haohong Biomedical Technology Co.). The reaction mixture was heated at 110°C for 16 h with stirring under nitrogen protection. After the completion of the reaction was monitored by TLC, the reaction solution was then cooled and concentrated under reduced pressure, pulped with ethyl acetate, filtered and dried to give the title compound **99c** (4.20 g) (white solid), yield: 67.2%. ¹H NMR (400 MHz, Chloroform-*d*) δ 9.73 (d, *J*= 0.6 Hz, 1H), 9.16 (s, 1H), 7.73 (dd, *J*= 7.9, 6.3 Hz, 1H), 7.44 (ddd, *J*= 16.7, 8.4, 1.5 Hz, 2H), 3.17 (d, *J*= 4.9 Hz, 3H), 2.59 (s, 3H).

### Step 3

### Ethyl (E)-3-(4-(4-cyano-3-fluorophenyl)-6-(methylamino)-2-(methylthio)pyrimidin-5-yl)acrylate 99d

Compound 2-fluoro-4-(5-formyl-6-(methylamino)-2-(methylthio)pyrimidin-4-yl)benzonitrile **99c** (4.00 g, 13.23 mmol) was dissolved in tetrahydrofuran (50 mL), and ethyl (triphenylphosphoranylidene)acetate (5.99 g, 17.20 mmol) was added.The reaction mixture was heated to reflux for 1.5 h. After the completion of the reaction was monitored by TLC, the reaction solution was cooled to room temperature, concentrated under reduced pressure and directly purified by column chromatography (EA:PE; 1:10 to 1:5) to give the title compound **99d** (4.10 g) (white solid), yield:83.2%.¹H NMR (400 MHz, DMSO-d₆) δ 8.05 - 7.96 (m, 1H), 7.64 (dd, *J*= 10.3, 1.5 Hz, 1H), 7.51 - 7.44 (m, 2H), 7.36 (d, *J*= 16.3 Hz, 1H), 5.99 (d, *J*= 16.3 Hz, 1H), 4.11 (q, *J*= 7.1 Hz, 2H), 2.91 (d, *J*= 4.4 Hz, 3H), 2.48 (s, 3H), 1.20 (t, *J*= 7.1 Hz, 3H).

### Step 4

### 2-Fluoro-4-(8-methyl-2-(methylthio)-7-oxo-7,8-dihydropyrido[2,3-d]pyrimidin-4-yl)benzonitrile99e

To a suspention of compound ethyl (E)-3-(4-(4-cyano-3-fluorophenyl)-6-(methylamino)-2-(methylthio)pyrimidin-5-yl) acrylate **99d** (4.00 g, 10.74 mmol) in methanol was added sodium methanoxide(29 mg, 537.03 µmol), and the reaction mixture was stirred and heated at 70 °C for 6 h. After the completion of the reaction was monitored by TLC,the reaction solution was cooled to room temperature, a solid was precipitated, and the reaction solution was filtered, and dried to give a crude product of the title compound **99e,** which was directly used in the next step without purification. MS m/z (ESI):327. 31 [M+1]⁺.

### Step 5

### 6-Bromo-8-methyl-2-methylthio-7-oxo-7,8-dihydropyridin-2,3-dihydroxypyrimidin-4-yl-2-fluorobenzo nitrile 99f

### Compound

2-fluoro-4-(8-methyl-2-(methylthio)-7-oxo-7,8-dihydropyrido[2,3-d]pyrimidin-4-yl)benzonitrile **99e** (2.00 g, 6.13 mmol) was dissolved in dichloromethane (30 mL) and then liquid bromine (1.96 g, 12.26 mmol) was added slowly dropwise. The reaction mixture was stirred at room temperature for 12 h. After the completion of the reaction was monitored by TLC, the reaction solution was poured into saturated sodium thiosulfate solution and extracted with ethyl acetate. The organic phase was washed with saturated sodium chloride solution, dried over anhydrous sodium sulfate, and filtered. The filtrate was concentrated, and purified by silica gel column chromatography (EA:PE; 1:10 to 1:5) to give the title compound **99f** (1.90 g) (white solid), yield: 76.5%.¹H NMR (400 MHz, Chloroform-*d*) δ 8.06 (s, 1H), 7.84 (dd, *J=* 7.8, 6.3 Hz, 1H), 7.58 - 7.49 (m, 2H), 3.88 (s, 3H), 2.66 (s, 3H).

### Step 6

### 4-Bromo-8-methyl-2-methylsulfonyl-7-oxo-7,8-dihydropyrido[2,3-d]pyrimidin-4-yl)-2-fluorobenzonitri le 99g

### Compound

6-bromo-8-methyl-2-methylthio-7-oxo-7,8-dihydropyridin-2,3-dihydroxypyrimidin-4-yl-2-fluorobenzonitril e **99f** (1.90 g, 4.96 mmol) was dissolved in dichloromethane (30 mL), and 3-chloroperoxybenzoic acid (2.43 g, 14.07 mmol) was added in batches at 0 °C. The reaction mixture was stirred at room temperature for 1 h. After the completion of the reaction was monitored by TLC, the reaction solution was poured into saturated sodium bicarbonate solution and extracted with ethyl acetate. The organic phase was washed with saturated sodium chloride solution, dried over anhydrous sodium sulfate, and filtered. The filtrate was concentrated to give a crude product of the title compound **99 g,** which was directly used in the next step without purification.

### Step 7

### Tert-butyl

### 1-(6-bromo-4-(4-cyano-3-fluorophenyl)-8-methyl-7-oxo-7, 8-dihydropyrido [2,3-d]pyrimidin-2-ylpiperidin-4-carbamate 99h

### Compound

4-bromo-8-methyl-2-methylsulfonyl-7-oxo-7,8-dihydropyrido[2,3-d]pyrimidin-4-yl)-2-fluorobenzonitrile **99 g** (2.05 g, 4.96 mmol) and 4-tert-butyloxycarbonylaminopiperidine (1.13 g, 5.63 mmol) were dissolved in acetonitrile (20 mL), and potassium carbonate (1.30 g, 9.38 mmol) was added.The reaction mixture was stirred at room temperature for 12 h.After the completion of the reaction was monitored by TLC, the reaction solution was concentrated under reduced pressure, added with 30mL of water and 30 mL of ethyl acetate, mixed homogeneously and allowed to separate into layers. The aqueous phase was extracted with ethyl acetate (30mLX 3). The organic phases were combined, washed with saturated sodium chloride solution, dried over anhydrous sodium sulfate, and filtered; the filtrate was concentrated, and purified by silica gel column chromatography (MeOH: DCM; 1:100 to 1:50) to give the title compound **99h** (2.40 g) (yellow solid), yield: 91.8%.¹H NMR (400 MHz, Chloroform-*d*) δ 7.89 (s, 1H), 7.81 (dd, *J*= 8.1, 6.3 Hz, 1H), 7.48 (ddd, *J*= 8.2, 4.8, 1.4 Hz, 2H), 4.84 - 4.76 (m, 2H), 4.51 - 4.43 (m, 1H), 3.76 (s, 3H), 3.22 - 3.11 (m, 2H), 2.14 - 2.05 (m, 2H), 1.46 (s, 9H), 1.44 - 1.36 (m, 2H).

### Step 8

### Tert-butyl

### 1-(4-(4-cyano-3-fluorophenyl)-8-methyl-7-oxo-6-(2-trifluoromethylphenyl)-7, 8-dihydropyrido [2,3-d]pyrimi din-2-yl)carbamate 99i

To a mixture of compound tert-butyl 1-(6-bromo-4-(4-cyano-3-fluorophenyl)-8-methyl-7-oxo-7,8-dihydropyrido[2,3-d]pyrimidin-2-ylpiperidin-4-carbamate **99h** (200 mg, 358.79 µmol), tetrakis(triphenylphosphine)palladium (20.73 mg, 17.94 µmol), potassium carbonate (149 mg. 1.08 mmol) and 1,4-dioxane/water (4:1) (5 mL) was added 2-trifluoromethylphenylboronic acid (102 mg, 538.19 µmol). The reaction mixture was stirred at 110°C for 2 h under nitrogen protection. After the completion of the reaction was monitored by TLC, the reaction solution was cooled and concentrated under reduced pressure, added with 15 mL of water and 15 mL of ethyl acetate, mixed homogeneously, and allowed to separate into layers. The aqueous phase was extracted with ethyl acetate (10mLX 3). The organic phases were combined, washed with saturated sodium chloride solution, dried over anhydrous sodium sulfate, and filtered. The filtrate was concentrated, and purified by silica gel column chromatography (EA:PE; 1:4 to 1:2) to give the title compound **99i** (181mg) (yellow solid), yield: 81.0%.¹H NMR (400 MHz, Chloroform-d) δ 7.72 (dd, *J*= 8.1, 6.3 Hz, 2H), 7.58 - 7.52 (m, 1H), 7.51 - 7.43 (m, 4H), 7.32 (d, *J*= 7.6 Hz, 1H), 4.88 - 4.80 (m, 2H), 4.55 - 4.48 (m, 1H), 3.75 (s, 3H), 3.24 - 3.13 (m, 2H), 2.14 - 2.06 (m, 2H), 1.46 (s, 9H), 1.44 - 1.35 (m, 2H).

### Step 9

### 4-(2-(4-atninopiperidin-1-yl)-8-methyl-7-oxo-6-(2-trifluoromethylphenyl)-7, 8-dihydropyrido [2,3-d]pyri midin-4-yl)-2-fluorobenzonitrile 99

Compound tert-butyl 1-(4-(4-cyano-3-fluorophenyl)-8-methyl-7-oxo-6-(2-trifluoromethylphenyl)-7, 8-dihydropyrido [2,3-d]pyrimi din-2-yl)carbamate **99i** (181 mg) was dissolved in 4 mL of dichloromethane, and 4 mL of a solution of 4N hydrogen chloride in 1,4-dioxane was added.The reaction mixture was stirred at room temperature for 3 hours. The reaction solutionwas concentrated under reduced pressure and washed with ethyl acetate solution to give the title compound **99**hydrochloride (152 mg) (yellow solid), yield: 93.8%.¹H NMR (400 MHz, Methanol-*d*₄) δ 7.90 (dd, *J*= 8.0, 6.5 Hz, 1H), 7.77 (d, *J*= 7.8 Hz, 1H), 7.67 - 7.62 (m, 2H), 7.61 - 7.53 (m, 3H), 7.39 (d, *J*= 7.6 Hz, 1H), 5.13 - 5.06 (m, 2H), 3.77 (s, 3H), 3.54 - 3.43 (m, 1H), 3.23 - 3.12 (m, 2H), 2.21 - 2.11 (m, 2H), 1.70 - 1.57 (m, 2H).

### Biological evaluation

### Test Example 1: In vitro enzyme inhibition activity assay - LSD1

The purpose of this experiment was to test the in vitro inhibitory activity of the compounds against LSD1. The human recombinant LSD1 protein used in the experiment was purchased from Active Motif, and the enzyme level activity testing assay of LSD1 was performed using the Lance Ultra LSD1 Histone H3 Lysine 4 Demethylase Assay Kit (PerkinElmer). Tris-buffer (50 mMTris-HCl, 50 mMNaCl, 0.01% Tween-20, 1 mM DTT, 10 µM FAD, 10% glycerol, pH 9.0) was first prepared, and after preparation, the LSD1 fractions required for test were placed in this buffer and incubated for one hour at room temperature;the fractions included 4 µL of substrate solution (2.5 µL Bio-H3K4me2, 1-24aa), 4 µL of enzyme solution (4 nM LSD1), and 2 µL of inhibitor in a 384-well microplate.After these operations, another 5 µL of assay mixture containing Eu-labeled H3K4me0 antibody and ULight-Streptavidin anti-biotin protein was added to the system, after which the fluorescence intensity of the components to be tested was detected by using Envision (PerkinElmer) in TR-FRET mode (excitation wavelength of 320 nm, emission wavelength of 620 nm).The test was repeated a total of three times to minimize errors. The final IC₅₀ data were calculated by the software GraphPad Prism 5, and finally the sigmoidal dose-response (variable slope) method was used to obtain the fitted curve.

The LSD1 inhibitory activities of representative compounds of the present application as well as the positive control compound CC90011 were provided in Table 2. All compounds were tested in the form of hydrochloride.

**Table 2**

| Compound No. | LSD1 IC₅₀ |
|---|---|
| CC90011 | A |
| 3 | B |
| 6 | B |
| 8 | B |
| 9 | B |
| 10 | B |
| 11 | A |
| 12 | A |
| 13 | B |
| 14 | A |
| 15 | B |
| 16 | A |
| 17 | B |
| 19 | A |
| 20 | A |
| 21 | A |
| 22 | A |
| 23 | A |
| 24 | B |
| 25 | B |
| 29 | B |
| 34 | A |
| 35 | B |
| 36 | B |
| 37 | B |
| 38 | B |
| 39 | B |
| 40 | A |
| 41 | B |
| 42 | A |
| 43 | B |
| 44 | B |
| 45 | A |
| 46 | A |
| 47 | B |
| 48 | A |
| 49 | B |
| 50 | B |
| 51 | A |
| 52 | B |
| 53 | A |
| 54 | A |
| 55 | A |
| 56 | B |
| 57 | A |
| 59 | B |
| 60 | B |
| 61 | B |
| 63 | A |
| 65 | B |
| 66 | A |
| 67 | A |
| 68 | A |
| 69 | A |
| 70 | A |
| 71 | A |
| 72 | A |
| 73 | A |
| 74 | A |
| 75 | B |
| 76 | A |
| 77 | A |
| 78 | A |
| 79 | B |
| 80 | A |
| 81 | B |
| 82 | A |
| 83 | B |
| 84 | B |
| 85 | B |
| 86 | A |
| 87 | A |
| 88 | A |
| 89 | A |
| 90 | A |
| 91 | A |
| 92 | B |
| 93 | B |
| 94 | B |
| 98 | A |
| 99 | A |

| | |
|---|---|
| NOTE: 1. The IC₅₀ data determined byBiochemical assay were designated within the following ranges: A: ≤30 nM B: >30 nM to ≤300 nM C: >300 nM to ≤10000 nM D: >10000 nM. | |

2. Chemical structure ofCC90011:

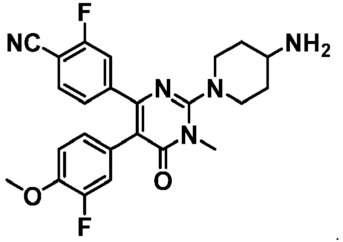

### Test Example 2: In vivo efficacy evaluation in mouse transplanted tumor model

Experimental purpose: To test the in vivo efficacy of compounds in mouse transplanted tumor model
Experimental materials: female NU/NU nude mice, MV4-11 cell line
Experimental methods: female NU/NU nude mice, weighing 20±2 g, wereinoculated with MV4-11 cell line inthe right axillary subcutaneous with a cell inoculation amount of5 × 10° /mouse. When the tumor formed, the diameter of the transplanted tumor was measured with vernier calipers, and the tumor was grew up to 100-300 mm³; the animals were divided into model control group and administration group according to their weights and tumor volumes, with 6 animals per group. The administration group was administered with a suspension ground with 0.5% CMC -Na, while the model control group was given an equal amount of blank solvent. After grouping, the drug was administered orally every day at a dose of 20 mg/kg for 21 days. During the experiment, the diameter of the transplanted tumorwasmeasured and the weight of the mice was weighed twice a week, and the tumor tissues were taken from the animal after euthanasia on the 21st day. Observations: Tumor volume TV= 1/2×a×b² (Note: a and b represented length and width, respectively), relative tumor volume RTV = TVₜ /TV₀ (Note: wherein TV₀ was the tumor volume measured at the time of animal feed cage points (i.e., d₀), and TVₜ was the tumor volume at each measurement), relative tumor proliferation rate T/C (%) = (T_{RTV}/C_{RTV}) × 100 % (Note: T_{RTV}: R_{TV} of the treatment group; C_{RTV}: R_{TV} of the negative control group), and tumor weight inhibition rate % = (W_{C}-W_{T})/W_{c} × 100 % (Note: W_{C}: tumor weight of the control group, W_{T}: tumor weight of the treatment group).
Conclusion: representative test compounds 19 and 89 exhibited significant in vivo antitumor activity (T/C less than 50% at a dose of 20 mg/kg administered by gavage).

## Claims

1. An aromatic heterocyclic compound of Formula I, a pharmaceutically acceptable salt thereof, a stereoisomer, a racemate, a prodrug, a co-crystalline complex, a hydrate, or a solvate thereof; 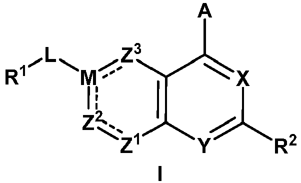
wherein, 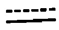 indicates a single bond or a double bond;
A is substituted 5-14-membered heteroaryl or substituted 6-14-membered aryl; wherein the substituted 5-14-membered heteroaryl and substituted 6-14-membered aryl are independently substituted by 1, 2 or 3 R^{a}; the heteroatoms in the 5-14-membered heteroaryl are selected from N, O and S and the number of heteroatoms is 1, 2, 3 or 4;
R^{a} is independently CN, F, Cl, Br, I or C₁-₃ alkyl, and at least one is CN;
R¹ is hydrogen, halogen, substituted or unsubstituted (amino)-(C₁-C₄alkylidene)-, substituted or unsubstituted C₃₋₁₂cycloalkyl, substituted or unsubstituted 3-10-memberedheterocycloalkyl, substituted or unsubstituted C₆-C₁₄ aryl, or substituted or unsubstituted 5-14-membered heteroaryl, wherein the substituted (amino)-(C₁-C₄alkylidene)-, substituted C₃₋₁₂cycloalkyl, substituted 3-10-memberedheterocycloalkyl, substituted C₆-C₁₄ aryl and substituted 5-14-membered heteroaryl are independently substituted by 1, 2 or 3 R^{1a}; the heteroatoms in the 5-14-membered heteroaryl are selected from N, O and S, and the number of heteroatom is 1, 2, 3 or 4; the heteroatoms in the 3-10-membered heterocycloalkyl are selected from N, O and S, and the number of heteroatom is 1, 2, 3 or 4;
R² is halogen, substituted or unsubstituted C₁-₄ alkyl, substituted or unsubstituted saturated or unsaturated C₃₋₁₀cyclic hydrocarbyl, substituted or unsubstituted C₆-C₁₄ aryl, substituted or unsubstituted 3-10-membered heterocyclyl, substituted or unsubstituted 5-14-membered heteroaryl, substituted or unsubstituted 3-10-membered heterocyclyl-N(R³)-, substituted or unsubstituted 3-10-membered heterocyclyl-(C₁-C₄alkylene)-N(R³)-, substituted or unsubstituted N(R³)₂-(C₁-C₄alkylidene)-N(R³)-, substituted or unsubstituted 3-10-membered heterocyclyl-O-, substituted or unsubstituted 3-10-membered heterocyclyl-(C₁-C₄alkylidene)-O-, substituted or unsubstituted N(R³)₂ -(C₁-C₄alkyfidene)-O-,substituted or unsubstituted3-10-membered heterocyclyl-(C₁-C₄alkylene)-; wherein the substituted C₁₋₄ alkyl, substituted saturated or unsaturated C₃₋₁₀cyclic hydrocarbyl, substituted C₆-C₁₄ aryl, substituted 3-10-membered heterocyclyl, substituted 5-14-membered heteroaryl, substituted 3-10-membered heterocyclyl-N(R³)-, substituted 3-10-membered heterocyclyl-(C₁-C₄alkylidene)-N(R³)-, substituted N(R³)₂-(C₁-C₄alkylene)-N(R³)-, substituted 3- 10-membered heterocyclyl-O-, substituted 3-10-membered heterocyclyl-(C₁-C₄alkylidene)-O-, and substituted N(R³)₂-(C₁-C₄alkylidene)-O- are independently substituted by 1, 2, or 3 R^{1c}; the heteroatoms in the 5-14-membered heteroaryl group are selected from N, O, and S, and the number of heteroatom is 1, 2, 3, or 4; and the 3-10-membered heterocyclyl is saturated or unsaturated 3-10-membered heterocyclyl and the heteroatoms of which are selected from N, O and S, and the number of heteroatom is 1, 2, 3 or 4;
each R^{1a} and R^{1c} areindependently F, Cl, Br, I, OH, N(R³)₂, CN, COOH, CON(R³)₂ , SO₂N(R³)₂ , C₁₋₃ alkyl, C₃₋₁₂cycloalkyl, C₁₋₃ alkyl-O-, or C₃₋₁₂cycloalkyl-O-, wherein the C₁₋₃ alkyl, C₃₋₁₂cycloalkyl, C₁₋₃ alkyl-O-, and C₃₋₁₂cycloalkyl-O- are optionally substituted by 1, 2, or 3 R^{1b};
eachR^{1b} is independently F, Cl, Br, I, OH or NH₂;
each R³ is independently hydrogen, substituted or unsubstituted C₁₋₄ alkyl, substituted or unsubstituted C₃₋₁₂cycloalkyl; wherein the substituted C₁₋₄ alkyl and substituted C₃₋₁₂cycloalkyl are independently substituted by 1, 2 or 3 R^{1b};
L is -NH-, -N(C₁-C₃alkyl)-, -O-, or absent;
X and Y are each independently N or C(R⁴);
M is C, CH or N;
Z', Z² and Z³ are each independently a linking bond, N, N(R⁴), C(H)(R⁴), C(R⁴), -(C=O)-, or -(C=S)-; and at most one of Z¹ , Z² and Z³ is a linking bond;
R⁴ is hydrogen, halogen, substituted or unsubstituted C₁-₄ alkyl, substituted or unsubstituted C₃₋₁₂cycloalkyl; wherein the substituted C₁₋₄ alkyl and substituted C₃₋₁₂cycloalkyl are independently substituted by 1, 2 or 3 R^{1b}.

2. The aromatic heterocyclic compound of Formula I according to claim 1, a pharmaceutically acceptable salt thereof, a stereoisomer, a racemate, a prodrug, a co-crystalline complex, a hydrate, or a solvate thereof, wherein thearomatic heterocyclic compound of Formula I, a pharmaceutically acceptable salt thereof, a stereoisomer, a racemate, a prodrug, a co-crystalline complex, a hydrate, or a solvate thereof satisfy one or more of the following conditions:
(1) Z', Z² and Z³ are each independently a linking bond, N, N(R⁴), C(H)(R⁴), C(R⁴), or -(C=O)-; and at most one of Z¹ , Z² and Z³ is a linking bond;
(2) R⁴ is hydrogen, halogen, or C₁₋₄ alkyl.

3. The aromatic heterocyclic compound of Formula I according to claim 1 or 2, a pharmaceutically acceptable salt thereof, a stereoisomer, a racemate, a prodrug, a co-crystalline complex, a hydrate, or a solvate thereof, wherein
the aromatic heterocyclic compound of formula I is as follows: 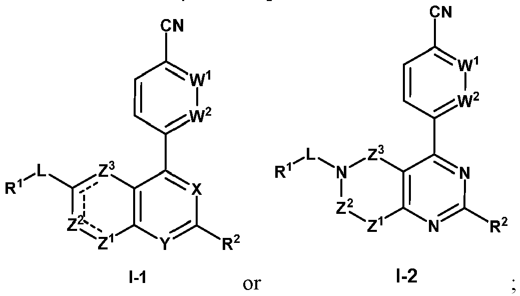 wherein W¹ and W² are each independently N, C(H), or C(R^{a}); 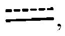 R¹, R², L, X, Y, Z¹ , Z² , Z³, and R^{a} are as defined in claim 1.

4. The aromatic heterocyclic compound of Formula I according to claim 1 or 2, a pharmaceutically acceptable salt thereof, a stereoisomer, a racemate, a prodrug, a co-crystalline complex, a hydrate, or a solvate thereof, wherein the aromatic heterocyclic compound of Formula I, a pharmaceutically acceptable salt thereof, a stereoisomer, a racemate, a prodrug, a co-crystalline complex, a hydrate, or a solvate thereof satisfy one or more of the following conditions:
(1) A is substituted 6-14-membered aryl, R¹ is substituted or unsubstituted C₆-C₁₄ aryl, and R² is substituted or unsubstituted C₆-C₁₄ aryl, wherein the C₆-C₁₄ aryl is independently phenyl or naphthyl;
(2) A is substituted 5-14-membered heteroaryl, R¹ is substituted or unsubstituted 5-14-membered heteroaryl, and R² is substituted or unsubstituted 5-14-membered heteroaryl, wherein the 5-14-membered heteroaryl is 5-10-membered heteroarylwiththe heteroatom selected from N, O, and S, and the number of heteroatom being 1 or 2; for example, 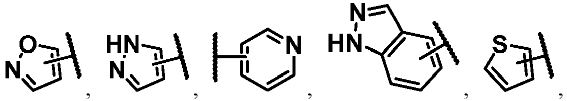 and also e.g. 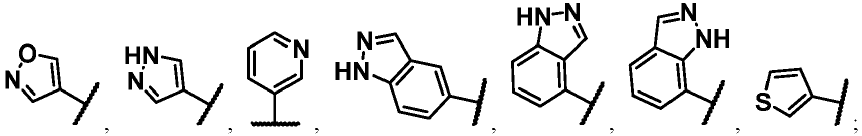
(3) R¹, R² and R⁴ are halogen, wherein the halogen is independently F, Cl, Br, I, for example Br;
(4) R¹ is substituted or unsubstituted (amino)-(C₁-C₄alkylidene)-, and R² is substituted or unsubstituted 3-10-membered heterocyclyl-(C₁-C₄alkylidene)-N(R³)-, substituted or unsubstituted N(R³)₂-(C₁-C₄alkylidene)-N(R³)-, substituted or unsubstituted 3-10-membered heterocyclyl-O-, substituted or unsubstituted 3-10-membered heterocyclyl-(C₁-C₄alkylidene)-O-, or substituted or unsubstituted N(R³)₂-(C₁-C₄alkylidene)-O-, wherein the (C₁-C₄alkylidene) is -CH₂-, -CH₂CH₂-, -CH(CH₃)-, -CH(CH₃)CH₂- or -C(CH₃)₂-;
(5) R¹ is substituted or unsubstituted C₃₋₁₂cycloalkyl, R^{1a} and R^{1c} are independently C₃₋₁₂cycloalkyl or C₃₋₁₂cycloalkyl-O-, R³ is substituted or unsubstituted C₃₋₁₂cycloalkyl, and R⁴ is substituted or unsubstituted C₃₋₁₂cycloalkyl, wherein the C₃₋₁₂cycloalkyl is independently C₃₋₇ monocyclic cycloalkyl, C₄₋₁₂ bridged or fused cycloalkyl, or C₇₋₁₂spirocycloalkyl; the C₃-₇ monocyclic cycloalkyl may becyclopropyl, cyclobutyl, cyclopentyl or cyclohexyl;
(6) R¹ is substituted or unsubstituted 3-10-membered heterocycloalkyl, wherein the 3-10-membered heterocycloalkyl is independently C₃₋₇ monocyclic heterocycloalkyl, C₄₋₁₂ bridged or fused heterocycloalkyl, or C₇₋₁₂spiroheterocycloalkyl; the C₃₋₇ monocyclic heterocycloalkyl may be 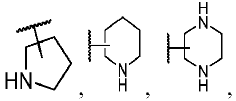 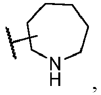 e.g. 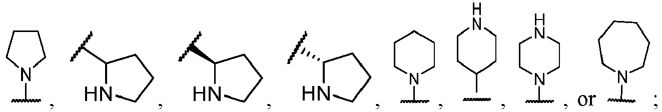 the C₄₋₁₂ bridged or fused heterocycloalkyl may be 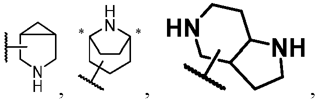 e.g., 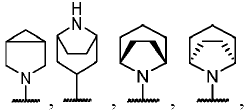 or 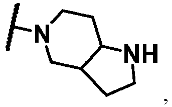 the C₇₋₁₂spiroheterocycloalkyl may be 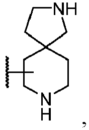 e.g. 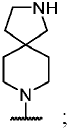
(7) R² is substituted or unsubstituted C₁₋₄ alkyl, R³ is substituted or unsubstituted C₁₋₄ alkyl, and R⁴ is substituted or unsubstituted C₁₋₄ alkyl, wherein the C₁₋₄ alkylis independently methyl, ethyl, n-propyl, isopropyl, n-butyl, sec-butyl, isobutyl, or tert-butyl;
(8) R² is substituted or unsubstituted saturated or unsaturated C₃₋₁₂ cyclic hydrocarbyl, wherein the saturated C₃₋₁₂cyclic hydrocarbylis C₃₋₁₂cycloalkyl, such as cyclopropyl, cyclobutyl, cyclopentyl or cyclohexyl;
(9) R² is substituted or unsubstituted 3-10-membered heterocyclyl, substituted or unsubstituted 3-10-membered heterocyclyl-N(R³)-, substituted or unsubstituted 3-10-membered heterocyclyl-(C₁-C₄alkylidene)-N(R³)-, substituted or unsubstituted 3-10-membered heterocyclyl-O-, or substituted or unsubstituted 3-10-membered heterocyclyl-(C₁-C₄alkylidene)-O-, when the 3-10-membered heterocyclyl is independently 3-10-membered heterocycloalkyl, the 3-10-membered heterocycloalkyl is independently C₃₋₇ monocyclic heterocycloalkyl, C₄₋₁₂ bridged or fused heterocycloalkyl, C₇₋₁₂spiroheterocycloalkyl; the C₃₋₇ monocyclic heterocycloalkyl may be 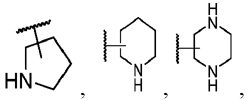 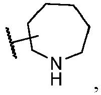 e.g. 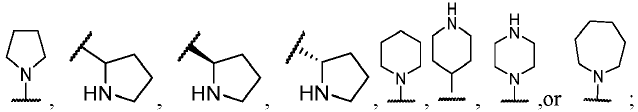 the C₄₋₁₂ bridged or fused heterocycloalkyl may be 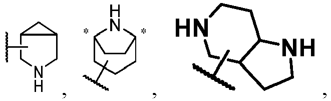 e.g., 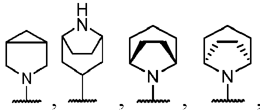 or 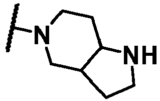 ), the C₇₋₁₂spiroheterocycloalkyl may be 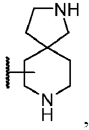 e.g. 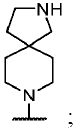
(10) R^{a} is independently C₁₋₃ alkyl, R^{1a} and R^{1c} are independently C₁₋₃ alkyl or C₁₋₃ alkyl-O-, and L is -N(C₁-C₃ alkyl)-, wherein the C₁₋₃ alkyl is independently methyl, ethyl, n-propyl or isopropyl.

5. The aromatic heterocyclic compound of Formula I according to claim 1 or 2, a pharmaceutically acceptable salt thereof, a stereoisomer, a racemate, a prodrug, a co-crystalline complex, a hydrate, or a solvate thereof, wherein the aromatic heterocyclic compound of Formula I, a pharmaceutically acceptable salt thereof, a stereoisomer, a racemate, a prodrug, a co-crystalline complex, a hydrate, or a solvate thereof satisfy one or more of the following conditions:
(1) A is 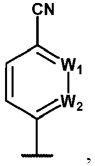 and W¹ and W² are each independently N, C(H), or C(R^{a}); wherein R^{a} is F, Cl, Br, I, or C₁₋₃ alkyl;
(2) R¹ is halogen, substituted or unsubstituted C₆-C₁₄ aryl, or substituted or unsubstituted 5-14-membered heteroaryl;
(3) R² is substituted or unsubstituted 3-10-membered N-containing heterocyclyl, substituted or unsubstituted 3-10-membered heterocyclyl-(C₁-C₄alkylidene)-N(R³)-, or substituted or unsubstituted 3-10-membered heterocyclyl-(C₁-C₄alkylidene)-O-;
(4) eachR^{1a} and R^{1c} areindependently F, Cl, OH, N(R³)₂, CON(R³)₂ , SO₂N(R³)₂, C₁₋₃alkyl, or C₁₋₃alkyl-O-, wherein the C₁₋₃ alkyl and C₁₋₃ alkyl-O- are optionally substituted by 1, 2, or 3 R^{1b};
(5) eachR^{1b} is independently F or NH₂;
(6) R³ is hydrogen or substituted or unsubstituted C₁₋₄ alkyl;
(7) L is -NH-, -O- or absent;
(8) X and Y are each independently N;
(9) Z', Z² and Z³ are each independently N, C(H)(R⁴) or C(R⁴);
(10) R⁴ is hydrogen or halogen.

6. The aromatic heterocyclic compound of Formula I according to claim 1 or 2, a pharmaceutically acceptable salt thereof, a stereoisomer, a racemate, a prodrug, a co-crystalline complex, a hydrate, or a solvate thereof, wherein the aromatic heterocyclic compound of Formula I, a pharmaceutically acceptable salt thereof, a stereoisomer, a racemate, a prodrug, a co-crystalline complex, a hydrate, or a solvate thereof satisfy one or more of the following conditions:
(1) R¹ is 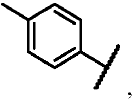 Br, 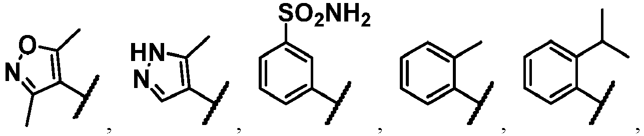 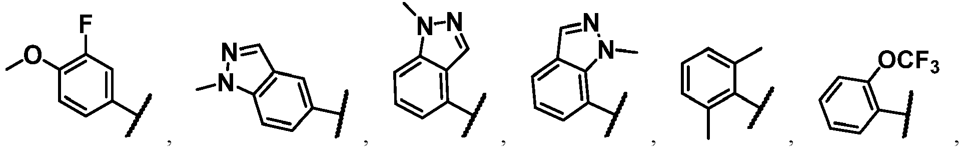 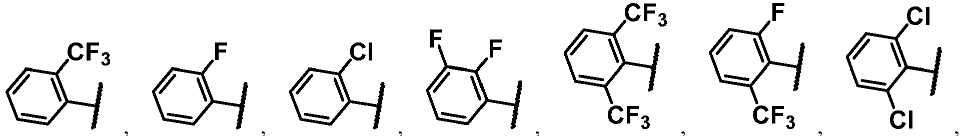 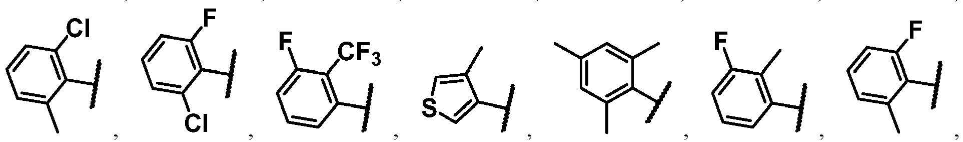 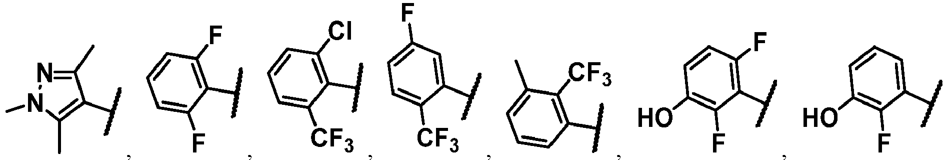 or 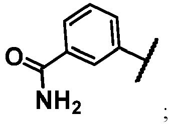
(2) A is 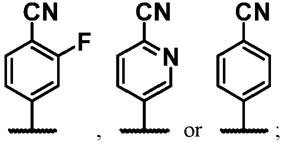
(3) 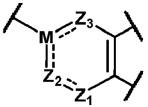 is 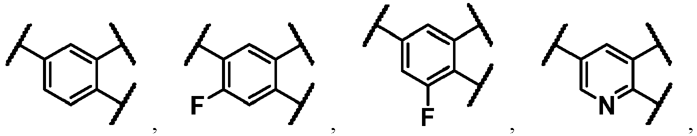 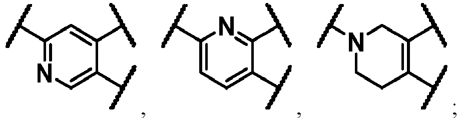
(4) R²is

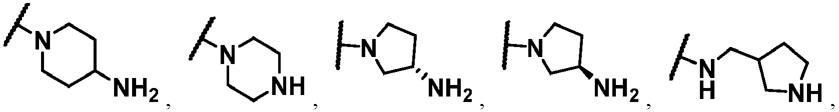

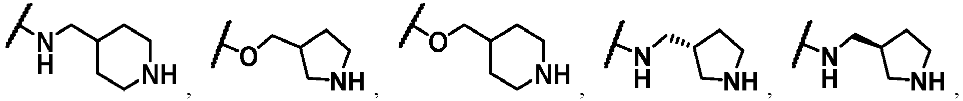

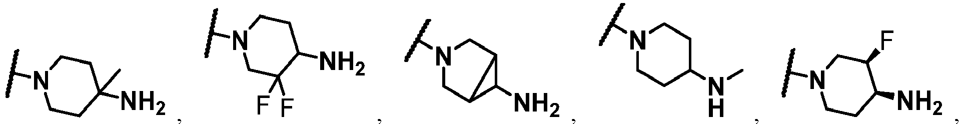

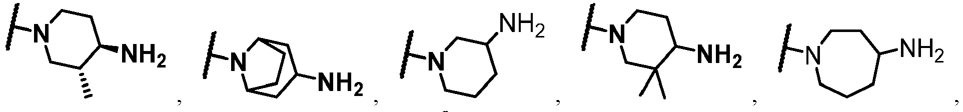

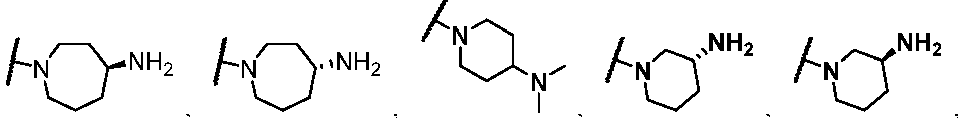

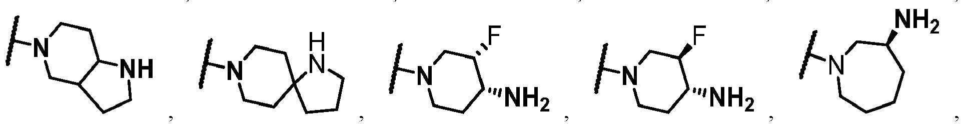

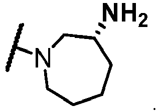

7. The aromatic heterocyclic compound of Formula I according to claim 1 or 2, a pharmaceutically acceptable salt thereof, a stereoisomer, a racemate, a prodrug, a co-crystalline complex, a hydrate, or a solvate thereof, wherein the aromatic heterocyclic compound of Formula I, a pharmaceutically acceptable salt thereof, a stereoisomer, a racemate, a prodrug, a co-crystalline complex, a hydrate, or a solvate thereof satisfy one or more of the following conditions:
(1) R'is 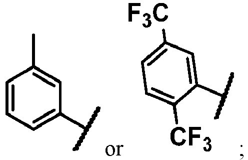
(2) 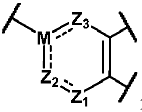 is 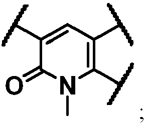
(3) R² is 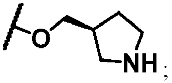
(4) R⁴ is H, F or methyl.

8. The aromatic heterocyclic compound of Formula I according to claim 1 or 2, a pharmaceutically acceptable salt thereof, a stereoisomer, a racemate, a prodrug, a co-crystalline complex, a hydrate, or a solvate thereof, wherein the aromatic heterocyclic compound of Formula I, or a pharmaceutically acceptable salt thereof is a compound of the following structure or hydrochloride thereof:
| | | | |
|---|---|---|---|
| 1 | 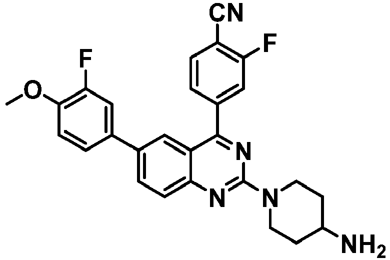 | 2 | 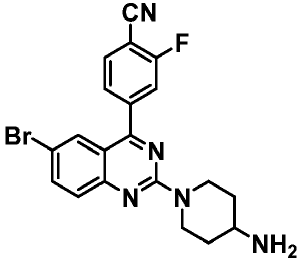 |
| 3 | 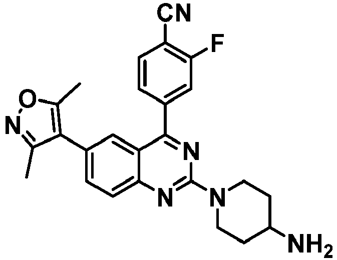 | 4 | 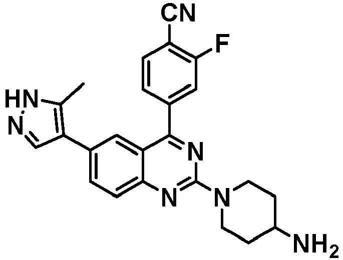 |
| 5 | 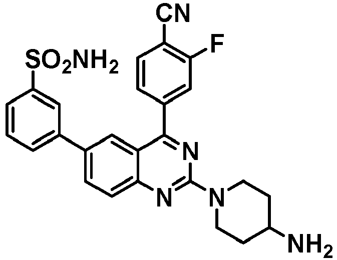 | 6 | 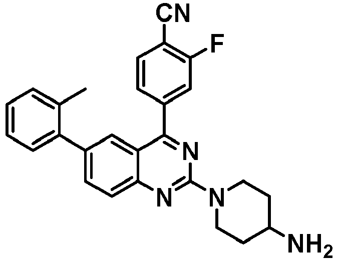 |
| 7 | 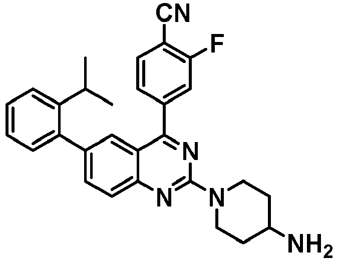 | 8 | 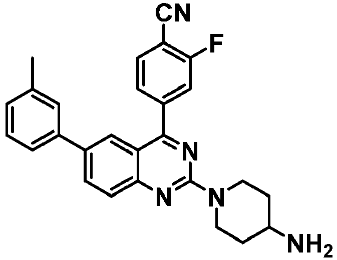 |
| 9 | 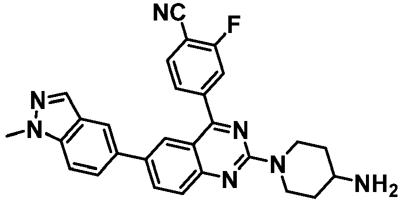 | 10 | 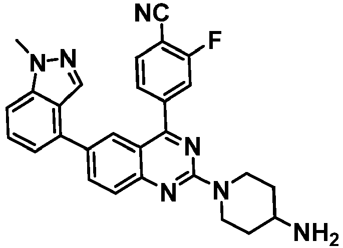 |
| 11 | 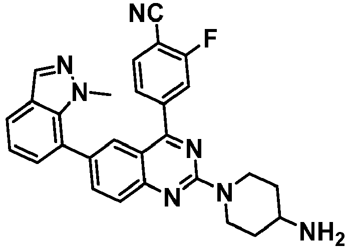 | 12 | 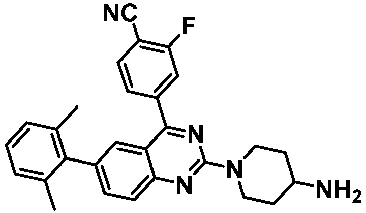 |
| 13 | 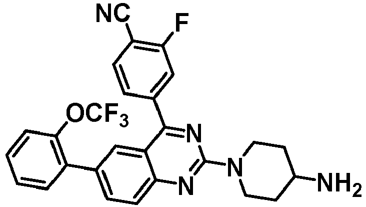 | 14 | 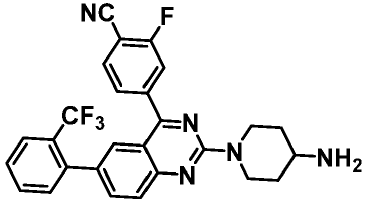 |
| 15 | 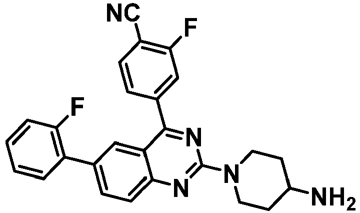 | 16 | 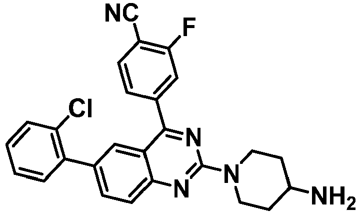 |
| 17 | 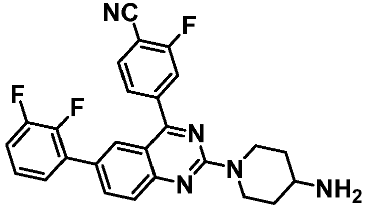 | 18 | 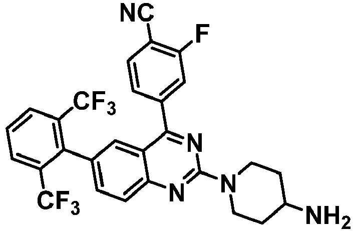 |
| 19 | 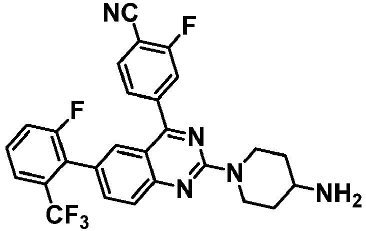 | 20 | 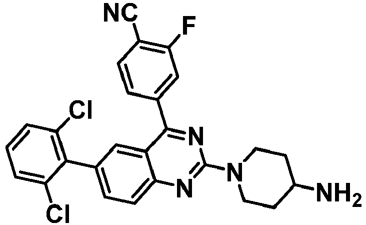 |
| 21 | 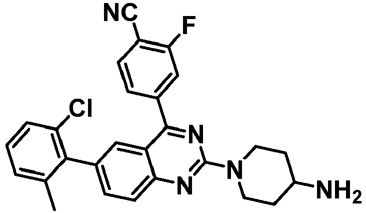 | 22 | 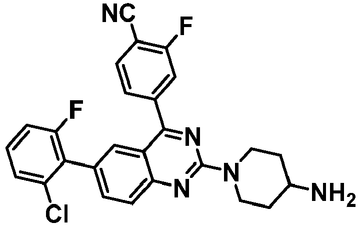 |
| 23 | 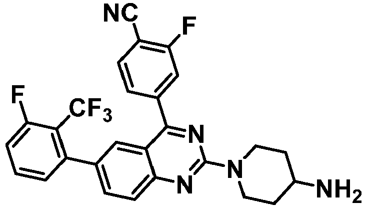 | 24 | 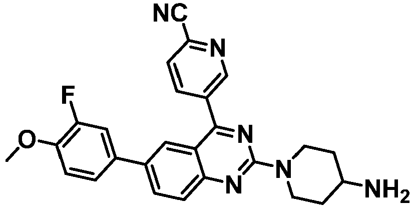 |
| 25 | 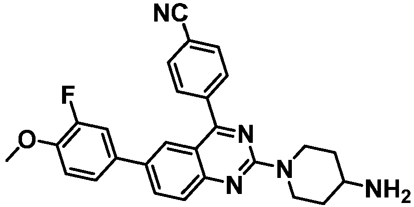 | 26 | 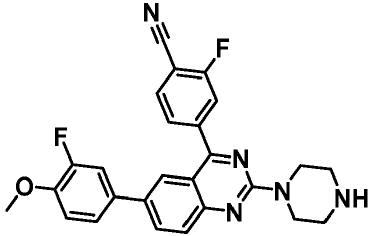 |
| 27 | 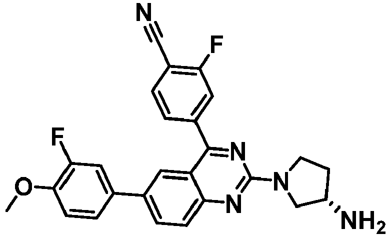 | 28 | 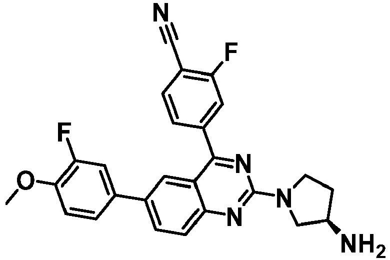 |
| 29 | 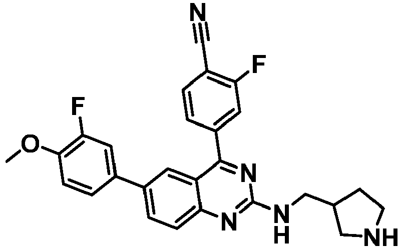 | 30 | 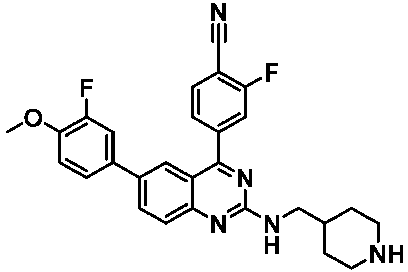 |
| 31 | 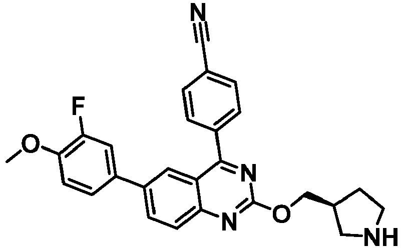 | 32 | 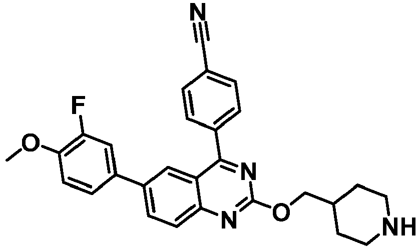 |
| 33 | 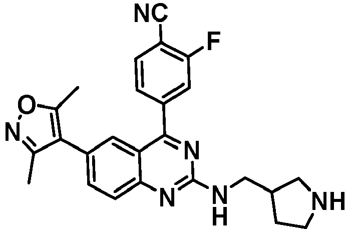 | 34 | 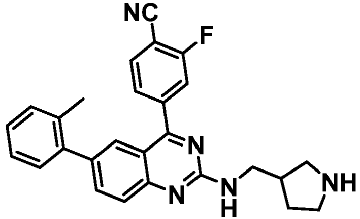 |
| 35 | 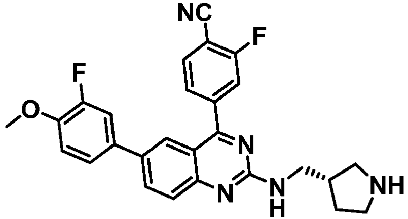 | 36 | 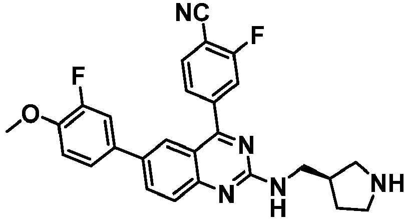 |
| 37 | 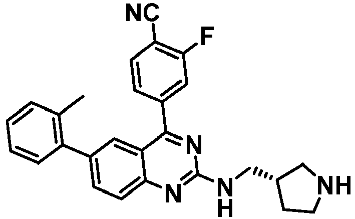 | 38 | 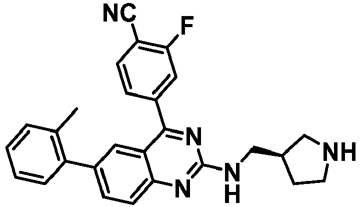 |
| 39 | 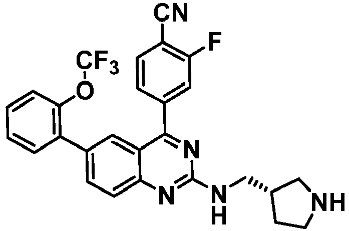 | 40 | 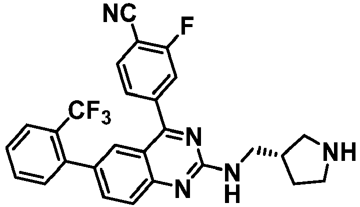 |
| 41 | 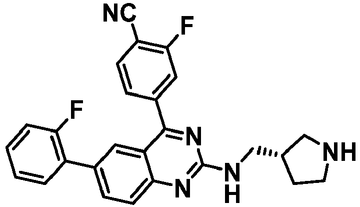 | 42 | 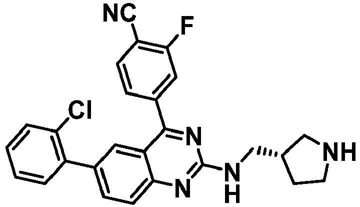 |
| 43 | 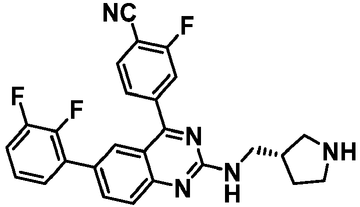 | 44 | 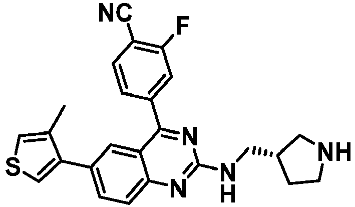 |
| 45 | 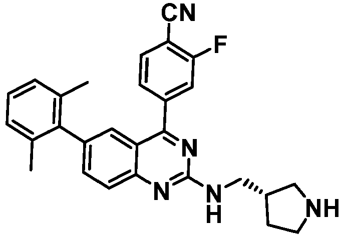 | 46 | 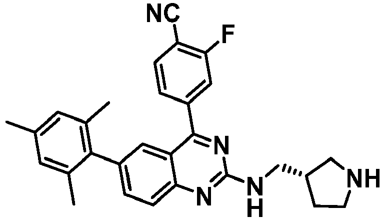 |
| 47 | 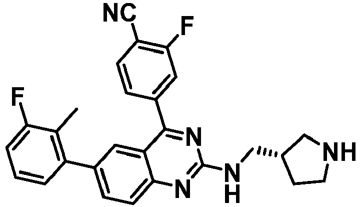 | 48 | 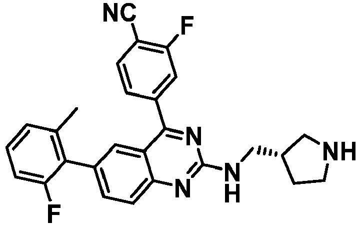 |
| 49 | 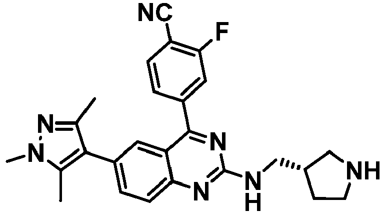 | 50 | 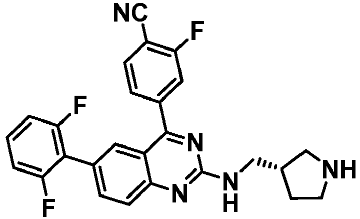 |
| 51 | 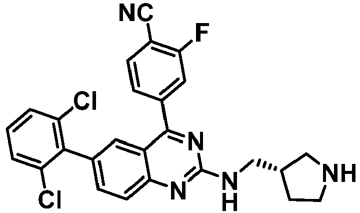 | 52 | 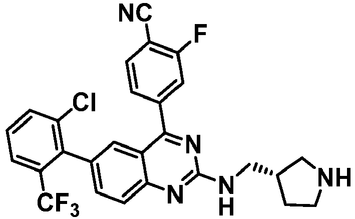 |
| 53 | 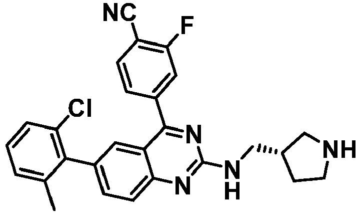 | 54 | 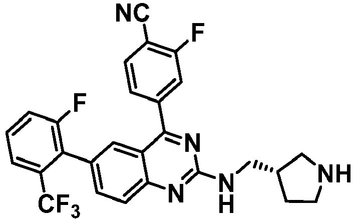 |
| 55 | 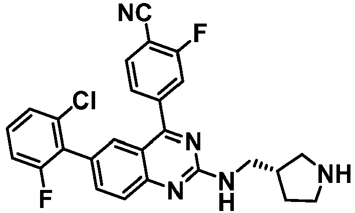 | 56 | 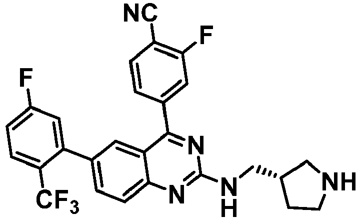 |
| 57 | 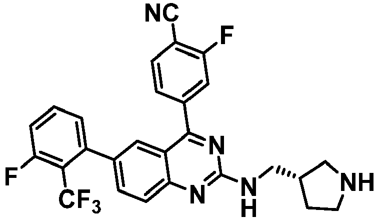 | 58 | 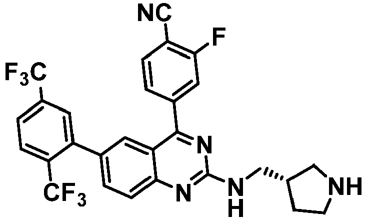 |
| 59 | 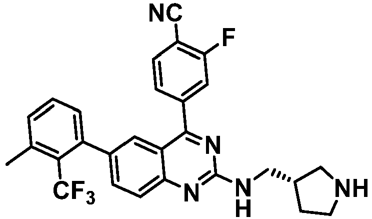 | 60 | 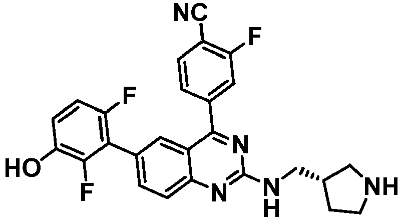 |
| 61 | 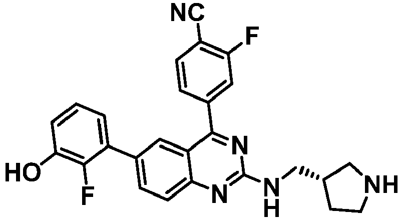 | 62 | 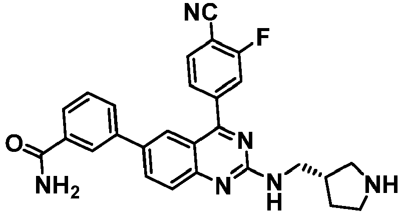 |
| 63 | 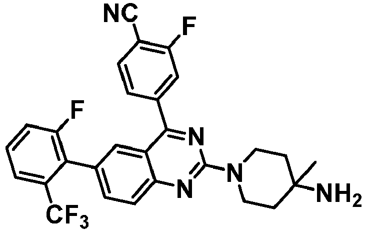 | 64 | 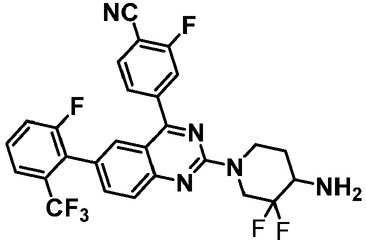 |
| 65 | 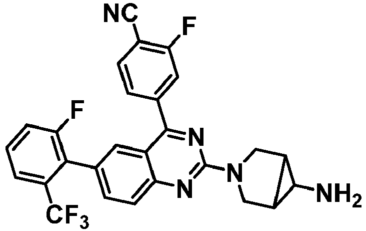 | 66 | 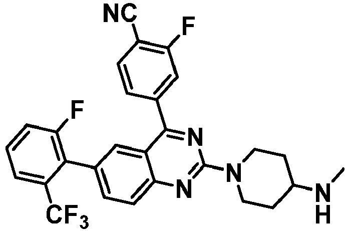 |
| 67 | 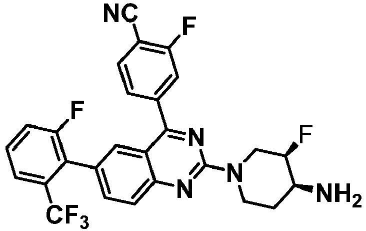 | 68 | 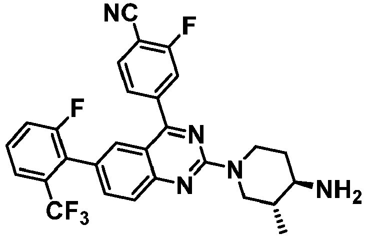 |
| 69 | 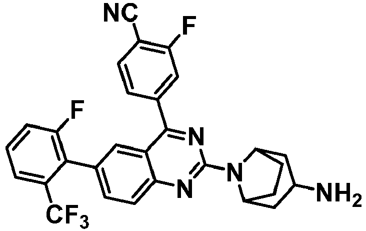 | 70 | 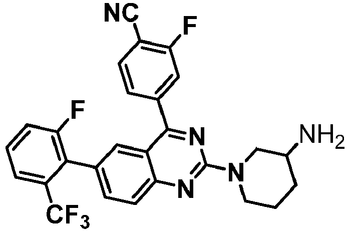 |
| 71 | 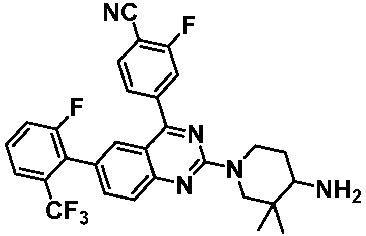 | 72 | 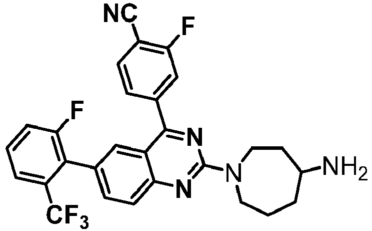 |
| 73 | 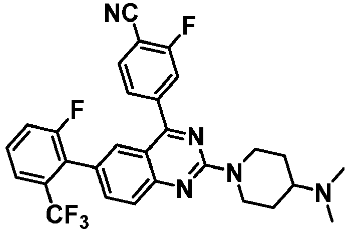 | 74 | 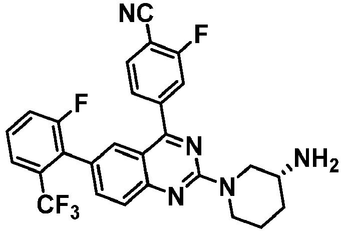 |
| 75 | 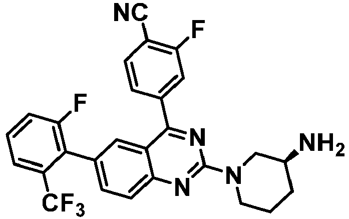 | 76 | 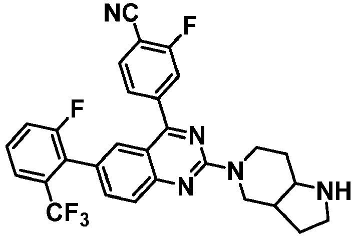 |
| 77 | 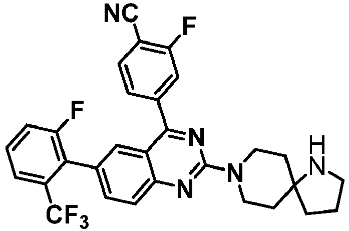 | 78 | 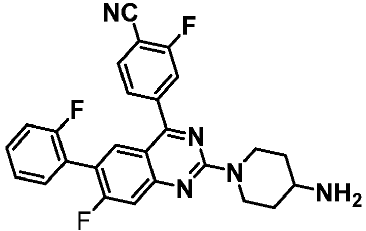 |
| 79 | 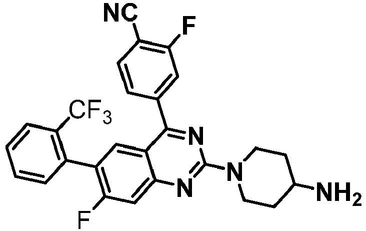 | 80 | 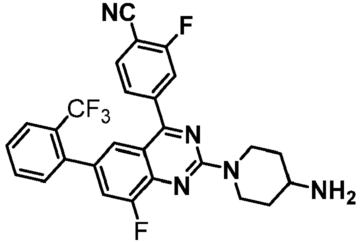 |
| 81 | 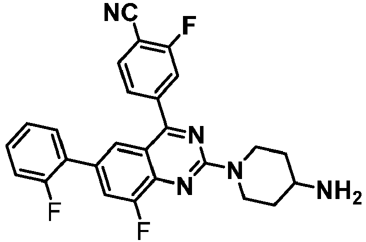 | 82 | 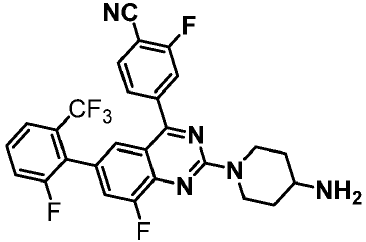 |
| 83 | 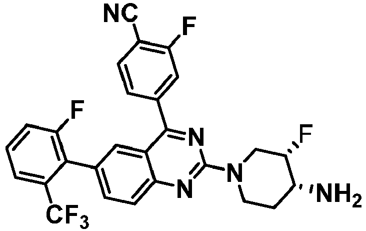 | 84 | 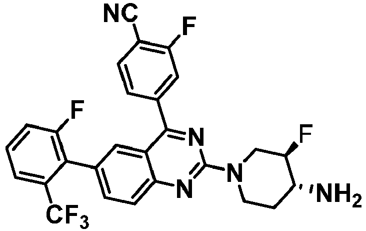 |
| 85 | 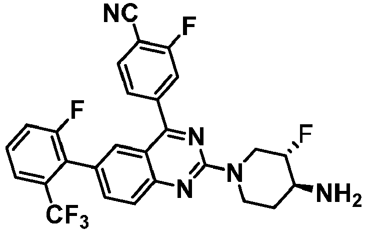 | 86 | 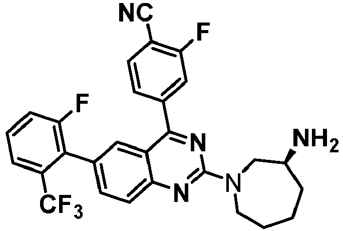 |
| 87 | 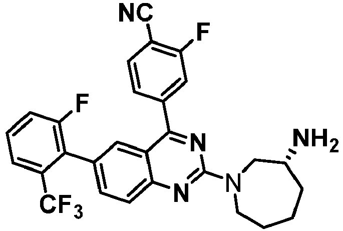 | 88 | 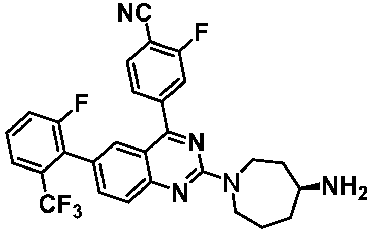 |
| 89 | 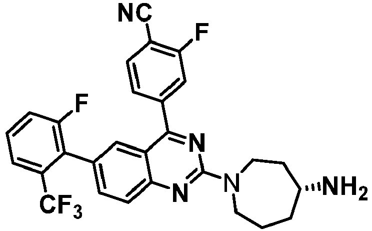 | 90 | 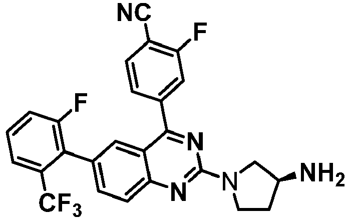 |
| 91 | 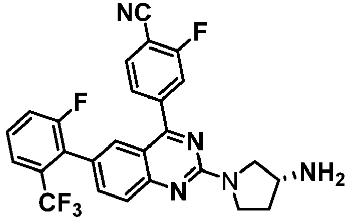 | 92 | 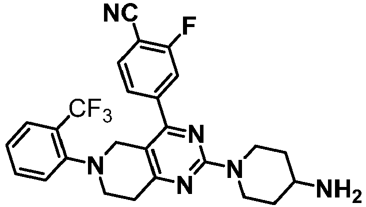 |
| 93 | 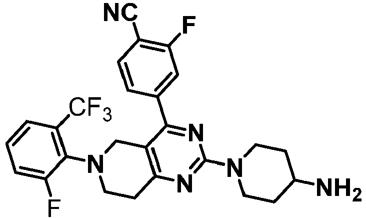 | 94 | 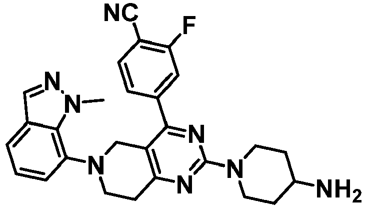 |
| 95 | 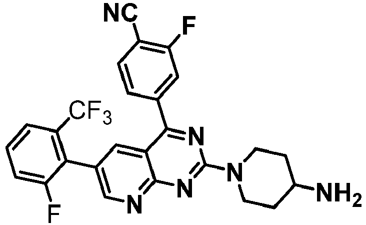 | 96 | 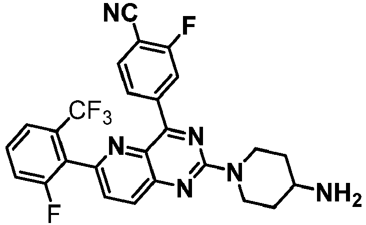 |
| 97 | 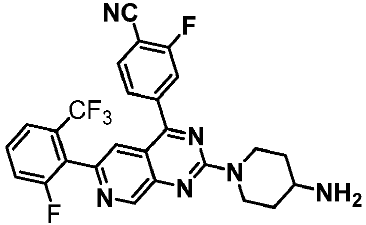 | 98 | 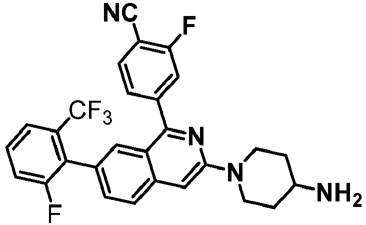 |
| 99 | 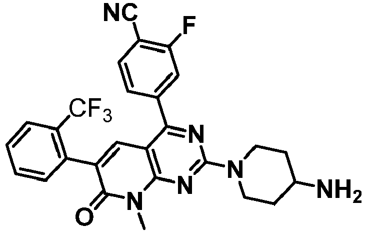 | | |

9. A pharmaceutical composition comprising a therapeutically effective amount of substance A and pharmaceutically acceptable carriers; wherein the substance A is one or more of the aromatic heterocyclic compound of Formula I according to any one of claims 1-8, a pharmaceutically acceptable salt thereof, a stereoisomer, a racemate, a prodrug, a co-crystalline complex, a hydrate, or a solvate thereof.

10. A use of substance A in the preparation of a LSD1 inhibitor, wherein the substance A is one or more of the aromatic heterocyclic compound of Formula I according to any one of claims 1-8, a pharmaceutically acceptable salt thereof, a stereoisomer, a racemate, a prodrug, a co-crystalline complex, a hydrate, or a solvate thereof.

11. A use of substance A in the preparation of a drug, wherein the substance A is one or more of the aromatic heterocyclic compound of Formula I according to any one of claims 1-8, a pharmaceutically acceptable salt thereof, a stereoisomer, a racemate, a prodrug, a co-crystalline complex, a hydrate, or a solvate thereof;
wherein the drug is used for the treatment or prevention of LSD1-mediated disease or cancer.

12. The use according to claim 11, wherein the LSD1-mediated disease is cancer;
and the cancer is selected from one or more of bladder cancer, breast cancer, prostate cancer, pancreatic cancer, thyroid cancer, liver cancer, colon cancer, rectal cancer, esophageal cancer, cervical cancer, ovarian cancer, acute leukemia, acute lymphoblastic leukemia, acute myeloid leukemia, acute T-cell leukemia, chondrosarcoma, chronic lymphocytic leukemia, chronic myeloid leukemia, large cell lymphoma, fibrosarcoma, testicular germ cell carcinoma, neuroglioma, lymphoma, multiple myeloma, lymph cancer, myeloma, neuroblastic tumor, neuroblastoma, non-small cell lung cancer, and small cell lung cancer.
